(19)

Europäisches
Patentamt
European
Patent Office
Office européen
des brevets

(11) **EP 4 477 673 A1**

(12) **EUROPEAN PATENT APPLICATION**
published in accordance with Art. 153(4) EPC

(43) Date of publication:
**18.12.2024 Bulletin 2024/51**

(21) Application number: **23752439.2**

(22) Date of filing: **10.02.2023**

(51) International Patent Classification (IPC):
*C07K 19/00* (2006.01)   *C07K 16/28* (2006.01)
*C07K 14/55* (2006.01)   *C12N 15/62* (2006.01)
*C12N 15/63* (2006.01)   *A61P 35/00* (2006.01)
*A61K 38/20* (2006.01)   *A61K 39/00* (2006.01)

(52) Cooperative Patent Classification (CPC):
**A61K 47/6851; A61K 47/6813; A61P 35/00;
C07K 14/5443; C07K 14/55; C07K 14/7155;
C07K 16/2818; C12N 15/62; C12N 15/63;**
A61K 38/00; A61K 2039/505; C07K 2317/22;
C07K 2317/24; C07K 2317/40; C07K 2317/52;
(Cont.)

(86) International application number:
**PCT/CN2023/075448**

(87) International publication number:
**WO 2023/151661 (17.08.2023 Gazette 2023/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.02.2022 CN 202210127604**

(71) Applicants:
• **Jiangsu Hengrui Pharmaceuticals Co., Ltd.
Lianyungang, Jiangsu 222047 (CN)**
• **Shanghai Shengdi Pharmaceutical Co., Ltd
Shanghai 201210 (CN)**

(72) Inventors:
• **LIN, Yuan
Shanghai 201210 (CN)**

• **RUN, Changqing
Shanghai 201210 (CN)**
• **WANG, Xue
Shanghai 201210 (CN)**
• **ZHOU, Caihong
Shanghai 201210 (CN)**
• **LIAO, Cheng
Shanghai 201210 (CN)**

(74) Representative: **Dragotti & Associati S.R.L.
Via Nino Bixio, 7
20129 Milano (IT)**

Remarks:
The complete document including Reference
Table(s) and the Sequence Listing(s) can be
downloaded from the EPO website

(54) **IMMUNOCONJUGATE AND USE THEREOF**

(57) The present disclosure relates to an immuno-
conjugate and the use thereof. In particular, the present
disclosure relates to an immunoconjugate comprising a
PD-1 antibody or an antigen-binding fragment thereof
and IL-2, and the use of the immunoconjugate for cancer
treatment.

EP 4 477 673 A1

**(Cont. next page)**

(52) Cooperative Patent Classification (CPC): (Cont.)
C07K 2317/569; C07K 2317/70; C07K 2317/73;
C07K 2317/732; C07K 2317/76; C07K 2317/92;
C07K 2319/00

**Description**

The present disclosure claims priority to Chinese Patent Application No. CN202210127604.7, filed on Feb. 11, 2022, entitled "IMMUNOCONJUGATE AND USE THEREOF", which is incorporated herein by reference in its entirety.

**TECHNICAL FIELD**

The present disclosure relates to the field of biopharmaceutics and particularly to an immunoconjugate comprising a PD-1 antibody and IL-2, a method thereof for treating cancer, and related pharmaceutical use thereof.

**BACKGROUND**

Human interleukin-2 (IL-2), also known as T cell growth factor (TCGF), has a gene located on chromosome 4 (4q27) and comprising a sequence of 7 kb in total, and consists of 133 amino acids with a molecular weight of about 15 kD. Early *in vitro* cell experiments showed that T cells could secrete IL-2 and express the IL-2 receptor (IL-2R) on the cell surface after being activated by TCR and CD28. The binding of IL-2 to its receptor can trigger T cell proliferation and enable T cell response. This model makes IL-2 a molecule that plays a central role in T cell immune responses. However, it was found in subsequent *in vivo* experiments that animals developed autoimmunity after IL-2 or its receptor was knocked out. Subsequent experiments showed that IL-2 could activate not only effector cells such as T cells and NK cells, but also regulatory T cells (Tregs), thereby suppressing excess self-directed autoimmunity.

IL-2 acts through IL-2R on the cell surface. IL-2R includes three subunits: IL-2R$\alpha$ (i.e., CD25), IL-2R$\beta$ (i.e., CD122), and IL-2R$\gamma$ (i.e., CD132). Those three subunits can form three forms of receptors: the high-binding affinity receptor comprising all of the three subunits IL-2R$\alpha$/$\beta$/$\gamma$, the medium-binding affinity receptor comprising IL-2R$\beta$/$\gamma$, and the low-binding affinity receptor being IL-2R$\alpha$. Among them, IL-2R$\beta$ and IL-2R$\gamma$ are necessary for IL-2 to activate downstream signaling pathways, and when IL-2 binds to both IL-2R$\beta$ and IL-2R$\gamma$, those two receptor subunits form a heterodimer that phosphorylates STATS in cells, and enters cell nucleus to cause corresponding gene transcription and expression; and IL-2R$\alpha$ is not necessary for signaling, but can enhance the binding of IL-2 to IL-2R$\beta$ and IL-2R$\gamma$. Tregs continuously express the high-binding affinity receptor IL-2R$\alpha$/$\beta$/$\gamma$, while unactivated CD8$^+$ T cells, NK cells and the like only express the medium-binding affinity receptor IL-2R$\beta$/$\gamma$. Therefore, low-dose IL-2 preferentially binds to and activates Tregs, while high-dose IL-2 can activate immune effector cells such as CD8$^+$ T cells and NK cells. Due to its ability to activate immune effector cells, high-dose IL-2 can be used for tumor immunotherapy. However, high-dose IL-2 treatment is accompanied by severe toxic and side effects, including fever, diarrhea, capillary leak syndrome, etc. These side effects are associated with the excessive activation of peripheral T cells and NK cells. By reducing or eliminating IL-2's activity on peripheral T cells and NK cells and specifically targeting the tumor microenvironment or tumor-associated immune cells, IL-2's anti-tumor effects can be exerted without it causing systemic toxicity.

PD-1 (programmed cell death-1), an immunosuppressive receptor, belongs to the CD28 receptor family (Riley et al., 2009, Immunol. Rev. 29:114-25). This family also includes CD28, CTLA-4, ICOS, PD-1, and BTLA. PD-1 is expressed mainly by activated B cells, T cells, and bone marrow cells (Chen et al., 2013, Nat. Rev. Immunol. 13:227-42). There are two cell surface glycoprotein ligands: PD-L1 and PD-L2. PD-L1 is widely expressed by lymphocytes (e.g., CD4$^+$ T cells, CD8$^+$ T cells, and macrophages), peripheral tissues, various tumor cells, virus-infected cells, etc. PD-L2 is expressed mainly by activated dendritic cells and macrophages (Dong et al., 1999, Nat. Med. 5:1365-9). Upon binding to its ligand PD-L1 or PD-L2, PD-1 down-regulates T cell function, including reducing T cell activation, differentiation, proliferation, cytokine secretion, etc.

PD-1 is characterized by low or no expression on the surfaces of peripheral T cells but high expression on the surfaces of tumor-associated T cells. Currently, several PD-1 monoclonal antibodies, such as pembrolizumab, nivolumab, and camrelizumab, have been developed for cancer immunotherapy. These monoclonal antibodies act by blocking the interaction between PD-1 and the ligand in the tumor microenvironment and have shown good therapeutic effects in various tumor types. However, there is still a significant proportion of patients who respond poorly to PD-1 mAbs. For instance, in advanced non-squamous non-small cell lung cancer, the response rate to nivolumab is 19% (Borghaei et al., 2015, N Engl J Med. 373:1627-39). The low response rate may be partly due to low levels of tumor-infiltrating lymphocytes (Daud et al., 2016, J. Clin. Invest. 126:3447-52) and lack of a T cell-activated tumor microenvironment (Ayers et al., 2017, J. Clin. Invest. 127:2930-2940). Clinical studies suggest that the combination of PD-1 and IL-2 might be very effective. However, such an IL-2 has certain systemic toxicities, such as flu-like symptoms (86.8%), rash (78.9%), and fatigue (73.7%) (Diab et al., 2020, Cancer Discov. 10:1158-1173), which limit efficacy.

The prior art lacks a solution that can simultaneously utilize IL-2 to enhance the anti-tumor effect of PD-1 while avoiding IL-2-related toxicities. The present disclosure provides an immunoconjugate comprising a PD-1 antibody and an IL-2 or a variant thereof, wherein the IL-2 variant has a reduced affinity for the high-affinity receptors (IL-2R$\alpha$/$\beta$/$\gamma$) and the intermediate-affinity receptors (IL-2R$\beta$/$\gamma$) as compared to wild-type IL-2 and has an eliminated affinity for the low-affinity receptor (IL-2Rα) as compared to wild-type IL-2, and the affinity for the high-affinity receptors is reduced more than the affinity for the intermediate-affinity receptors. Due to its reduced affinity for IL-2Rα/β/γ and IL-2Rβ/γ as compared to wild-type IL-2, the immunoconjugate is inactive on immune cells that lowly express or do not express PD-1 but can activate immune cells that highly express PD-1 in two ways. First, the PD-1 antibody blocks the interactions of PD-1/PD-L1 and PD-1/PD-L2, eliminating tumors' immunosuppression of T cells. Second, the immunoconjugate binds to PD-1 on the T cell surface, and only upon binding to PD-1 can its IL-2 variant bind to IL-2 receptors on the cell surface in an in-cis manner, thereby activating PD-1 positive T cells. This design utilizes the IL-2 variant to enhance the activation and proliferation of T cells by the PD-1 antibody and the anti-tumor effect and also increases the specificity of IL-2 for PD-1 positive immune cells, avoiding the systemic toxicity commonly associated with wild-type IL-2. This provides a more effective solution for clinical cancer treatment.

## SUMMARY

The present disclosure provides a PD-1 antibody or an antigen-binding fragment thereof; an IL-2 variant; an immunoconjugate comprising an antigen-binding domain (e.g., a PD-1-binding domain) and a cytokine (e.g., an IL-2 or a variant thereof); a coding nucleic acid, a vector, a host cell, and a pharmaceutical composition of the PD-1 antibody or the antigen-binding fragment thereof, the IL-2 variant, and the immunoconjugate; a method for treating or preventing a disease or disorder (e.g., a cancer) by using same; and related pharmaceutical use thereof.

PD-1 Antibody or Antigen-Binding Fragment Thereof

The present disclosure provides a PD-1 antibody or an antigen-binding fragment thereof comprising a PD-1-binding domain, wherein the PD-1-binding domain comprises at least one immunoglobulin single variable domain, and the immunoglobulin single variable domain comprises:

a CDR1, a CDR2, and a CDR3 from the amino acid sequence set forth in any one of SEQ ID NOs: 3, 13-15, 17-35, and 88-92, or
a CDR1, a CDR2, and a CDR3 from the amino acid sequence set forth in any one of SEQ ID NOs: 2 and 16;
the CDR1, CDR2, and CDR3 are defined according to the Kabat, IMGT, Chothia, AbM, or Contact numbering scheme. The immunoglobulin single variable domain specifically binds to PD-1 antigen or a fragment thereof.

The present disclosure provides a PD-1 antibody or an antigen-binding fragment thereof comprising a PD-1-binding domain, wherein the PD-1-binding domain comprises any one or any combination of the CDR1, CDR2, and CDR3 described above.

In some embodiments, in the aforementioned PD-1-binding domain, according to the Kabat numbering scheme, the amino acid sequences of the CDR1, CDR2, and CDR3 of the immunoglobulin single variable domain are set forth in

SEQ ID NOs: 97, 100, and 101, respectively,
SEQ ID NOs: 7, 36, and 37, respectively,
SEQ ID NOs: 97, 98, and 99, respectively, or
SEQ ID NOs: 4, 5, and 6, respectively.

In some embodiments, the amino acid sequence of the CDR1 of the immunoglobulin single variable domain in the aforementioned PD-1-binding domain is set forth in SEQ ID NO: 7 or 93, the amino acid sequence of the CDR2 is set forth in any one of SEQ ID NOs: 8, 38-40, and 94-95, and the amino acid sequence of the CDR3 is set forth in any one of SEQ ID NOs: 9, 41, 42, and 96.
In some embodiments, the amino acid sequences of the CDR1, CDR2, and CDR3 of the immunoglobulin single variable domain in the aforementioned PD-1-binding domain are set forth in SEQ ID NOs: 7, 38, and 41, respectively.
In some embodiments, the amino acid sequences of the CDR1, CDR2, and CDR3 of the immunoglobulin single variable domain in the aforementioned PD-1-binding domain are set forth in SEQ ID NOs: 7, 38, and 96, respectively.
In some embodiments, the amino acid sequences of the CDR1, CDR2, and CDR3 of the immunoglobulin single variable domain in the aforementioned PD-1-binding domain are set forth in SEQ ID NOs: 7, 94, and 41, respectively.
In some embodiments, the amino acid sequences of the CDR1, CDR2, and CDR3 of the immunoglobulin single variable domain in the aforementioned PD-1-binding domain are set forth in SEQ ID NOs: 93, 94, and 41, respectively.
In some embodiments, the amino acid sequences of the CDR1, CDR2, and CDR3 of the immunoglobulin single variable domain in the aforementioned PD-1-binding domain are set forth in SEQ ID NOs: 93, 94, and 96, respectively.
In some embodiments, the amino acid sequences of the CDR1, CDR2, and CDR3 of the immunoglobulin single variable domain in the aforementioned PD-1-binding domain are set forth in SEQ ID NOs: 93, 95, and 96, respectively.

In some embodiments, the amino acid sequences of the CDR1, CDR2, and CDR3 of the immunoglobulin single variable domain in the aforementioned PD-1-binding domain are set forth in SEQ ID NOs: 7, 8, and 9, respectively.

In some embodiments, the amino acid sequences of the CDR1, CDR2, and CDR3 of the immunoglobulin single variable domain in the aforementioned PD-1-binding domain are set forth in SEQ ID NOs: 7, 8, and 42, respectively.

In some embodiments, the amino acid sequences of the CDR1, CDR2, and CDR3 of the immunoglobulin single variable domain in the aforementioned PD-1-binding domain are set forth in SEQ ID NOs: 7, 38, and 6, respectively.

In some embodiments, the amino acid sequences of the CDR1, CDR2, and CDR3 of the immunoglobulin single variable domain in the aforementioned PD-1-binding domain are set forth in SEQ ID NOs: 7, 39, and 9, respectively.

In some embodiments, the amino acid sequences of the CDR1, CDR2, and CDR3 of the immunoglobulin single variable domain in the aforementioned PD-1-binding domain are set forth in SEQ ID NOs: 7, 40, and 6, respectively.

In some embodiments, the amino acid sequences of the CDR1, CDR2, and CDR3 of the immunoglobulin single variable domain in the aforementioned PD-1-binding domain are set forth in SEQ ID NOs: 7, 8, and 41, respectively.

In some embodiments, the amino acid sequences of the CDR1, CDR2, and CDR3 of the immunoglobulin single variable domain in the aforementioned PD-1-binding domain are set forth in SEQ ID NOs: 7, 8, and 42, respectively.

In some embodiments, the amino acid sequences of the CDR1, CDR2, and CDR3 of the immunoglobulin single variable domain in the aforementioned PD-1-binding domain are set forth in SEQ ID NOs: 7, 38, and 41, respectively.

In some embodiments, the amino acid sequences of the CDR1, CDR2, and CDR3 of the immunoglobulin single variable domain in the aforementioned PD-1-binding domain are set forth in SEQ ID NOs: 7, 38, and 42, respectively.

In some embodiments, the amino acid sequences of the CDR1, CDR2, and CDR3 of the immunoglobulin single variable domain in the aforementioned PD-1-binding domain are set forth in SEQ ID NOs: 7, 39, and 41, respectively.

In some embodiments, the amino acid sequences of the CDR1, CDR2, and CDR3 of the immunoglobulin single variable domain in the aforementioned PD-1-binding domain are set forth in SEQ ID NOs: 7, 39, and 42, respectively.

In some embodiments, the amino acid sequences of the CDR1, CDR2, and CDR3 of the immunoglobulin single variable domain in the aforementioned PD-1-binding domain are set forth in SEQ ID NOs: 7, 40, and 41, respectively.

In some embodiments, the amino acid sequences of the CDR1, CDR2, and CDR3 of the immunoglobulin single variable domain in the aforementioned PD-1-binding domain are set forth in SEQ ID NOs: 7, 40, and 42, respectively.

In some embodiments, the immunoglobulin single variable domain in the aforementioned PD-1 antibody or the antigen-binding fragment thereof is modified by humanization, affinity maturation, T cell epitope removal, reduction of antibody deamidation, and/or reduction of antibody isomerization. In some embodiments, the immunoglobulin single variable domain is obtained by affinity maturation and has one or more changes in one or more CDRs that result in an increase in affinity for PD-1 as compared to the parent.

In some specific embodiments, the humanization modification process uses a human germline template's heavy chain framework region IGHV3-23 *01 or IGHV3-23*04.

In some embodiments, the immunoglobulin single variable domain in the aforementioned PD-1-binding domain comprises the amino acid sequence set forth in any one of SEQ ID NOs: 3, 13-15, 17-35, and 88-92, or the amino acid sequence set forth in any one of SEQ ID NOs: 2 and 16, or a sequence having at least 80%, at least 85%, or at least 90% sequence identity to any one of the aforementioned sequences.

In some embodiments, the aforementioned PD-1 antibody or the antigen-binding fragment thereof comprises or is an antibody or an antigen-binding fragment thereof that specifically binds to PD-1 or a fragment thereof; the antibody or the antigen-binding fragment thereof is, for example, a camelid antibody, a chimeric antibody, a humanized antibody, a fully human antibody, or an antigen-binding fragment thereof; the antibody or the antigen-binding fragment thereof is, for example, a recombinant antibody or a fragment thereof. In some specific embodiments, the antigen-binding fragment is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, at least one immunoglobulin single variable domain in the aforementioned PD-1 antibody or the antigen-binding fragment thereof is a VHH.

In some embodiments, the PD-1 antibody or the antigen-binding fragment thereof comprises one or more (e.g., 2, 3, 4, 5, or 6) aforementioned immunoglobulin single variable domains, and the immunoglobulin single variable domains may be identical or different and may form a dimeric or multimeric molecule.

In some embodiments, in the aforementioned PD-1 antibody or the antigen-binding fragment thereof, the PD-1-binding domain comprises: a PD-1-binding domain of a heavy chain variable region (VH) and a light chain variable region (VL). For example, the PD-1-binding domain comprises or is a Fab, a Fab', a (Fab')$_2$, an ScFv, an Fv, and the like. In some specific embodiments, the PD-1-binding domain comprising a heavy chain variable region and a light chain variable region is a Fab.

In some specific embodiments, the PD-1 antibody or the antigen-binding fragment thereof comprises a PD-1-binding domain 1 and a PD-1-binding domain 2, or comprises a PD-1-binding domain 1, or comprises a PD-1-binding domain 2. The PD-1-binding domain 1 comprises at least one of any one of the aforementioned immunoglobulin single variable domains. The PD-1-binding domain 2 comprises a heavy chain variable region (VH) and a light chain variable region (VL) that bind to PD-1.

Illustratively, the PD-1-binding domain 2 is selected from the group consisting of the PD-1-binding domains in the following PD-1 antibodies: pembrolizumab, nivolumab, sintilimab, tislelizumab, cemiplimab, toripalimab, camrelizumab, penpulimab, zimberelimab, sasanlimab, spartalizumab, retifanlimab, balstilimab, cetrelimab, dostarlimab (TSR-042), CS-1003, SCT-I10A, SSI-361, MEDI-0680(AMP-514), BAT-1306, JTX-4014, JTX-4014, BI-754091, and AK-103.

In some other embodiments, the PD-1-binding domain 2 in the immunoconjugate is selected from the group consisting of the antigen-binding domains of the following antibodies: PD-L1 antibodies (e.g., durvalumab, atezolizumab, durvalumab, avelumab, adebrelimab, envafolimab, STI-A1110, CS-1001, TQB-2450, KL-A167, IMC-001, and CX-072); CTLA-4 antibodies (e.g., tremelimumab and ipilimumab); and 4-1BB antibodies (e.g., urelumab and utomilumab).

The antigen-binding domain (e.g., PD-1-binding domain 2) is a portion of an antibody (e.g., a PD-1 antibody) that binds to an antigen (e.g., PD-1), e.g., the Fab' thereof, the F(ab')2 thereof, or the heavy chain variable region and the light chain variable region thereof, or a domain that comprises the HCDR1, HCDR2, and HCDR3 of the heavy chain variable region thereof and the LCDR1, LCDR2, and LCDR3 of the light chain variable region thereof.

In some embodiments, the PD-1-binding domain 2 comprises pembrolizumab or an antigen-binding fragment thereof (e.g., the Fab), e.g., comprises a heavy chain variable region (VH) and a light chain variable region (VL), wherein the heavy chain variable region comprises a HCDR1, a HCDR2, and a HCDR3 of the amino acid sequences set forth in SEQ ID NOs: 54, 55, and 56, and the light chain variable region comprises a LCDR1, a LCDR2, and a LCDR3 of the amino acid sequences set forth in SEQ ID NOs: 57, 58, and 59. In some specific embodiments, the heavy chain variable region is a heavy chain variable region from a Fab heavy chain of the amino acid sequence set forth in SEQ ID NO: 52, and the light chain variable region comprises a light chain variable region from a Fab light chain of the amino acid sequence set forth in SEQ ID NO: 53.

In some embodiments, the aforementioned PD-1 antibody or the antigen-binding fragment thereof also comprises a human immunoglobulin Fc region; for example, the Fc region is an Fc region of human IgG1, IgG2, or IgG4. The Fc region may have mutations. Exemplary mutations are L234A/L235A on IgG1, V234A/G237A/P238S/H268A/ V309L/A330S/P331S on IgG2, F234A/L235A on IgG4, S228P/F234A/L235A on IgG4, N297A on IgG1, IgG2, IgG3, or IgG4, V234A/G237A on IgG2, K214T/E233P/L234V/L235A/G236 deletion/A327G/P331A/D365E/L358M on IgG1, H268Q/V309L/A330S/P331S on IgG2, , L234F/L235E/D265A on IgG1, L234A/L235A/G237A/P238-S/H268A/A330S/P331S on IgG1, S228P/F234A/L235A/ G237A/P238S on IgG4, and S228P/F234A/L235A/G236 deletion/G237A/P238S on IgG4. A hybrid IgG2/4Fc domain may also be used, e.g., an Fc with residues 117-260 from IgG2 and residues 261-447 from IgG4. In some specific embodiments, the Fc region of human IgG4 has the S228P, F234A, L235A, and/or K447A mutation(s). In some specific embodiments, the Fc region of human IgG1 has the L234A/L235A or L234A/L235A/P329G mutations. In some specific embodiments, the Fc region of human IgG1 has or does not have the C220S mutation.

The mutations in the Fc region described above are numbered according to the EU numbering scheme.

In some embodiments, the Fc region contained in the aforementioned PD-1 antibody or the antigen-binding fragment thereof causes the binding protein to form a dimeric molecule and meanwhile extends the *in vivo* half-life of the binding protein.

In some embodiments, in the aforementioned PD-1 antibody or the antigen-binding fragment thereof, the immunoglobulin single variable domain is linked to the Fc region by a linker. The linker may be a non-functional amino acid sequence of 1-20 or more amino acids without a secondary or higher structure. For example, the linker is a flexible linker, for example, $G_4S$, GS, GAP, $(G_4S)_2$, $(G_4S)_3$, $(G_4S)_4$, $(G_4S)_5$, or ASGS.

In some embodiments, the aforementioned PD-1 antibody or the antigen-binding fragment thereof has activity selected from the group consisting of at least one of the following:

(a) binding to human PD-1 or an epitope thereof with a $K_D$ value of $\leq 10^{-7}$;
(b) inhibiting the binding of PD-1 to PD-L1;
(c) inhibiting the binding of PD-1 to PD-L2;
(d) inducing CD4$^+$ T cells to secrete IFN-$\gamma$;
(e) enhancing PBMC activation;
(f) enhancing T cell activation; and
(g) inhibiting tumor growth.

In some embodiments, the aforementioned PD-1 antibody or the antigen-binding fragment thereof of the present disclosure may bind to PD-1 with a $K_D$ value of $\leq 1 \times 10^{-7}$ M, e.g., $\leq 1 \times 10^{-8}$ M, or $\leq 1 \times 10^{-9}$ M, or $\leq 1 \times 10^{-10}$ M.

In some embodiments, the PD-1 antibody or the antigen-binding fragment thereof of the present disclosure is capable of specifically binding to human PD-1 and blocking the interaction between PD-1 and PD-L1, and/or the interaction between PD-1 and PD-L2. In some embodiments, the aforementioned PD-1 antibody or the antigen-binding fragment thereof of the present disclosure is capable of inhibiting tumor growth by at least about 10%, e.g., at least about 20%, about 30%, about 40%, about 50%, about 60%, about 70%, or about 80%.

In some embodiments, the aforementioned PD-1 antibody or the antigen-binding fragment thereof of the present disclosure encompasses variants, e.g., comprises one or more amino acid substitutions, preferably conservative amino acid substitutions, e.g., 1, 2, 3, 4, 5, 6, 7, 8, 9, or 10 conservative amino acid substitutions, as compared to any one of SEQ ID NOs: 2-3 and 13-35, and the substitutions may occur in a CDR and/or an FR. In some embodiments, the aforementioned PD-1 antibody or the antigen-binding fragment thereof of the present disclosure is resistant to heat treatment or has relatively high stability. For example, no significant aggregation or degradation is observed after up to 30 days of treatment at 40 °C, and they are stable at least at 60 °C.

In some embodiments, provided is a PD-1 antibody or an antigen-binding fragment thereof that binds to or competes for binding to the same epitope with the immunoglobulin single variable domain in the aforementioned PD-1 antibody or the antigen-binding fragment thereof of the present disclosure.

In some embodiments, provided is a PD-1 antibody or an antigen-binding fragment thereof that blocks the binding of the immunoglobulin single variable domain in the aforementioned PD-1 antibody or the antigen-binding fragment thereof of the present disclosure to PD-1 (e.g., human PD-1).

In some embodiments, provided is a PD-1 antibody or an antigen-binding fragment thereof whose binding to PD-1 (e.g., human PD-1) is blocked by the immunoglobulin single variable domain in the aforementioned PD-1 antibody or the antigen-binding fragment thereof of the present disclosure.

In some embodiments, the aforementioned PD-1 antibody or the antigen-binding fragment thereof of the present disclosure reduces the binding of PD-1 to PD-L1 and/or PD-L2 by at least 60%, at least 70%, at least 80%, at least 90%, at least 95%, or at least 98%.

In some embodiments, the present disclosure provides a PD-1-binding protein comprising at least one of the aforementioned immunoglobulin single variable domains that binds to PD-1 (such as a VHH).

As previously described, and in the present disclosure, "at least 90% identity" includes at least 90%, at least 91%, at least 92%, at least 93%, at least 94%, at least 95%, at least 96%, at least 97%, at least 98%, and at least 99% identity.

## IL-2 and Variant Thereof

The present disclosure provides IL-2 and a variant thereof. The IL-2 is preferably human IL-2, and the amino acid sequence of wild-type human IL-2 is set forth in, for example, SEQ ID NO: 87. The position of the IL-2 variant of the present disclosure is numbered according to a wild-type IL-2 amino acid sequence set forth in SEQ ID NO: 87.

In some embodiments, the present disclosure provides an IL-2 variant having a reduced affinity for the high-affinity receptors (IL-2R$\alpha$/$\beta$/$\gamma$) and the intermediate-affinity receptors (IL-2R$\beta$/$\gamma$) as compared to wild-type IL-2 and/or having an eliminated affinity for the low-affinity receptor (IL-2R$\alpha$) as compared to wild-type IL-2. In some specific embodiments, the affinity of the IL-2 variant for IL-2R$\alpha$/$\beta$/$\gamma$ is reduced more than the affinity for IL-2R$\beta$/$\gamma$. The affinity can be measured by conventional methods, such as ELISA, ForteBio, and SPR, such as the SPR measuring method provided in Example 13 of the present disclosure.

In some embodiments, the present disclosure provides an IL-2 variant comprising or consisting of the following mutation (i), mutation (ii), mutation (iii), mutation (iv), or any combination thereof.

Regarding mutation (i):

The present disclosure provides a mutation that makes the IL-2 variant have an altered (e.g., increased or reduced) affinity for IL-2R$\beta$/$\gamma$ as compared to wild-type IL-2.

In some embodiments, the IL-2 variant has a mutation (i) that results in a reduced affinity for IL-2R$\beta$/$\gamma$ as compared to wild-type IL-2. In some specific embodiments, the IL-2 variant has a 1-1000 fold, 2-800 fold, 5-500 fold, 5-200 fold, 10-200 fold, 10-100 fold, 10-50 fold, 20-200 fold, 20-100 fold, 5-20 fold, 2-20 fold, 50-100 fold, or 50-70 fold, e.g., about 5, about 10, about 13, about 15, about 20, about 30, about 40, about 50, about 60, about 63, about 70, about 80, about 90, about 100, about 110, about 120, about 130, about 140, about 150, about 180, about 200, about 250, about 300, about 400, or about 500 fold reduction in affinity for IL-2R$\beta$/$\gamma$ as compared to wild-type IL-2. In some specific embodiments, the IL-2 variant has a 10-100 fold reduction in affinity for IL-2R$\beta$/$\gamma$ as compared to wild-type IL-2. The affinity can be measured by conventional methods, such as the SPR measuring method provided in Example 13 of the present disclosure.

In some embodiments, the aforementioned mutation (i) is located at positions 12-20, positions 84-95, and positions 126-130 of IL-2.

In some embodiments, the aforementioned mutation (i) is selected from the group consisting of positions 12, 15, 16, 18, 19, 20, 23, 43, 69, 84, 87, 88, 91, 92, 95, 123, 126, 127, 129, and 130 or any combination thereof; in some embodiments, the mutation is selected from the group consisting of positions L12, E15, H16, L18, L19, D20, M23, Q22, K43, V69, D84, S87, N88, V91, I92, E95, T123, Q126, S127, I129, and S130 or any combination thereof, e.g., N88/D20; in some embodiments, the mutation is selected from the group consisting of 12R, 12K, 12E, 12Q, 15Q, 15R, 15A, 15S, 16N, 16A, 16E, 16D, 16G, 16S, 16T, 16V, 16P, 20A, 20H, 20Y, 20N, 20Q, 20E, 20R, 20S, 23A, 23R, 23K, 23G, 23S, 23T, 23V, 23P, 87K, 87A, 84S, 84L, 84N, 84V, 84H, 84Y, 84R, 84K, 84G, 84A, 84T, 84P, 88D, 88A, 88S, 88E, 88T, 88R, 88I, 91A, 91T, 91E, 91I, 91L, 91D, 91N, 91Q, 91S, 91H, 92E, 92T, 92K, 92R, 92L, 95S, 95A, 95R, 95Q, 95G, 95T, 95V, 95P, 95H, 95N, 123A, 123E, 123K, 123Q, 126D, 126L, 126A, 126S, 126T, 126E, 127A, 127E, 127K, 127Q, 84N/95Q, 84E/95Q, 84T/95Q, 84Q/95Q, 84T/16T, 84N/91I, 84T/126E, 84N/126E, 16T/84Q, and 16T/91I; in some specific embodiments, the mutation is selected from the group consisting of L12R, L12K, L12E, L12Q, E15Q, E15R, E15A, E15S, H16N, H16A, H16E, H16D, H16G, H16S, H16T, H16V, H16P, D20A, D20H, D20Y, D20N, D20Q, D20E, D20R, D20S, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88E, N88T, N88R, N88I, V91A, V91T, V91E, V91I, V91L, V91D, V91N, V91Q, V91S, V91H, I92E, I92T, I92K, I92R, I92L, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, S127Q, D84N/E95Q, D84E/E95Q, D84T/E95Q, D84Q/E95Q, D84T/H16T, D84N/V91I, D84T/Q126E, D84N/Q126E, H16T/D84Q, and H16T/V91I; in some embodiments, the mutation (i) is selected from the group consisting of N88D, N88R, D20A, D20N, V91T, Q126D, and H16A/D84S; in some embodiments, the mutation (i) is N88D or D20A.

Regarding mutation (ii):

The present disclosure provides a mutation that makes the IL-2 variant have an altered (e.g., reduced) affinity for IL-2Rα or do not bind to IL-2Rα, as compared to wild-type IL-2.

In some embodiments, the IL-2 variant has a mutation (ii) that results in a reduced affinity for IL-2Rα as compared to wild-type IL-2 or not binding to IL-2Rα. The affinity can be measured by conventional methods, such as the SPR measuring method provided in Example 13 of the present disclosure.

In some specific embodiments, the aforementioned mutation (ii) is selected from the group consisting of positions 35, 37, 38, 41, 42, 43, 45, 61, 62, 65, 68, and 72 or any combination thereof; in some embodiments, the mutation is selected from the group consisting of 38A, 38D, 38E, 62Q, 42A, 42G, 42S, 42T, 42Q, 42E, 42N, 42D, 42R, 42K, 45A, 45G, 45S, 45T, 45Q, 45E, 45N, 45D, 45R, 45K, 65R, 65E, 65K, 65H, 65Y, 65Q, 65D, 65N, 68A, 68Q, 68K, 68R, 72G, 72A, 72S, 72T, 72Q, 72E, 72N, 72D, 72R, and 72K; in some embodiments, the mutation (ii) is selected from the group consisting of positions K35, T37, R38, E62, P65, E68, F42, Y45, and L72 or any combination thereof; in some embodiments, the mutation is selected from the group consisting of R38A, R38D, R38E, E62Q, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, P65R, P65E, P65K, P65H, P65Y, P65Q, P65D, P65N, E68A, E68Q, E68K, E68R, L72G, L72A, L72S, L72T, L72Q, L72E, L72N, L72D, L72R, and L72K; in some embodiments, the mutation (ii) is selected from the group consisting of F42A, Y45A, L72G, R38E/F42A, F42A/L72G, F42A/Y45A, Y45A/L72G, and F42A/Y45A/L72G.

Regarding mutation (iii):

The present disclosure provides a mutation (iii) that makes the IL-2 variant have increased stability, expression, purity, and druggability.

The IL-2 variants in WO2020125743A are incorporated herein by reference in their entirety.

In some embodiments, the IL-2 variant has a mutation (iii) that is selected from the group consisting of positions 11, 26, 27, 29, 30, 31, 32, 33, 34, 35, 36, 37, 38, 39, 40, 41, 42, 43, 44, 70, 71, 72, 78, 82, 88, and 132 or any combination thereof; in some embodiments, the mutation (iii) is selected from the group consisting of 26Q, 29S, 30S, 71Q, 11C, 132C, 70C, 82C, 27C, and 78C or any combination thereof; in some embodiments, the mutation (iii) is selected from the group consisting of positions N26, N29, N30, N71, Q11, L132, L70, P82, G27, and F78 or any combination thereof; in some embodiments, the mutation (iii) is selected from the group consisting of N26Q, N29S, N30S, N71Q, Q11C/L132C, L70C/P82C, and G27C/F78C, or any combination thereof; in some embodiments, the mutation (iii) is selected from the group consisting of N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

Regarding mutation (iv):

In some embodiments, the IL-2 variant comprises a mutation (iv) that is selected from the group consisting of position(s) 3 and/or 125; in some embodiments, the mutation (iv) is selected from the group consisting of position(s) T3 and/or C125; in some embodiments, the mutation (iv) is selected from the group consisting of 3A, 3G, 3Q, 3E, 3N, 3D, 3R, 3K, 3P, and/or 125A, 125S, 125T, 125V mutations. In some embodiments, the mutation (iv) is selected from the group consisting of the T3A, T3G, T3Q, T3E, T3N, T3D, T3R, T3K, T3P, and/or C125A, C125S, C125T, C125V mutations.

By way of example, "mutation 1/mutation 2" means that both mutation 1 and mutation 2 are included.

Exemplary IL-2 Variants

The present disclosure provides an IL-2 comprising or only comprising the following mutations:

(a) F42A and N88D, N88A, N88S, N88E, N88T, N88R, or N88I;
(b) F42A and D20A, D20H, D20Y, D20N, D20Q, D20E, D20R, or D20S;
(c) F42A and Q126D, Q126L, Q126A, Q126S, Q126T, or Q126E;
(d) F42A and V91A, V91T, V91E, V91I, V91L, V91D, V91N, V91Q, V91S, or V91H;
(e) F42A/L72G and N88D, N88A, N88S, N88E, N88T, N88R, or N88I;
(f) F42A/L72G and D20A, D20H, D20Y, D20N, D20Q, D20E, D20R, or D20S;
(g) F42A/L72G and Q126D, Q126L, Q126A, Q126S, Q126T, or Q126E;
(h) F42A/L72G and V91A, V91T, V91E, V91I, V91L, V91D, V91N, V91Q, V91S, or V91H;
(s) F42A/R38E and V91A, V91T, V91E, V91I, V91L, V91D, V91N, V91Q, V91S, or V91H;
(i) any one of the aforementioned (a)-(h) and (s) and N26Q;
(j) any one of the aforementioned (a)-(h) and (s) and N29S;
(k) any one of the aforementioned (a)-(h) and (s) and N71Q;
(l) any one of the aforementioned (a)-(h) and (s) and N26Q/N29S;
(m) any one of the aforementioned (a)-(h) and (s) and N26Q/N29S/N71Q;
(n) any one of the aforementioned (a)-(h) and (s) and Q11C/L132C;
(o) any one of the aforementioned (a)-(h) and (s) and L70C/P82C;
(p) any one of the aforementioned (a)-(h) and (s) and G27C/F78C;
(q) any one of the aforementioned (a)-(h) and (s) and T3A;
(r) any one of the aforementioned (a)-(h) and (s) and T3A/C125A.

In some embodiments, the present disclosure provides an IL-2 variant comprising or having the amino acid sequence set forth in any one of SEQ ID NOs: 60-68 or has at least 80%, at least 85%, or at least 90% sequence identity thereto.
In some embodiments, any of the aforementioned IL-2 variants that comprise the mutation (i) has any one or more of 1)-5) as compared to wild-type IL-2:

1) a reduced affinity for the high-affinity IL-2R receptor (IL-2R$\alpha\beta\gamma$);
2) a reduced affinity for the intermediate-affinity IL-2R receptor (IL-2R$\beta\gamma$);
3) reduced activation of T cells (e.g., CD4$^+$ and CD8$^+$ T cells);
4) reduced activation of NK cells;
5) reduced IL-2 stimulated release of T cell and/or NK cell inflammatory factors.

In some embodiments, any of the aforementioned IL-2 variants that comprise the mutations (i) and (ii) has any one or more of the aforementioned 1)-5) and the following 6)-8) as compared to wild-type IL-2:

6) a reduced affinity for IL-2R$\alpha$ or no binding to IL-2R$\alpha$;
7) the affinity for IL-2R$\alpha\beta\gamma$ being reduced more than the affinity for IL-2R$\beta\gamma$;
8) reduced activation of CD25$^+$ cells (e.g., CD25$^+$ CD8$^+$ T cells, and Treg cells).

In some embodiments, any of the aforementioned IL-2 variants that comprise the mutations (i) and (ii) and (iii) and/or (iv) has any one or more of the aforementioned 1)-8) and the following 9) as compared to wild-type IL-2:
9) increased stability, druggability, expression, purity, and/or prolonged *in vivo* half-life. Immunoconjugate
The present disclosure provides an immunoconjugate comprising an antigen-binding domain and an IL-2 or a variant thereof.
In some embodiments, the antigen-binding domain in the immunoconjugate targets an immune effector cell or a tumor cell; in some specific embodiments, the immune effector cell is capable of responding to stimulation or activation by the IL-2 in the immunoconjugate; in some specific embodiments, the immune effector cell has IL-2R$\beta\gamma$ thereon; in some specific embodiments, the immune effector cell is, for example, an NK cell, a CD4$^+$ CD25$^-$ T cell, or a CD8$^+$ CD25$^-$ T cell.
In some embodiments, the antigen-binding domain in the immunoconjugate targets PD-1, CTLA-4, LAG3, TIM3, 4-1BB, OX40, GITR, CD8a, CD8b, CD4, NKp30, NKG2A, TIGIT, TGF$\beta$R$\gamma$, TGF$\beta$R2, Fas, NKG2D, NKp46, PD-L1, CD107a, ICOS, TNFR2, FAP, TNC A1, TNC A2, CEA, EDB, MCSP, or CD16a; in some specific embodiments, the antigen-binding domain targets PD-1 (i.e., a PD-1-binding domain).
In some embodiments, the PD-1-binding domain is any of the aforementioned PD-1-binding domains provided by the present disclosure.

Illustratively, in some specific embodiments, the PD-1-binding domain comprises at least one immunoglobulin single variable domain, and the immunoglobulin single variable domain comprises: a CDR1, a CDR2, and a CDR3 from the amino acid sequence set forth in any one of SEQ ID NOs: 3, 13-15, 17-35, and 88-92, or a CDR1, a CDR2, and a CDR3 from the amino acid sequence set forth in any one of SEQ ID NOs: 2 and 16; the CDR1, CDR2, and CDR3 are defined according to the Kabat, IMGT, Chothia, AbM, or Contact numbering scheme.

Illustratively, in some specific embodiments, in the PD-1-binding domain, according to the Kabat numbering scheme, the amino acid sequences of the CDR1, CDR2, and CDR3 of the immunoglobulin single variable domain are set forth in SEQ ID NOs: 7, 36, and 37, or SEQ ID NOs: 4, 5, and 6, respectively.

Illustratively, in some specific embodiments, the amino acid sequence of the CDR1 of the immunoglobulin single variable domain in the PD-1-binding domain is set forth in SEQ ID NO: 7, the amino acid sequence of the CDR2 is set forth in any one of SEQ ID NOs: 8 and 38-40, and the amino acid sequence of the CDR3 is set forth in any one of SEQ ID NOs: 9, 41, and 42.

Illustratively, in some specific embodiments, the immunoglobulin single variable domain comprises a CDR1, a CDR2, and a CDR3 set forth in SEQ ID NOs: 7, 36, and 37, or SEQ ID NOs: 4, 5, and 6, or SEQ ID NOs: 7, 38, and 41.

Illustratively, in some specific embodiments, the amino acid sequence of the immunoglobulin single variable domain comprises the amino acid sequence set forth in any one of SEQ ID NOs: 3, 13-15, 17-35, and 88-92; or the amino acid sequence set forth in any one of SEQ ID NOs: 2 and 16; or an amino acid sequence having at least 90% sequence identity to any one of the sequences.

In some embodiments, the PD-1-binding domain may also comprise a PD-1-targeting antigen-binding fragment other than the immunoglobulin single variable domain described above, wherein the antigen-binding fragment comprises a heavy chain variable region (VH) and a light chain variable region (VL). Illustratively, the PD-1-targeting antigen-binding fragment is selected from the group consisting of the PD-1-binding domains in the following PD-1 antibodies: pembrolizumab, nivolumab, sintilimab, tislelizumab, cemiplimab, toripalimab, camrelizumab, penpulimab, zimberelimab, sasanlimab, spartalizumab, retifanlimab, balstilimab, cetrelimab, dostarlimab (TSR-042), CS-1003, SCT-I10A, SSI-361, MEDI-0680(AMP-514), BAT-1306, JTX-4014, JTX-4014, BI-754091, and AK-103.

In some other embodiments, the antigen-binding domain in the immunoconjugate is selected from the group consisting of the antigen-binding domains of the following antibodies: PD-L1 antibodies (e.g., durvalumab, atezolizumab, durvalumab, avelumab, adebrelimab, envafolimab, STI-A1110, CS-1001, TQB-2450, KL-A167, IMC-001, and CX-072); CTLA-4 antibodies (e.g., tremelimumab and ipilimumab); and 4-1BB antibodies (e.g., urelumab and utomilumab).

The antigen-binding domain (e.g., a PD-1-binding domain) is a portion of an antibody (e.g., a PD-1 antibody) that binds to an antigen (e.g., PD-1), e.g., the Fab' thereof, the F(ab')2 thereof, or the heavy chain variable region and the light chain variable region thereof, or a domain that comprises the HCDR1, HCDR2, and HCDR3 of the heavy chain variable region thereof and the LCDR1, LCDR2, and LCDR3 of the light chain variable region thereof.

In some embodiments, the PD-1-binding domain comprises pembrolizumab or an antigen-binding fragment thereof (e.g., the Fab), e.g., comprises a heavy chain variable region (VH) and a light chain variable region (VL), wherein the heavy chain variable region comprises a HCDR1, a HCDR2, and a HCDR3 of the amino acid sequences set forth in SEQ ID NOs: 54, 55, and 56, and the light chain variable region comprises a LCDR1, a LCDR2, and a LCDR3 of the amino acid sequences set forth in SEQ ID NOs: 57, 58, and 59. In some specific embodiments, the heavy chain variable region is a heavy chain variable region from a Fab heavy chain of the amino acid sequence set forth in SEQ ID NO: 52, and the light chain variable region comprises a light chain variable region from a Fab light chain of the amino acid sequence set forth in SEQ ID NO: 53.

In some embodiments, the immunoconjugate comprises any one of the following:

1) a PD-1-binding domain 1 and an IL-2 or a variant thereof, wherein the PD-1-binding domain 1 comprises at least one of any of the immunoglobulin single variable domains provided by the present disclosure; and
2) a PD-1-binding domain 1, a PD-1-binding domain 2, and an IL-2 or a variant thereof, wherein the PD-1-binding domain 1 comprises at least one of any of the immunoglobulin single variable domains provided by the present disclosure; the PD-1-binding domain 2 is any PD-1-binding domain provided by the present disclosure that comprises a VH and a VL.

In some embodiments, the IL-2 or the variant thereof in the immunoconjugate is any of the aforementioned IL-2 or the variants thereof provided by the present disclosure.

Illustratively, in some specific embodiments, the IL-2 is wild-type human IL-2.

Illustratively, in some specific embodiments, the IL-2 variant comprises or consists of the aforementioned mutation (i), mutation (ii), mutation (iii), or mutation (iv) provided by the present disclosure or any combination thereof.

Illustratively, in some specific embodiments, the IL-2 variant comprises or only comprises a mutation of any one of the aforementioned (a)-(r) and (s) provided by the present disclosure.

Illustratively, in some specific embodiments, the IL-2 variant comprises or consists of the amino acid sequence set forth in any one of SEQ ID NOs: 60-68 or a sequence having at least 80%, at least 85%, or at least 90% identity thereto.

In some embodiments, the immunoconjugate also comprises a human immunoglobulin Fc region, e.g., an Fc region of human IgG1, IgG2, IgG3, or IgG4.

In some embodiments, the antigen-binding domain (e.g., PD-1-binding domain) in the immunoconjugate is located at the N-terminus of the Fc region, and the IL-2 or the variant thereof is located at the N-terminus or C-terminus of the Fc region. In some specific embodiments, the antigen-binding domain is located at the N-terminus of the Fc region and the IL-2 or the variant thereof is located at the C-terminus of the Fc region.

In some embodiments, the valence ratio of the antigen-binding domain (e.g., PD-1-binding domain) to the IL-2 or the variant thereof in the immunoconjugate is between 6:1 and 1:3 (e.g., 4:1 to 1:2), specifically, e.g., 4:1, 2:1, 3:1, or 1:1.

As used herein, the valence ratio of the PD-1-binding domain to the IL-2 or the variant thereof refers to the ratio of the number of PD-1-binding domains to the number of IL-2 or variants thereof contained in one immunoconjugate molecule. In some embodiments, the valence ratio is the ratio of the number of PD-1-binding domains 1 comprising at least one immunoglobulin single variable domain to the number of IL-2 or variants thereof. In some embodiments, the valence ratio is the ratio of the sum of the number of PD-1-binding domains 1 and the number of PD-1-binding domains 2 to the number of IL-2 or variants thereof.

In some embodiments, in the immunoconjugate, one immunoconjugate comprises 1 or 2 IL-2 or variants thereof and 1, 2, 3, 4, 5, or 6 antigen-binding domains (e.g., PD-1-binding domains).

In some embodiments, the immunoconjugate also comprises an Fc region, wherein the Fc region comprises a knob-into-hole modification of a first subunit Fc1 and a second subunit Fc2 capable of associating with each other.

In some embodiments, Fc1 and Fc2 comprise such amino acid mutations that Fc1 preferentially pairs with Fc2 or preferentially forms a heterodimer as compared to pairing with Fc1. In some embodiments, the mutation is located in the CH3 of Fc1 and Fc2. In some embodiments, the amino acid mutations in Fc1 and Fc2 result in greater electrostatic complementarity than a wild type without the mutations. In some embodiments, the amino acid mutations in Fc1 and Fc2 result in greater steric complementarity than a wild type without the mutations.

In some embodiments, in Fc1 and Fc2, for example, within the CH3/CH3 interface, one or more amino acid residues in the CH3 domain of Fc1 are mutated with one or more amino acid residues having a larger side-chain volume, thereby forming protuberances (or knobs) in the surface of the CH3 domain of Fc1; one or more, preferably two or three, of the amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some embodiments, an import residue having a larger side-chain volume is phenylalanine (F), tyrosine (Y), arginine (R), or tryptophan (W). In some embodiments, an import residue having a smaller side-chain volume is serine (S), alanine (a), valine (V), or threonine (T).

In some embodiments, the Fc1 comprises at least one or at least two amino acid mutations selected from the group consisting of T366, L368, and Y407 (hole mutation modifications, also known as Hole modifications), and the Fc2 comprises T366 (a knob mutation modification, also known as a Knok modification); or the Fc1 comprises T366 (a knob mutation modification), and the Fc2 comprises at least one or at least two amino acid mutations selected from the group consisting of T366, L368, and Y407 (hole mutation modifications).

In some specific embodiments, the Fc1 comprises at least one or at least two amino acid mutations selected from the group consisting of T366S, L368A, and Y407V (hole mutation modifications), and the Fc2 comprises T366W (a knob mutation modification); or the Fc1 comprises T366W (a knob mutation modification), and the Fc2 comprises at least one or at least two amino acid mutations selected from the group consisting of T366S, L368A, and Y407V (hole mutation modifications).

In some specific embodiments, the IL-2 or the variant thereof is located in the polypeptide chain where Fc1 is present.

In some specific embodiments, Fc1 and Fc2 may comprise natural-non-cysteine-to-cysteine mutations in, for example, the CH3; for example, Fc1 comprises S354C, and Fc2 comprises Y349C; or Fc1 comprises Y349C, and Fc2 comprises S354C.

In some specific embodiments, Fc1 and Fc2 comprise the following amino acid mutations or combinations thereof within, for example, the Fc1 CH3/Fc2 CH3 interfaces:

T366Y/Y407T;
T366W/Y407A;
T366Y/Y407T;
T394W/F405A;
T366Y/F405A;
T394W/Y407T;
T366W/F405W/T394S/Y407A;
F405W/T394S;

D399C/K392C;
T366W/T366S/L368A/Y407V;
T366W/D399C/T366S/L368A/K392C/Y407V;
T366W/K392C/T366S/D399C/L368A/Y407V;
S354C/T366W/Y349C/T366S/L368A/Y407V;
Y349C/T366W/S354C/T366S/L368A/Y407V;
E356C/T366W/Y349C/T366S/L368A/Y407V;
Y349C/T366W/E356C/T366S/L368A/Y407V;
E357C/T366W/Y349C/T366S/L368A/Y407V; and
Y349C/T366W/E357C/T366S/L368A/Y407V.

In some specific embodiments, Fc1 and Fc2 also comprise amino acid mutations that cause the formation of an electrostatic interaction interface between Fc1 and Fc2 (e.g., CH3 and CH3). The amino acid mutations that cause the formation of the electrostatic interaction interface are selected from the group consisting of, for example, the following:

K370E/D399K/K439D/D356K/E357K/K409D;
K409D/D399K;
K409E/D399K;
K409E/D399R;
K409D/D399R;
D339K/E356K;
D399K/E356K/K409D/K392D;
D399K/E356K/K409D/K439D;
D399K/E357K/K409D/K370D;
D399K/E356K/E357K/K409D/K392D/K370D;
D399K/E357K/K409D/K392D;
K392D/K409D/D399K; and
K409D/K360D/D399K.

In some embodiments, Fc1 and/or Fc2 comprise(s) domains from different antibody subtypes, e.g., CH3 from different antibody subtypes.
Additionally, the present disclosure cites the scheme of modifying the CH3 region of the Fc region to enhance heterodimerization in WO96/27011, WO98/050431, EP1870459, WO2007/110205, WO2007/147901, WO2009/089004, WO2010/129304, WO2011/90754, WO2011/143545, WO2012058768, WO2013157954, and WO2013096291.
In some embodiments, Fc1 and/or Fc2 comprise(s) amino acid mutations for altering effector function in, for example, the CH3. Examples of the antibody effector function include, but are not limited to: C1q binding and complement-dependent cytotoxicity, Fc receptor (e.g., FcyR) binding, antibody-dependent cellular cytotoxicity (ADCC), phagocytosis, down-regulation of cell surface receptors (e.g., B-cell receptor), and B cell activation. The amino acid mutations for altering effector function are selected from the group consisting of, for example, the following:

S298A/E333A/K334A;
S239D/I332E/A330L;
S239D/I332E/G236A;
G236A/S239D/A330L/I332E;
F243L/R292P/Y300L/V305I/P396L;
K326A/E333A;
K326W/E333S;
K326M/E333S;
C221D/D222C;
S267E/H268F/S324T;
E345R;
S298A/E333A/K334A/N434A;
E294 deletion/T307P/N434Y;
T256N/A378V/S383N/N434Y;
T252L/T253S/T254F;
M252Y/S254T/T256E;
M428L/N434S;
L234A/L235A;

S228P/L235E;
L234A/L235A/P331S;
L234A/L235A/P329G;
D265A/E233P;
H268Q/V309L/A330S/P331S;
V234A/G237A/P238S/H268A/V309L/A300S/P331S;
L234A/L235A/G237A/P238S/H268A/V309L/A300S/P331S;
S228P/F234A/L235A;
D270A/P329A;
L234F/L235E;
L234F/L235E/P331S;
F241A/V264A/D265A;
N297G/D265A; and
L234Y/G236W/S298A.

In some specific embodiments, the Fc1 and/or the Fc2 comprises mutations that reduce effector function (e.g., ADCC), e.g., L234A/L235A or L234A/L235A/P329G.

In some embodiments, Fc1 and Fc2 comprise amino acid mutations for altering half-life (e.g., extending half-life) in, for example, the CH3 thereof. In some embodiments, the half-life is dependent on FcRn binding affinity. The extension of half-life can allow for a reduction in the amount of a drug given to a patient and/or a reduction in the frequency of administration. In some specific embodiments, the Fc region has the M252Y, S254T, and/or T256E mutation(s). In some embodiments, the immunoconjugate has a PEGylation modification or is conjugated/fused to (human serum) albumin or an albumin-binding protein.

In some embodiments, Fc1 and/or Fc2 comprise(s) one or more isoallotype mutations in, for example, the CH3. In some embodiments, the isoallotype mutations are D356E and L358M.

In some embodiments, Fc1 and/or Fc2 comprise(s) a C220 mutation, for example, the C220S mutation.

In some embodiments, the immunoconjugate comprises a polypeptide chain combination of the following amino acid sequences: SEQ ID NOs: 73 and 74; SEQ ID NOs: 75 and 76; SEQ ID NOs: 77 and 78; SEQ ID NOs: 79 and 80; SEQ ID NOs: 43 and 44; SEQ ID NOs: 45 and 46; SEQ ID NOs: 47, 44, and 48; SEQ ID NOs: 47, 46, and 48; SEQ ID NOs: 49, 50, and 48; SEQ ID NOs: 51 and 46; SEQ ID NOs: 102 and 103; or an amino acid sequence having at least 80%, at least 85%, at least 90%, at least 95%, at least 96%, at least 97%, at least 98%, or at least 99% identity to any one of the polypeptide chain combinations described above.

Exemplary Immunoconjugates

Regarding immunoconjugates, the present disclosure provides the following embodiments:

Embodiment 1. An immunoconjugate, comprising:

1) a PD-1-binding domain, and an IL-2 variant, wherein the IL-2 variant comprises a mutation selected from the group consisting of N88D and D20A; or
2) a PD-1-binding domain, and an IL-2 variant, wherein the IL-2 variant has a reduced or eliminated affinity for the high-affinity receptors (IL-2R$\alpha/\beta/\gamma$) as compared to wild-type IL-2 and has a reduced affinity for the intermediate-affinity receptors (IL-2R$\beta/\gamma$) as compared to wild-type IL-2, and the affinity for the high-affinity receptors is reduced more than the affinity for the intermediate-affinity receptors; for example, the IL-2 variant comprises a mutation selected from the group consisting of N88D and D20A.

Embodiment 2. The immunoconjugate according to embodiment 1, wherein the IL-2 variant comprises a mutation selected from the group consisting of F42, Y45, and L72 or any combination thereof;

for example, the IL-2 variant comprises a mutation selected from the group consisting of F42A, Y45A, and L72G or any combination thereof;
as yet another example, the IL-2 variant comprises the F42A/L72G, F42A/Y45A, Y45A/L72G, or F42A/Y45A/L72G mutations.

Embodiment 3. The immunoconjugate according to embodiment 1 or 2, wherein the IL-2 variant comprises a mutation selected from the group consisting of N26Q, N29S, N30S, N71Q, Q11C and L132C, L70C and P82C, and G27C and F78C or any combination thereof;

for example, the IL-2 variant comprises a mutation or combination selected from the group consisting of the following: N26Q, N29S, N30S, N26Q/N29S/N71Q, N26Q/N29S, N26Q/N30S, N26Q/N30S/Q11C/ L132C, N26Q/N29S/L70C/P82C, and N26Q/N30S/G27C/F78C.

Embodiment 4. An immunoconjugate, comprising:

an antigen-binding domain, and an IL-2 or a variant thereof, wherein the IL-2 variant comprises a first class of mutation that makes the IL-2 variant have a reduced affinity for the intermediate-affinity receptors (IL-2Rβ/γ) as compared to wild-type IL-2;

for example, the first class of mutation is selected from the group consisting of: L12, E15, H16, L18, L19, D20, M23, Q22, K43, V69, D84, S87, N88, V91, I92, E95, T123, Q126, S127, I129, and S130;

as another example, the first class of mutation is selected from the group consisting of: L12R, L12K, L12E, L12Q, E15Q, E15R, E15A, E15S, H16A, H16E, H16D, H16G, H16S, H16T, H16V, H16P, D20A, D20H, D20Y, D20N, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88T, N88R, N88I, V91A, V91T, V91E, I92A, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, and S127Q;

as another example, the first class of mutation is selected from the group consisting of: N88D, N88R, D20A, D20N, V91T, Q126D, and H16A/D84S;

as yet another example, the first class of mutation is selected from the group consisting of N88D and D20A.

Embodiment 5. The immunoconjugate according to embodiment 4, wherein the IL-2 variant comprises a second class of mutation that makes the IL-2 variant have a reduced or eliminated affinity for the high-affinity receptors (IL-2Rα/β/γ) as compared to wild-type IL-2;

for example, the second class of mutation is selected from the group consisting of R38, E62, P65, E68, F42, Y45, and L72, or any combination thereof;

as another example, the second class of mutation is selected from the group consisting of R38A, R38D, R38E, E62Q, P65R, P65E, P65K, P65H, P65Y, P65Q, P65D, P65N, E68A, E68Q, E68K, E68R, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, L72G, L72A, L72S, L72T, L72Q, L72E, L72N, L72D, L72R, and L72K;

as yet another example, the second class of mutation is selected from the group consisting of F42A, Y45A, L72G, F42A/L72G, F42A/Y45A, Y45A/L72G, and F42A/Y45A/L72G. Embodiment 6. The immunoconjugate according to embodiment 4 or 5, wherein the IL-2 variant comprises a third class of mutation that makes the IL-2 variant have increased stability as compared to wild-type IL-2;

for example, the third class of mutation is selected from the group consisting of N26, N29, N30, N71, Q11, L132, L70, P82, G27, and F78 or any combination thereof;

as another example, the third class of mutation is selected from the group consisting of N26Q, N29S, N30S, N71Q, Q11C/L132C, L70C/P82C, and G27C/F78C or any combination thereof;

as yet another example, the third class of mutation is selected from the group consisting of: N26Q, N29S, N30S, N26Q/N29S/N71Q, N26Q/N29S, N26Q/N30S, N26Q/N30S/ Q11C/L132C, N26Q/N29S/L70C/P82C, and N26Q/N30S/G27C/F78C.

Embodiment 7. An immunoconjugate, comprising: a PD-1-binding domain, and an IL-2 or a variant thereof, wherein the PD-1-binding domain PD-1-binding domain comprises at least one immunoglobulin single variable domain, and the immunoglobulin single variable domain comprises:

a CDR1, a CDR2, and a CDR3 from the amino acid sequence set forth in any one of SEQ ID NOs: 3, 13-15, and 17-35, or

a CDR1, a CDR2, and a CDR3 from the amino acid sequence set forth in any one of SEQ ID NOs: 2 and 16;

the CDR1, CDR2, and CDR3 are defined according to the Kabat, IMGT, Chothia, AbM, or Contact numbering scheme;

for example, according to the Kabat numbering scheme, the amino acid sequences of the CDR1, CDR2, and CDR3 of the immunoglobulin single variable domain are set forth in SEQ ID NOs: 7, 36, and 37, respectively, or SEQ ID NOs: 4, 5, and 6, respectively;

as another example, the amino acid sequence of the CDR1 of the immunoglobulin single variable domain is set forth in SEQ ID NO: 7, the amino acid sequence of the CDR2 is set forth in any one of SEQ ID NOs: 8 and 38-40, and the amino acid sequence of the CDR3 is set forth in any one of SEQ ID NOs: 9, 41, and 42;

as yet another example, the amino acid sequences of the CDR1, CDR2, and CDR3 of the immunoglobulin single variable domain are set forth in SEQ ID NOs: 7, 38, and 41, respectively.

Embodiment 8. The immunoconjugate according to any one of embodiments 1 to 7, wherein the IL-2 variant comprises a mutation combination selected from the group consisting of groups 1) and 2):

group 1) mutations are selected from the group consisting of: F42A/L72G, F42A/Y45A, Y45A/L72G, and F42A/Y45A/L72G;

group 2) mutations are selected from the group consisting of: N88R, N88G, N88I, N88D, D20H, D20Y, V91T, Q126L, Q126D, and H16A/D84S;

for example, the IL-2 variant comprises the following mutation combination: F42A/L72G/N88D, F42A/L72G/N88R, F42A/L72G/D20A, F42A/L72G/D20N, F42A/L72G/V91T, F42A/L72G/Q126D, or F42A/L72-G/H16A/D84S.

Embodiment 9. The immunoconjugate according to embodiment 8, wherein the IL-2 variant also comprises group 3) mutations, and the group 3) mutations are selected from the group consisting of: N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/ G27C/F78C;

for example, the IL-2 variant comprises the following mutation combination:

F42A/L72G/N88D/N26Q/N29S/N71Q,
F42A/L72G/N88R/N26Q/N29S/N71Q,
F42A/L72G/D20A/N26Q/N29S/N71Q,
F42A/L72G/D20N/N26Q/N29S/N71Q,
F42A/L72G/V91T/N26Q/N29S/N71Q,
F42A/L72G/Q126D/N26Q/N29S/N71Q, or
F42A/L72G/H16A/D84S/N26Q/N29S/N71Q.

Embodiment 10. The immunoconjugate according to any one of embodiments 1 to 9, wherein the IL-2 variant comprises a mutation selected from the group consisting of T3 and/or C125 or a combination thereof;

for example, the T3A and/or C125A, C125S, C125T, C125V mutations.

Embodiment 11. The immunoconjugate according to any one of embodiments 1 to 10, wherein the IL-2 variant has the amino acid sequence set forth in any one of SEQ ID NOs: 60-68.

Embodiment 12. In the immunoconjugate according to any one of embodiments 4 to 6 and 8 to 10,

1) the antigen is selected from the group consisting of:

1-1) antigens on tumor cells or on immune cells in the tumor microenvironment;

1-2) PD-1, CTLA-4, LAG3, TIM3, 4-1BB, OX40, GITR, CD8a, CD8b, CD4, NKp30, NKG2A, TIGIT, TGFβR1, TGFβR2, Fas, NKG2D, NKp46, PD-L1, CD107a, ICOS, TNFR2, FAP, TNC A1, TNC A2, CEA, EDB, MCSP, or CD16a; and

1-3) immune checkpoint antigens, such as PD-1, PD-L1, and CTLA-4;

2) the antigen-binding domain comprises a VH domain and a VL domain, or a HCDR1, a HCDR2, and a HCDR3 of a VH and a LCDR1, a LCDR2, and a LCDR3 of a VL, of an antibody selected from the group consisting of the following:

PD-1 antibodies (e.g., pembrolizumab, nivolumab, sintilimab, tislelizumab, cemiplimab, toripalimab, camrelizumab, penpulimab, zimberelimab, sasanlimab, spartalizumab, retifanlimab, balstilimab, cetrelimab, dostarlimab (TSR-042), CS-1003, SCT-I10A, SSI-361, MEDI-0680(AMP-514), BAT-1306, JTX-4014, JTX-4014, BI-754091, and AK-103);

CTLA-4 antibodies (e.g., tremelimumab and ipilimumab);

4-1BB antibodies (e.g., urelumab and utomilumab); and

PD-L1 antibodies (e.g., durvalumab, atezolizumab, durvalumab, avelumab, adebrelimab, envafolimab, STI-A1110, CS-1001, TQB-2450, KL-A167, IMC-001, and CX-072).

Embodiment 13. The immunoconjugate according to any one of embodiments 1 to 3 and 12, wherein the PD-1-binding domain comprises at least one immunoglobulin single variable domain, and the immunoglobulin single variable domain comprises:

a CDR1, a CDR2, and a CDR3 from the amino acid sequence set forth in any one of SEQ ID NOs: 3, 13-15, 17-35, and 88-92, or

a CDR1, a CDR2, and a CDR3 from the amino acid sequence set forth in any one of SEQ ID NOs: 2 and 16; the CDR1, CDR2, and CDR3 are defined according to the Kabat, IMGT, Chothia, AbM, or Contact numbering scheme;

for example, according to the Kabat numbering scheme, the amino acid sequences of the CDR1, CDR2, and CDR3 of the immunoglobulin single variable domain are set forth in

SEQ ID NOs: 97, 100, and 101, respectively,
SEQ ID NOs: 7, 36, and 37, respectively,
SEQ ID NOs: 97, 98, and 99, respectively, or
SEQ ID NOs: 4, 5, and 6, respectively;

as another example, the amino acid sequence of the CDR1 of the immunoglobulin single variable domain is set forth in SEQ ID NO: 7 or 93, the amino acid sequence of the CDR2 is set forth in any one of SEQ ID NOs: 8, 38-40, and 94-95, and the amino acid sequence of the CDR3 is set forth in any one of SEQ ID NOs: 9, 41, 42, and 96; as another example, the immunoglobulin single variable domain is selected from the group consisting of any one of the following:

1) comprising a CDR1, a CDR2, and a CDR3 set forth in SEQ ID NOs: 7, 38, and 41;
2) comprising a CDR1, a CDR2, and a CDR3 set forth in SEQ ID NOs: 7, 38, and 96;
3) comprising a CDR1, a CDR2, and a CDR3 set forth in SEQ ID NOs: 7, 94, and 41;
4) comprising a CDR1, a CDR2, and a CDR3 set forth in SEQ ID NOs: 93, 94, and 41;
5) comprising a CDR1, a CDR2, and a CDR3 set forth in SEQ ID NOs: 93, 94, and 96;
6) comprising a CDR1, a CDR2, and a CDR3 set forth in SEQ ID NOs: 93, 95, and 96;
7) comprising a CDR1, a CDR2, and a CDR3 set forth in SEQ ID NOs: 7, 8, and 9;
8) comprising a CDR1, a CDR2, and a CDR3 set forth in SEQ ID NOs: 7, 8, and 42;
9) comprising a CDR1, a CDR2, and a CDR3 set forth in SEQ ID NOs: 7, 38, and 6;
10) comprising a CDR1, a CDR2, and a CDR3 set forth in SEQ ID NOs: 7, 39, and 9;
11) comprising a CDR1, a CDR2, and a CDR3 set forth in SEQ ID NOs: 7, 40, and 6;
12) comprising a CDR1, a CDR2, and a CDR3 set forth in SEQ ID NOs: 7, 8, and 41;
13) comprising a CDR1, a CDR2, and a CDR3 set forth in SEQ ID NOs: 7, 8, and 42;
14) comprising a CDR1, a CDR2, and a CDR3 set forth in SEQ ID NOs: 7, 38, and 41;
15) comprising a CDR1, a CDR2, and a CDR3 set forth in SEQ ID NOs: 7, 38, and 42;
16) comprising a CDR1, a CDR2, and a CDR3 set forth in SEQ ID NOs: 7, 39, and 41;
17) comprising a CDR1, a CDR2, and a CDR3 set forth in SEQ ID NOs: 7, 39, and 42;
18) comprising a CDR1, a CDR2, and a CDR3 set forth in SEQ ID NOs: 7, 40, and 41; and
19) comprising a CDR1, a CDR2, and a CDR3 set forth in SEQ ID NOs: 7, 40, and 42;

as yet another example, the amino acid sequences of the CDR1, CDR2, and CDR3 of the immunoglobulin single variable domain are set forth in SEQ ID NOs: 7, 38, and 41, respectively.

Embodiment 14. The immunoconjugate according to embodiment 7 or 13, wherein the immunoglobulin single variable domain is:

1) modified by humanization, affinity maturation, T cell epitope removal, reduction of antibody deamidation, and/or reduction of antibody isomerization; for example, the heavy chain framework region of the human germline template used in the humanization modification process is IGHV3-23 *01 or IGHV3-23*04;
2) a camelid antibody, a chimeric antibody, a humanized antibody, a fully human antibody, or an antigen-binding fragment thereof.

Embodiment 15. The immunoconjugate according to embodiment 13 or 14, wherein the immunoglobulin single variable domain comprises

the amino acid sequence set forth in any one of SEQ ID NOs: 3, 13-15, 17-35, and 88-92; or
the amino acid sequence set forth in any one of SEQ ID NOs: 2 and 16; or an amino acid sequence having at least 90% sequence identity to any one of the aforementioned sequences.

Embodiment 16. The immunoconjugate according to any one of embodiments 1 to 3 and 12, wherein the PD-1-binding domain comprises a heavy chain variable region (VH) and a light chain variable region (VL), wherein the heavy chain variable region comprises a HCDR1, a HCDR2, and a HCDR3 of the amino acid sequences set forth in SEQ ID NOs: 54, 55, and 56, and the light chain variable region comprises a LCDR1, a LCDR2, and a LCDR3 of the amino acid sequences set forth in SEQ ID NOs: 57, 58, and 59;

for example, the heavy chain variable region is a heavy chain variable region from a Fab heavy chain of the amino acid sequence set forth in SEQ ID NO: 52, and the light chain variable region comprises a light chain variable region from a Fab light chain of the amino acid sequence set forth in SEQ ID NO: 53.

Embodiment 17. The immunoconjugate according to any one of the preceding embodiments, wherein the immunoconjugate also comprises a human immunoglobulin Fc region; for example, the Fc region is an Fc region of human IgG1, IgG2, IgG3, or IgG4.

Embodiment 18. The immunoconjugate according to embodiment 17, wherein the Fc region of human IgG1 comprises one or more amino acid mutations that reduce binding to Fc receptors (FcRs, e.g., FcγR) and/or reduce effector functions;

for example, the Fc region of human IgG1 comprises one or more amino acid mutations that reduce binding to Fcγ receptors (FcγRs) and/or reduce ADCC effects;

as another example, the Fc region of human IgG1 comprises L234, L235, and P329 mutations or any combination thereof;

as yet another example, the Fc region of human IgG1 comprises the L234A/L235A or L234A/L235A/P329G mutations.

Embodiment 19. The immunoconjugate according to embodiment 17 or 18, wherein the Fc region comprises a knob-into-hole modification that promotes the association (or heterodimerization) of a first subunit and a second subunit of the Fc region;

as described herein, the knob-into-hole modification refers to, for example, an amino acid residue in the CH3 region of the first subunit of the Fc region is replaced with an amino acid residue having a larger side-chain volume, thereby forming a protuberance within the CH3 region of the first subunit which is positionable in a cavity within the CH3 region of the second subunit, and an amino acid residue in the CH3 region of the second subunit of the Fc domain is replaced with an amino acid residue having a smaller side-chain volume, thereby forming a cavity within the CH3 region of the second subunit in which the protuberance within the CH3 region of the first subunit is positionable; as another example, the amino acid residue having a larger side-chain volume is selected from the group consisting of R, F, Y, and W, and the amino acid residue having a smaller side-chain volume is selected from the group consisting of A, S, T, and V.

Embodiment 20. The immunoconjugate according to embodiment 19, wherein

the first subunit of the Fc region comprises a T366 mutation, and the second subunit comprises a mutation selected from the group consisting of T366, L368, and Y407 or any combination thereof;

the first subunit of the Fc region comprises an S354 or E356 mutation, and the second subunit comprises a mutation selected from the group consisting of Y349; or

the first subunit of the Fc region comprises an S354 mutation and a T366 mutation, and

the second subunit comprises a Y349 mutation, a T366 mutation, an L368 mutation, and a Y407 mutation;

for example,

the first subunit of the Fc region comprises the T366W mutation, and the second subunit comprises a mutation selected from the group consisting of T366S, L368A, and Y407V or any combination thereof;

the first subunit of the Fc region comprises the S354C or E356C mutation, and the second subunit comprises a mutation selected from the group consisting of Y349C; or

the first subunit of the Fc region comprises the S354C/T366W mutations, and the second subunit comprises the Y349C/T366S/L368A/Y407V mutations.

Embodiment 21. The immunoconjugate according to embodiment 19 or 20, wherein the second subunit of the Fc region also comprises an H435 mutation and/or an R436 mutation, preferably the H435R/Y436F mutations, the H435R mutation, or the H435K mutation.

Embodiment 22. The immunoconjugate according to any one of embodiments 17 to 21, wherein the Fc region of human IgG1 comprises a mutation that enhances FcRn-mediated recycling and/or increases half-life;

for example, the Fc region of human IgG1 comprises an M252 mutation and/or an M428 mutation;

for example, the Fc region of human IgG1 comprises the M252Y/M428V or M252Y/M428L mutations.

Embodiment 23. The immunoconjugate according to any one of embodiments 17 to 22, wherein the first subunit and/or the second subunit of the Fc region of human IgG1 comprise(s) a C220 mutation, for example, the C220S mutation.

Embodiment 24. The immunoconjugate according to any one of the preceding embodiments, wherein the PD-1-binding domain or the antigen-binding domain is located at the N-terminus of the Fc region, and the IL-2 variant is located at the N-terminus or C-terminus of the Fc region;

for example, the PD-1-binding domain or the antigen-binding domain is located at the N-terminus of the Fc region, and the IL-2 variant is located at the C-terminus of the Fc region.

Embodiment 25. The immunoconjugate according to any one of the preceding embodiments, wherein the valence ratio of the PD-1-binding domain or the antigen-binding domain to the IL-2 or the variant thereof is between 6:1 and 1:3 (e.g., 4:1 to 1:2), preferably 4:1, 2:1, 3:1, or 1:1; or, one immunoconjugate comprises 1 or 2 IL-2 or variants thereof and 1, 2, 3, 4, 5, or 6 PD-1-binding domains or antigen-binding domains.

Embodiment 26. The immunoconjugate according to any one of embodiments 1 to 3, 7, and 11 to 25, comprising a polypeptide chain combination set forth in any one of the following I)-VI):

I) a first polypeptide chain: [PD-1-binding domain 1]-[the first subunit of the Fc region]-[linker 1]-[IL-2 or the variant thereofJ, and
a second polypeptide chain: [PD-1-binding domain 1]-[the second subunit of the Fc region];

II) a first polypeptide chain: [PD-1-binding domain 1]-[linker 2]-[PD-1-binding domain 1]-[the first subunit of the Fc region]-[linker 1]-[IL-2 or the variant thereofJ, and
a second polypeptide chain: [PD-1-binding domain 1]-[linker 2]-[PD-1-binding domain 1]-[the second subunit of the Fc region];

III) a first polypeptide chain: [the Fab heavy chain of PD-1-binding domain 2]-[the first subunit of the Fc region]-[linker 1]-[IL-2 or the variant thereofJ,

a second polypeptide chain: [PD-1-binding domain 1]-[the second subunit of the Fc region], and
a third polypeptide chain: [the Fab light chain of PD-1-binding domain 2];

IV) a first polypeptide chain: [the Fab heavy chain of PD-1-binding domain 2]-[the first subunit of the Fc region]-[linker 1]-[IL-2 or the variant thereofJ,

a second polypeptide chain: [PD-1-binding domain 1]-[linker 2]-[PD-1-binding domain 1]-[the second subunit of the Fc region], and
a third polypeptide chain: [the Fab light chain of PD-1-binding domain 2];

V) a first polypeptide chain: [PD-1-binding domain 1]-[linker 2]-[the Fab heavy chain of PD-1-binding domain 2]-[the first subunit of the Fc region]-[linker 1]-[IL-2 or the variant thereofJ,

a second polypeptide chain: [PD-1-binding domain 1]-[linker 2]-[the Fab light chain of PD-1-binding domain 2]-[the second subunit of the Fc region], and
a third polypeptide chain: [the Fab light chain of PD-1-binding domain 2]; and

VI) a first polypeptide chain: [IL-2 or the variant thereof]-[linker 3]-[the first subunit of the Fc region], and

a second polypeptide chain: [PD-1-binding domain 1]-[linker 2]-[PD-1-binding domain 1]-[the second subunit of the Fc region];
wherein - represents a peptide bond; the linkers are polypeptides capable of fulfilling the function of linking, and linker 1, linker 2, and linker 3 may be identical or different; [PD-1-binding domain 1] is selected from the group consisting of any of the PD-1-binding domains according to embodiments 11 to 13; [PD-1-binding domain 2] is selected from the group consisting of any of the PD-1-binding domains according to embodiments 14; for example, the linkers are $(G_xS)_y$ linkers, wherein x is selected from the group consisting of integers of 1-5, and y is selected from the group consisting of integers of 1-6; as another example, linker 1 is $(G_4S)_3$ (SEQ ID NO: 104), linker 2 is $(G_4S)_2$ (SEQ ID NO: 105), and linker 3 is $G_4S$ (SEQ ID NO: 106).

Some other linkers include, but are not limited to, amino acid sequences represented by $(G_mS_n)_h$ or $(GGNGT)_h$ (SEQ ID NO: 107) or $(YGNGT)_h$ (SEQ ID NO: 108) or $(EPKSS)_h$ (SEQ ID NO: 109), wherein m and n are each independently selected from the group consisting of integers of 1-8 (e.g., 1, 2, 3, 4, 5, 6, 7, or 8), and h is independently selected from the group consisting of integers of 1-20 (e.g., 1, 2, 3, 4, 5, 6, 7, 8, 9, 10, 11, 12, 13, 14, 15, 16, 17, 18, 19, or 20).

The order of the polypeptide chains described above is from N-terminus to C-terminus. Embodiment 27. The immunoconjugate according to any one of the preceding embodiments, comprising a polypeptide chain combination of the following amino acid sequences:

    SEQ ID NOs: 73 and 74;
    SEQ ID NOs: 75 and 76;
    SEQ ID NOs: 77 and 78;
    SEQ ID NOs: 79 and 80;
    SEQ ID NOs: 43 and 44;
    SEQ ID NOs: 45 and 46;
    SEQ ID NOs: 47, 44, and 48;
    SEQ ID NOs: 47, 46, and 48;
    SEQ ID NOs: 49, 50, and 48;
    SEQ ID NOs: 51 and 46;
    SEQ ID NOs: 102 and 103.
    or an amino acid sequence having at least 80%, at least 85%, at least 90%, at least 95%, at least 96%, at least 97%, at least 98%, or at least 99% identity to any one of the polypeptide chain combination described above.

Unless otherwise specified, all the numbering schemes for the positions described above are the Kabat EU index.

The present disclosure provides a fusion protein that comprises or is any of the aforementioned immunoconjugates.

Polynucleotide and Vector

The present disclosure provides a polynucleotide encoding the PD-1 antibody or the antigen-binding fragment thereof, the IL-2 variant, and the immunoconjugate of the present disclosure. The nucleic acid of the present disclosure may be an RNA, DNA, or cDNA. According to some embodiments of the present disclosure, the nucleic acid of the present disclosure is a substantially isolated nucleic acid.

The nucleic acid of the present disclosure may also be present in the form of a vector, may be present in the vector, and/or may be part of the vector. The vector is, e.g., a plasmid, cosmid, YAC, or viral vector. The vector may especially be an expression vector, i.e., a vector that provides for the *in vitro* and/or *in vivo* (i.e., in suitable host cells, host organisms, and/or expression systems) expression of the PD-1 antibody or the antigen-binding fragment thereof. The expression vector generally comprises at least one of the nucleic acids of the present disclosure, which is operably linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expressing the PD-1 antibody or the antigen-binding fragment thereof of the present disclosure are, for example, promoters, enhancers, terminators, integration factors, selection markers, leader sequences or reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

Host Cell

The present disclosure provides a recombinant host cell that expresses or is capable of expressing one or more of the PD-1 antibody or the antigen-binding fragment thereof, the IL-2 variant, and the immunoconjugate of the present disclosure and/or comprises the nucleic acid or vector of the present disclosure. In some embodiments, the host cell is a bacterial cell, a fungal cell or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus;* or cells of the species *Saccharomyces* (e.g., *Saccharomyces cerevisiae*), *Schizosaccharomyces* (e.g., *Schizosaccharomyces pombe*), *Pichia* (*Pichia pastoris* and *Pichia methanolica*), and *Hansenula.*

Mammalian cells include, e.g., HEK293 cells, CHO cells, BHK cells, HeLa cells, COS cells, and the like.

However, amphibian cells, insect cells, plant cells, and any other cells used in the art for expressing heterologous proteins may also be used in the present disclosure.

Preparation Method

The present disclosure provides a method for preparing a PD-1 antibody or an antigen-binding fragment thereof, an IL-2 variant, or an immunoconjugate, comprising: expressing the target protein in a host cell as described previously and isolating the target protein from the host cell. Optionally, a purification step can also be included. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

Methods for producing and purifying antibodies are well known in the prior art and can be found in, for example, "Antibodies: A Laboratory Manual", Cold Spring Harbor Press (chapters 5-8 and 15).

The engineered antibody or antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free culture medium of a bioreactor to produce antibodies. The culture solution with the secreted antibody can be purified and collected by conventional techniques. The antibody can be filtered and concentrated by conventional methods. Soluble mixtures and multimers can also be removed by conventional methods, such as molecular sieves and ion exchange.

Composition

The present disclosure provides a composition comprising the aforementioned PD-1 antibody or the antigen-binding fragment thereof, the IL-2 variant, and/or the immunoconjugate. For example, provided is a pharmaceutical composition, comprising an effective cancer treating, alleviating, or preventing amount of the PD-1 antibody or the antigen-binding fragment thereof described above, the immunoconjugate described above, and at least one pharmaceutically acceptable excipient, diluent, or carrier.

In some specific embodiments, a unit dose of the pharmaceutical composition may comprise 0.01 to 99 wt% of the PD-1 antibody or the antigen-binding fragment thereof, the IL-2 variant, and/or the immunoconjugate, or the amount of the PD-1 antibody or the antigen-binding fragment thereof, the IL-2 variant, and/or the immunoconjugate contained in a unit dose of the pharmaceutical composition is 0.1-2000 mg; in some embodiments, 1-1000 mg.

In some embodiments, provided is a product, comprising the aforementioned PD-1 antibody or the antigen-binding fragment thereof, the IL-2 variant, and/or the immunoconjugate. Optionally, the product comprises a container and a label. Examples of containers are bottles, syringes, and test tubes. The container accommodates a composition effective in treating a disorder. The label on or associated with the container indicates that the composition is used for treating the disorder of choice. The composition comprises the aforementioned PD-1 antibody or the antigen-binding fragment thereof and/or the immunoconjugate.

Illustratively, provided is a composition, comprising:

any of the PD-1 antibodies or the antigen-binding fragments thereof provided by the present disclosure;
any of the IL-2 variants provided by the present disclosure; and/or
any of the immunoconjugates provided by the present disclosure;
optionally, when the composition is a pharmaceutical composition, it also comprises at least one pharmaceutically acceptable excipient, diluent, or carrier.

Treatment Method and Pharmaceutical Use

The present disclosure provides use of the aforementioned PD-1 antibody or the antigen-binding fragment thereof, the IL-2 variant, the immunoconjugate, the polynucleotide, the composition (including the pharmaceutical composition) in at least one of the following:

1) treating a cancer;
2) preparing a medicament for treating a cancer;
3) activating cytotoxic T cells (CTLs);

4) preparing a medicament for activating cytotoxic T cells (CTLs);

5) promoting the proliferation of CD4$^+$ and/or CD8$^+$ T cells;

6) preparing a medicament for promoting the proliferation of CD4$^+$ and/or CD8$^+$ T cells;

7) making cytotoxic T cells (CTLs) with high PD-1 antibody expression have higher cell activation efficiency than cytotoxic T cells (CTLs) with low or no PD-1 antibody expression; and

8) making CD4$^+$ and/or CD8$^+$T cells with high PD-1 antibody expression have higher cell proliferation efficiency than CD4$^+$ and/or CD8$^+$T cells with low or no PD-1 antibody expression.

The present disclosure provides a method for treating, alleviating, preventing, or diagnosing a disease or disorder by using the aforementioned PD-1 antibody or the antigen-binding fragment thereof, the IL-2 variant, the immunoconjugate, the polynucleotide, or the composition (including the pharmaceutical composition).

In some embodiments, provided is a method for ameliorating, alleviating, treating, or preventing a disease, comprising administering to a subject an effective ameliorating, alleviating, treating, or preventing amount of the aforementioned PD-1 antibody or the antigen-binding fragment thereof, the immunoconjugate, the polynucleotide, or the composition (including the pharmaceutical composition).

In some embodiments, provided is use of the PD-1 antibody or the antigen-binding fragment thereof, the IL-2 variant, or the immunoconjugate of the present disclosure for the preparation of a medicament for ameliorating, alleviating, treating, or preventing a disease.

In some embodiments, the aforementioned disease is a proliferative disorder or any other disease or disorder characterized by uncontrolled cell growth (e.g., a cancer; in the present disclosure, cancer and tumor are used interchangeably), e.g., a disease associated with PD-L1 overexpression, or a related disease responsive to treatment with a PD-1 antibody or an antigen-binding fragment thereof, e.g., a cancer.

In some embodiments, the aforementioned cancer is a solid tumor or hematological tumor.

In some embodiments, the aforementioned cancer is advanced or metastatic.

In some embodiments, the aforementioned cancer is selected from the group consisting of the following or a combination thereof: lung cancer, prostate cancer, breast cancer, head and neck cancer, esophageal cancer, stomach/-gastric cancer, colon cancer, colorectal cancer, bladder cancer, cervical cancer, uterine cancer, ovarian cancer, liver cancer, melanoma, renal cancer, squamous cell carcinoma, hematological cancer, and any other diseases or disorders characterized by uncontrolled cell growth.

For example, the cancer is selected from the group consisting of prostate cancer, liver cancer (HCC), colorectal cancer, ovarian cancer, endometrial cancer, breast cancer, triple-negative breast cancer, pancreatic cancer, stomach/-gastric cancer, cervical cancer, head and neck cancer, thyroid cancer, testicular cancer, urothelial cancer, lung cancer (small cell lung cancer or non-small cell lung cancer), melanoma, non-melanoma skin cancer (squamous and basal cell carcinoma), neuroglioma, renal carcinoma (RCC), lymphoma (NHL or HL), acute myelogenous leukemia (AML), T-cell acute lymphoblastic leukemia (T-ALL), diffuse large B-cell lymphoma, testis germ cell tumor, mesothelioma, esophageal cancer, Merkel cells cancer, MSI-high cancer, KRAS-mutant tumor, adult T-cell leukemia/lymphoma, and myelodysplastic syndrome (MDS).

For example, the cancer is selected from the group consisting of basal cell carcinoma, biliary tract cancer, bladder cancer, bone cancer, brain and central nervous system cancer, breast cancer, peritoneal cancer, cervical cancer, choriocarcinoma, colorectal cancer, connective tissue cancer, digestive system cancer, endometrial cancer, esophageal cancer, eye cancer, head and neck cancer, stomach/gastric cancer, gastrointestinal cancer, glioblastoma, liver cancer, liver tumor, intraepithelial tumor, kidney cancer or renal cancer, laryngeal cancer, liver cancer, lung cancer, small cell lung cancer, non-small cell lung cancer, lung adenocarcinoma, lung squamous carcinoma, melanoma, myeloma, neuroblastoma, oral cancer, ovarian cancer, pancreatic cancer, prostate cancer, retinoblastoma, rhabdomyosarcoma, rectal cancer, respiratory system cancer, salivary gland cancer, sarcoma, skin cancer, squamous cell carcinoma, stomach/gastric cancer, testicular cancer, thyroid cancer, uterine or endometrial cancer, urinary system cancer, vulval cancer, lymphoma, lymphoma, Hodgkin lymphoma, non-Hodgkin lymphoma, B-cell lymphoma, low-grade/follicular non-Hodgkin lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphoma, Waldenström macroglobulinemia, chronic lymphocytic leukemia (CLL), acute lymphoblastic leukemia (ALL), hairy cell leukemia, and chronic myeloblastic leukemia. In some embodiments, the aforementioned subject has a PD-L1-associated condition. In some specific embodiments, the subject's condition includes a cancer that abnormally expresses or does not abnormally express PD-L1, and further includes non-metastatic or non-infiltrative and infiltrative or metastatic cancers.

In some embodiments, provided are a method for activating the immune system of a subject, a method for activating cytotoxic T cells (CTLs) of a subject *in vivo* or *in vitro,* a method for increasing the proliferation of CD4$^+$ and/or CD8$^+$ T cells *in vivo* or *in vitro,* a method for activating NK cells of a subject *in vivo* or *in vitro,* a method for activating γδ T cells of a subject *in vivo* or *in vitro,* a method for activating Th1 cells of a subject *in vivo* or *in vitro,* a method for activating, reducing, or eliminating the cell number and/or activity of at least one type of regulatory T cells (Tregs) in a subject, a method for increasing IFN-y production and/or proinflammatory cytokine secretion in a subject, and a method for blocking or inhibiting the interaction between PD-1 and PD-L1/PD-L2 in a subject, each comprising administering to the subject an effective amount of the aforementioned PD-1 antibody or the antigen-binding fragment thereof, the IL-2 variant, the immunoconjugate, the polynucleotide, or the composition (including the pharmaceutical composition) of the present disclosure.

Detection

The present disclosure provides detection use of the PD-1 antibody or the antigen-binding fragment thereof, the immunoconjugate, the polynucleotide, or the composition. The present disclosure also provides a method, system, or device for detecting PD-1 *in vivo* or *in vitro,* comprising treating a sample with the aforementioned binding protein, polynucleotide, or composition of the present disclosure.

In some embodiments, the method, system, or device for *in vitro* detection may comprise, for example,

(1) contacting the sample with the PD-1 antibody or the antigen-binding fragment thereof, the immunoconjugate, the polynucleotide, or the composition;
(2) detecting a complex formed between the aforementioned binding protein or polynucleotide and the sample; and/or
(3) contacting a reference sample (e.g., a control sample) with the binding protein or nucleic acid; and
(4) determining the extent of formation of the complex by comparison with the reference sample. A change (e.g., a statistically significant change) in the formation of the complex in the sample or subject as compared to a control sample or subject indicates the presence of PD-1 in the sample.

In some other embodiments, the method, system, or device for *in vivo* detection may comprise:

(1) administering to a subject the aforementioned binding protein or polynucleotide; and
(2) detecting the formation of a complex between the aforementioned binding protein or polynucleotide and the subject.

The detection may include determining the location or time at which the complex is formed. The aforementioned binding protein or polynucleotide may be labeled with a detectable substance, and detection of a substance capable of binding to the protein or polynucleotide (e.g., PD-1) is achieved by detection of the label. Suitable detectable substances include various enzymes, prosthetic groups, fluorescent substances, luminescent substances, and radioactive substances. The formation of the complex from the binding protein or polynucleotide and PD-1 can be detected by measuring or visualizing substances that bind or do not bind to PD-1. Conventional detection assays may be used, such as enzyme-linked immunosorbent assay (ELISA), radioimmunoassay (RIA), or tissue immunohistochemistry. For detection purposes, the binding protein or polynucleotide of the present disclosure may be labeled with a fluorophore chromophore. In some embodiments, also provided is a kit, comprising the aforementioned binding protein or polynucleotide; the kit may further comprise instructions for diagnostic use. The kit may also comprise at least one additional reagent, such as a label or an additional diagnostic agent. For *in vivo* use, the antibody may be formulated into a pharmaceutical composition.

**BRIEF DESCRIPTION OF THE DRAWINGS**

FIGs. 1A-1B show the activity of anti-PD-1 nanobodies as measured using a PD-1/PD-L1 NFAT reporter gene system, with pembrolizumab as a positive control and human IgG4 as a negative control, wherein FIG. 1A shows the results for A6_IgG4 and A17_IgG4, and FIG. 1B shows the results for A17h1_IgG4, A17m09_hIgG4, A17m0901_hIgG4, A17m0902_hIgG4, A17m0903_hIgG4, and A17m0905_hIgG4.

FIG. 2 shows the extent to which A17m09_hIgG4 activates T cells as measured through the release of cytokines in a DC cell:T cell mix lymphocyte reaction, with pembrolizumab as a positive control and hIgG4 as a negative control.

FIGs. 3A and 3B show the *in vivo* efficacy of A17m09_hIgG4 in inhibiting the growth of mouse M38 colon cancer tumors, wherein FIG. 3A is a graph showing the mouse tumor volume, and FIG. 3B is a graph showing the mouse body weight; pembrolizumab is used as a positive control and hIgG4 as a negative control.

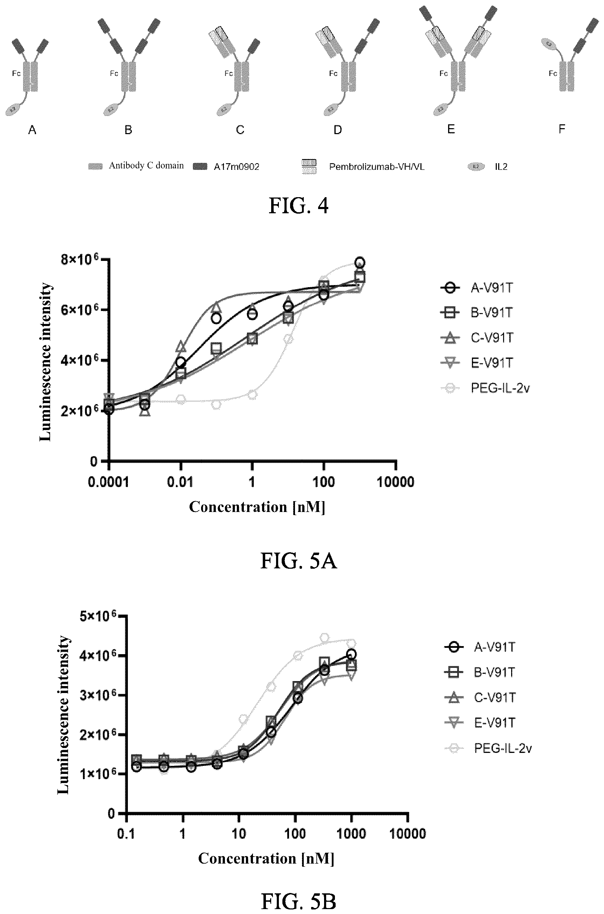

FIG. 4 shows the construction of fusion proteins of an anti-PD-1 antibody and an IL-2 mutant. In the molecular structure shown in A of FIG. 4, the anti-PD-1 nanobody A17m0902 is conjugated to the N-termini of the Knob and Hole chains via an IgG1 heavy chain hinge region. In the molecular structure shown in B of FIG. 4, two anti-PD-1 nanobodies A17m0902 are linked end-to-end by a $(G_4S)_2$ linker, and its C-terminus is then conjugated to the N-termini of the Knob and Hole chains via an IgG1 heavy chain hinge region. In the molecular structure shown in C of FIG. 4, the anti-PD-1 nanobody A17m0902 is conjugated to the N-terminus of the Hole chain via an IgG1 heavy chain hinge region, and the heavy chain of the corresponding Fab fragment of the anti-PD-1 antibody pembrolizumab is conjugated to the N-terminus of the Knob chain via an IgG1 heavy chain hinge region. In the molecular structure shown in D of FIG. 4, two anti-PD-1 nanobodies A17m0902 are linked end-to-end by a $(G_4S)_2$ linker, and its C-terminus is then conjugated to the N-terminus of the Hole chain via an IgG1 heavy chain hinge region; the heavy chain of the corresponding Fab fragment of the anti-PD-1 antibody pembrolizumab is conjugated to the N-terminus of the Knob chain via an IgG1 heavy chain hinge region. In the molecular structure shown in E of FIG. 4, the heavy chain of the corresponding Fab fragment of the anti-PD-1 antibody pembrolizumab is conjugated to the N-termini of the Knob and Hole chains via an IgG1 heavy chain hinge region, and the anti-PD-1 nanobody A17m0902 is conjugated to the N-terminus of the heavy chain of the corresponding Fab fragment of pembrolizumab via a $(G_4S)_2$ linker. In molecule F-V91T shown in F of FIG. 4, two anti-PD-1 nanobodies A17m0902 are linked end-to-end by a $(G_4S)_2$ linker, and its C-terminus is then conjugated to the N-terminus of the Hole chain via an IgG1 heavy chain hinge region. In the molecule of A-E of FIG. 4, the IL-2 mutant is linked to the C-terminus of the Knob chain of the Fc by a $(G_4S)_3$ linker. In the molecule of F, IL-2v (V91T) is linked to the N-terminus of the Knob chain by a $G_4S$ linker.

FIGs. 5A and 5B show the proliferation activity of PD-1-IL-2v (V91T) fusion proteins of different formats on M07e and M07e-hPD-1 cells, wherein FIG. 5A shows the proliferation activity of PD-1-IL-2v (V91T) fusion proteins of different formats on M07e-hPD-1 cells, and FIG. 5B shows the proliferation activity of PD-1-IL-2v (V91T) fusion proteins of different formats on M07e cells.

FIGs. 6A and 6B show the proliferation activity of PD-1-IL-2v fusion proteins of the same format with different IL-2 mutants on the M07e and M07e-hPD-1 cell strains, wherein FIG. 6A shows the proliferation activity of PD-1-IL-2v fusion proteins on M07e-hPD-1 cells, and FIG. 6B shows the proliferation activity of PD-1-IL-2v fusion proteins on M07e cells.

FIG. 7 shows the proliferation promoting activity of PD-1-IL-2v or IgG-IL-2v on M07e-hPD-1 cells.

FIGs. 8A and 8B show the proliferation activity of PD-1-IL-2v with different IL-2 mutants on unstimulated T cells, wherein FIG. 8A shows the proliferation of CD4$^+$ T cells, and FIG. 8B shows the proliferation of CD8$^+$ T cells.

FIGs. 9A and 9B show the proliferation activity of PD-1-IL-2v with different IL-2 mutants on T cells stimulated with an anti-CD3 antibody, wherein FIG. 9A shows the proliferation of CD4$^+$ T cells, and FIG. 9B shows the proliferation of CD8$^+$ T cells.

FIG. 10 shows the proliferation activity of PD-1-IL-2v with different anti-PD-1 nanobody mutants on the M07e-hPD-1 cell strain.

FIG. 11 shows the inhibition of tumor growth in a mouse CT26 tumor model by A-N88D, wherein A17m0902_hIgG4 is used as a control.

FIG. 12 shows changes in the body weight of the mouse CT26 tumor model after administration of A-N88D.

## DETAILED DESCRIPTION

### Definitions of Terms

In order to facilitate the understanding of the present disclosure, some technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558(1968).

As used herein, the term "immunoconjugate" refers to a fusion polypeptide molecule comprising one effector moiety and at least one antigen-binding moiety; optionally, the immunoconjugate may also comprise an Fc region. In certain embodiments, the immunoconjugate comprises one effector moiety, one antigen-binding moiety, and one Fc region. In certain embodiments, the immunoconjugate comprises one effector moiety, two antigen-binding moieties, and two Fc regions. In certain embodiments, the immunoconjugate comprises one effector moiety, four antigen-binding moieties, and two Fc regions. In certain embodiments, the immunoconjugate comprises one effector moiety, one antigen-binding moiety, and two Fc regions. In certain embodiments, the immunoconjugate comprises one effector moiety, three antigen-binding moieties, and two Fc regions.

In a specific embodiment, the immunoconjugate described herein is a fusion protein; that is, the components of the immunoconjugate are linked to each other by a peptide bond directly or by a linker.

In a specific embodiment herein, the antigen-binding moiety in the "immunoconjugate" is a PD-1-binding domain, and the effector moiety is an IL-2 or a variant thereof.

"Programmed death-1", "programmed cell death-1", "protein PD-1", "PD-1", "PDCD1" and "hPD-1" are used interchangeably and include variants, isoforms, and species homologs of human PD-1, and analogs having at least one common epitope with PD-1. The complete PD-1 sequence can be found under GenBank accession No. U64863. "Programmed death ligand-1 (PD-L1)" is one of two cell surface glycoprotein ligands of PD-1 (the other being PD-L2) that downregulates T cell activation and cytokine secretion upon binding to PD-1. "PD-L1" as used herein includes human PD-L1 (hPD-L1), variants, isoforms, and interspecies homologs of hPD-L1, and analogs sharing at least one epitope with hPD-L1. The complete hPD-L1 sequence can be found under GenBank accession No. Q9NZQ7.

As used herein, the term "PD-1-binding domain" or "PD-1-binding protein" is used interchangeably and refers to any protein capable of specifically binding to PD-1 or any molecule comprising the protein. The PD-1-binding protein may include an antibody against PD-1 defined in the present disclosure, an antigen-binding fragment thereof, or a conjugate thereof. The PD-1-binding protein also encompasses immunoglobulin superfamily antibodies (IgSF) or CDR-grafted molecules. The "PD-1-binding protein" of the present disclosure may comprise at least one immunoglobulin single variable domain (such as a VHH) that binds to PD-1. In some embodiments, the "PD-1-binding protein" may comprise 2, 3, 4 or more immunoglobulin single variable domains (such as VHHs) that bind to PD-1. The PD-1-binding protein of the present disclosure may also comprise, in addition to the immunoglobulin single variable domain of PD-1, a linker and/or a moiety with effector functions, such as a half-life extending moiety (e.g., an immunoglobulin single variable domain that binds to serum albumin), and/or a fusion partner (such as serum albumin) and/or a conjugated polymer (such as PEG) and/or an Fc region. In some embodiments, the "PD-1-binding protein" of the present disclosure also encompasses bispecific/multi-specific antibodies comprising immunoglobulins that bind to different antigens (e.g., a first antibody that binds to a first antigen (e.g., PD-1) and a second antibody that binds to a second antigen (e.g., 4-1BB), optionally a third antibody that binds to a third antigen, and further optionally a fourth antibody that binds to a fourth antigen).

The "PD-1-binding protein" or "PD-1 antibody or antigen-binding fragment thereof" of the present disclosure may comprise one or more effector molecules in, for example, a conjugated manner. The "effector molecule" includes, for example, antineoplastic agents, drugs, toxins, biologically active proteins such as enzymes, other antibody or antibody fragments, synthetic or naturally occurring polymers, nucleic acids, and fragments thereof (such as DNA, RNA, and fragments thereof), radionuclides, particularly radioiodide, radioisotopes, chelated metals, nanoparticles and reporter groups such as fluorescent compounds or compounds which may be detected by NMR or ESR spectroscopy. In some embodiments, the effector molecule is a cytokine (e.g., IL-2, IL-15, or a variant thereof). In some other embodiments, the effector molecule is a polymer, which may generally be a synthetic or naturally occurring polymer. Specific examples of synthetic polymers include optionally substituted straight or branched chain polyethylene glycol), polypropylene glycol), poly(vinyl alcohol), or derivatives thereof. Specific naturally occurring polymers include lactose, amylose, dextran, glycogen, or derivatives thereof. In some other embodiments, the polymer is albumin or a fragment thereof, e.g., human serum albumin or a fragment thereof. Conjugation of the polymer to the PD-1-binding protein or PD-1 antibody can be achieved by conventional methods.

"Cytokine" is a general term for proteins that are released by a cell population to act on other cells as intercellular mediators. Examples of such cytokines include lymphokines, monokines, chemokines, and traditional polypeptide hormones. Exemplary cytokines include: human IL-2, IL-15, IFN-y, IL-6, TNFα, IL-17, and IL-5 and variants thereof.

"Interleukin-2" or "IL-2" refers to any natural IL-2 of any vertebrate source, including mammals such as primates (e.g., humans) and rodents (e.g., mice and rats). The term encompasses unprocessed IL-2 as well as any form of processed IL-2 derived from cells. The term also encompasses naturally occurring IL-2 variants, such as splice variants or allelic variants. An exemplary amino acid sequence of wild-type human IL-2 is set forth in SEQ ID NO: 87. Unprocessed human IL-2 additionally comprises an N-terminal signal peptide of 20 amino acids (set forth in SEQ ID NO: 272 in WO2012107417), which is absent in the mature IL-2 molecule.

"Mutated IL-2" or "IL-2 variant" refers to a polypeptide that differs from the wild-type IL-2 amino acid sequence by a mutation (e.g., substitution, deletion, or addition) at at least one amino acid position.

In the present disclosure, when referring to an IL-2 variant, single amino acid substitutions are described in the following manner: [original amino acid residue/position/amino acid residue for substitution]. For example, the substitution of alanine for phenylalanine at position 42 may be represented by F42A. Single amino acid substitutions may be linked by "/" to indicate a combination of mutations at multiple given positions. For example, the combination of mutations at positions F42A, L72G, and N88R may be represented by: F42A/L72G/N88R, meaning that the variant comprises the F42A, L72G, and N88R mutations.

In the present disclosure, when referring to amino acid positions in an IL-2 protein or IL-2 sequence segment, they are determined by reference to the amino acid sequence SEQ ID NO: 87 of the wild-type human IL-2 protein (also referred to as wt IL-2).

Corresponding amino acid positions on other IL-2 proteins or polypeptides (including full-length sequences or truncated fragments) can be identified by amino acid sequence alignment with SEQ ID NO: 87. Thus, in the present disclosure, unless otherwise indicated, amino acid positions of an IL-2 protein or polypeptide are amino acid positions numbered according to SEQ ID NO: 87. For example, when referring to "F42", it refers to the phenylalanine residue F at position 42 of SEQ ID NO: 40, or an amino acid residue at the corresponding position on other IL-2 polypeptide sequences determined by alignment. Sequence alignments for amino acid position determination can be performed using Basic Local Alignment Search Tool available at https://blast.ncbi.nlm.nih.gov/Blast.cgi with default parameters.

"CD25" or "α subunit of IL-2 receptor" refers to any natural CD25 of any vertebrate source, including mammals such as primates (e.g., humans) and rodents (e.g., mice and rats). The term encompasses unprocessed "full-length" CD25 as well as any form of processed CD25 derived from cells; and naturally occurring CD25 variants, such as splice variants or allelic variants. In certain embodiments, CD25 is human CD25.

"High-affinity IL-2 receptor" refers to the heterotrimer form of the IL-2 receptor that consists of a receptor γ subunit (also known as a common cytokine-receptor γ subunit, yc, or CD132), a receptor β subunit (also known as CD122 or p70), and a receptor α subunit (also known as CD25 or p55). In contrast, a "moderate affinity IL-2 receptor" refers to an IL-2 receptor that comprises only γ and β subunits but no α subunit (see, e.g., Olejniczak and Kasprzak, MedSci Monit 14, RA179-189(2008)).

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin, a four-peptide-chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being μ, chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

"Antigen-binding fragment" encompasses a single-chain antibody (i.e., full-length heavy and light chains); Fab, a modified Fab, Fab', a modified Fab', F(ab')2, Fv, Fab-Fv, Fab-dsFv, a single-domain antibody (e.g., VH or VL or VHH), scFv, a bivalent or trivalent or tetravalent antibody, Bis-scFv, a diabody, a tribody, a triabody, a tetrabody and an epitope-binding fragment of any of the above (see, e.g., Holliger and Hudson, 2005, Nature Biotech. 23(9): 1126-1136; Adair and Lawson, 2005, Drug Design Reviews-Online 2(3), 209-217). In some embodiments, the antigen-binding fragment is a VHH. Methods for producing and preparing such antigen-binding fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170, and WO2005/003171. Multivalent antibodies may comprise multiple specificities (e.g., bispecificities) or may be monospecific (see, e.g., WO92/22583 and WO05/113605), and an example of the latter is Tri-Fab (or TFM) described in WO 92/22583.

"Fc domain" or "Fc region" is used to define a C-terminal region of an immunoglobulin heavy chain that comprises at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226 or Pro230 to the carboxyl-terminus of the heavy chain. However, antibodies produced by host cells may undergo post-translational cleavage of one or more, particularly one or two, amino acids from the C-terminus of the heavy chain. Therefore, an antibody produced by a host cell by expressing a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbered according to the Kabat EU index). Therefore, the C-terminal lysine (K447), or the C-terminal glycine (G446) and lysine (K447), of the Fc region may or may not be present. If not indicated otherwise, amino acid sequences of heavy chains including Fc regions (or a subunit of an Fc region defined in the present disclosure) are denoted in the present disclosure without a C-terminal glycine-lysine dipeptide. In one embodiment, a heavy chain of a subunit of an Fc region included in an immunoconjugate comprises an additional C-terminal glycine-lysine dipeptide (G446 and K447, numbered according to the EU index of Kabat). In one embodiment, a heavy chain of a subunit of an Fc region included in an immunoconjugate comprises an additional C-terminal glycine residue (G446, numbered according to the EU index of Kabat). The composition of the present disclosure (such as the pharmaceutical composition) comprises a population of immunoconjugates of the present disclosure. The population of immunoconjugates may comprise molecules having a full-length heavy chain and molecules having a cleaved variant heavy chain. The population of immunoconjugates may consist of a mixture of molecules having a full-length heavy chain and molecules having a cleaved variant heavy chain, wherein at least 50%, at least 60%, at least 70%, at least 80%, or at least 90% of the immunoconjugates have a cleaved variant heavy chain. The cleaved variant heavy chain may be the case where the C-terminal lysine (K447), or the C-terminal glycine (G446) and lysine (K447), of the Fc region is/are not present. Unless otherwise specified in the present disclosure, the numbering of amino acid residues in the Fc region or constant region conforms to the EU numbering scheme, also known as the EU index, as described in Kabat et al., Sequences of Proteins of Immunological Interest, 5th ed., Public Health Service, National Institutes of Health, Bethesda, MD, 1991.

A "subunit" of an Fc domain refers to one of the two polypeptides forming the dimeric Fc domain, i.e., a polypeptide comprising C-terminal constant regions of an immunoglobulin heavy chain, capable of stable self-association. For example, a subunit of an IgG Fc region comprises an IgG CH2 constant domain and an IgG CH3 constant domain. In the present disclosure, the use of "first subunit" or Fc1 and "second subunit" or Fc2 is not intended to confer a specific order or orientation but is for convenience of distinguishing.

"Modification (or mutation) promoting the association of the first subunit and the second subunit of the Fc domain" is a manipulation of the peptide backbone or a post-translational modification of an Fc domain subunit that reduces or prevents the association of a polypeptide comprising the Fc domain subunit with an identical polypeptide to form a homodimer. As used herein, a modification promoting association particularly includes separate modifications made to each of the two Fc domain subunits desired to associate (i.e. the first subunit and the second subunit of the Fc domain), wherein the modifications are complementary to each other so as to promote the association of the two Fc domain subunits. For example, a modification promoting association may alter the structure or charge of one or both of the Fc domain subunits so as to promote their association sterically or electrostatically, respectively. Thus, (hetero)dimerization occurs between a polypeptide comprising the first Fc domain subunit and a polypeptide comprising the second Fc domain subunit, which might be different in the sense that other components fused to each of the subunits (e.g., antigen-binding moieties) are different. In some embodiments, the modification promoting association comprises an amino acid mutation in the Fc domain, specifically an amino acid substitution.

"Complementarity" refers to, for example, a combination of interactions that affect heavy/light chain pairing at the interface of CH1 and CL (or CH3 and CH3) of the antigen-binding protein described herein. "Steric complementarity" or "conformational complementarity" refers to, for example, the compatibility of three-dimensional structures at the interaction surface of CH1 and CL (or CH3 and CH3). "Electrostatic complementarity" refers to, for example, the compatibility of negatively and/or positively charged atoms placed at the interaction surface of CH1 and CL (or CH3 and CH3). Methods for measuring electrostatic complementarity at a protein/protein interface are known in the art and are described, for example, in McCoy et al., (1997) J Mol Biol 268,570-584; Lee et al., (2001) Protein Sci. 10,362-377; and Chau et al., (1994) J Comp Mol Des 8,51325. Methods for measuring steric complementarity at a protein/protein interface are known in the art and are described, for example, in Lawrence et al., (1993) J Mol Biol 234,946-950; Walls et al., (1992) J Mol Biol 228,277-297; and Schueler-Furman et al., (2005) Proteins 60,187-194.

"Effector function" when used in reference to antibodies refers to those biological activities attributable to the Fc region of an antibody, which vary with the antibody isotype. Examples of antibody effector functions include: C1q binding and complement-dependent cytotoxicity (CDC), Fc receptor (FcR) binding, antibody-dependent cell-mediated cytotoxicity (ADCC), antibody-dependent cellular phagocytosis (ADCP), cytokine secretion, immune complex-mediated antigen uptake by antigen-presenting cells, down-regulation of cell surface receptors (e.g., B cell receptor), and B cell activation. ADCC is an immune mechanism leading to the lysis of antibody-coated target cells by immune effector cells. The target cells are cells to which antibodies or derivatives thereof comprising an Fc region specifically bind, generally via the N-terminal protein part of the Fc region. "Reduced ADCC" is defined as a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody that is produced by the same type of host cells using the same standard production, purification, formulation, and storage methods known to those skilled in the art, but that has not been engineered. For example, the reduction in ADCC mediated by an antibody comprising in its Fc domain an amino acid substitution that reduces ADCC is relative to the ADCC mediated by the same antibody without this amino acid substitution in the Fc domain. Suitable assays for measuring ADCC are well known in the art (see, e.g., WO2006/082515 or WO2012/130831).

"Activating Fc receptor" is an Fc receptor that, after engagement by an Fc domain of an antibody, elicits signaling events that stimulate cells bearing the receptor to perform effector functions. Human activating Fc receptors include FcγRIIIa (CD16a), FcyRI (CD64), FcyRIIa (CD32), and FcαRI(CD89).

"Immunoconjugate" refers to a polypeptide molecule comprising at least one IL-2 molecule and at least one antibody. As described herein, an IL-2 molecule may be linked to an antibody through a variety of interactions and in a variety of configurations. In a particular embodiment, the IL-2 molecule is fused to the antibody via a peptide linker. A particular immunoconjugate according to the present invention essentially consists of one IL-2 molecule and an antibody linked by one or more linker sequences.

"Reduced affinity" or "reduced binding", for example, reduced binding to IL-2R$\alpha$, refers to a reduction in affinity for the corresponding interaction, as measured by, for example, SPR. The term also includes reduction of the affinity to 0 or below the detection limit of the analytic method, i.e., complete elimination of the interaction. Conversely, "reduced affinity" or "increased binding" refers to an increase in binding affinity for the corresponding interaction.

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

The antibody of the present disclosure may be polyclonal, monoclonal, xenogenic, allogeneic, syngeneic, or modified forms thereof, with the monoclonal antibody being particularly useful in various embodiments. Generally, the antibody of the present disclosure is a recombinant antibody. The "recombinant" used herein generally refers to such products as a cell, a nucleic acid, a protein or a vector, and indicates that the cell, the nucleic acid, the protein or the vector has been modified by the introduction of a heterologous nucleic acid or protein or the alteration of a natural nucleic acid or protein, or that the cell is derived from a cell modified in this way. For example, recombinant cells express genes that are not found within the native (non-recombinant) cellular form or express native genes that are abnormally expressed, lowly expressed, or not expressed at all.

"Domain" of a polypeptide or protein refers to a folded protein structure that is capable of maintaining its tertiary structure independently of the rest of the protein. In general, a domain is responsible for a single functional property of a protein, and in many cases may be added, removed, or transferred to other proteins without loss of functions of the rest of the protein and/or the domain.

"Immunoglobulin domain" refers to a globular region of an antibody chain (e.g., a chain of a conventional antibody with a four-peptide-chain structure or a heavy-chain antibody) or a polypeptide essentially consisting of such globular regions. The immunoglobulin domain is characterized in that it retains the immunoglobulin fold characteristic of an antibody molecule, and it consists of a 2-layer sandwich of about 7 antiparallel β-strands arranged in two β-sheets, optionally stabilized by a conserved disulfide bond.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2" and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH and/or camelized VH, e.g. a camelized human VH), IgNAR, domains, (single-domain) antibodies as VH domains or derived from VH domains (such as dAbsTM) and (single-domain) antibodies as VL domains or derived from VL domains (such as dAbsTM). Immunoglobulin single variable domains based on and/or derived from heavy chain variable domains (such as VH or VHH domains) are generally preferred. A specific example of an immunoglobulin single variable domain is a "VHH domain" (or abbreviated as "VHH") as defined below.

"VHH domain", also known as a heavy chain single-domain antibody, a VHH, a VHH antibody fragment, a VHH antibody, or a nanobody, is a variable domain of an antigen-binding immunoglobulin known as a "heavy chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. "Heavy chain single-domain antibody", "VHH domain", "VHH", "VHH antibody fragment", "VHH antibody", and "domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. In some embodiments, the VHH domain comprises three CDRs and four framework regions designated FR1, CDR1, FR2, CDR2, FR3, CDR3, and FR4. In some embodiments, the VHH domain may be truncated at the N-terminus or C-terminus such that it comprises only part of FR1 and/or FR4, or lacks one or two of those framework regions, as long as the VHH substantially retains antigen binding and specificity. The total number of amino acid residues in a VHH domain will usually be in the range of 110 to 120, often between 112 and 115. However, it should be noted that smaller and longer sequences may also be suitable for the purposes described in the present disclosure.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Other numbering systems or numbering schemes include Chothia, IMGT and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences, and in some specific embodiments, may contain the human framework region sequence of IGHV3. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. The humanized antibody of the present disclosure also includes humanized antibodies which are further subjected to CDR affinity maturation by phage display. Additionally, in order to avoid the decrease in activity caused by the decrease in immunogenicity, the framework region sequence in the human antibody variable region can be subjected to minimum reverse mutation or back mutation to maintain activity.

An "affinity-matured" antibody is an antibody that has one or more changes in one or more hypervariable regions (HVRs) that result in an improvement in the affinity of the antibody for the antigen, as compared to the parent antibody which does not have such changes. For example, an "affinity-matured" PD-1 antibody has one or more changes in one or more CDRs that result in an increase in the affinity for the antigen, as compared to its parent antibody. Affinity-matured antibodies can be prepared, for example, by methods known in the art as described below: Marks et al., 1992, Biotechnology 10: 779-783 or Barbas et al., 1994, Proc. Nat. Acad. Sci, USA 91: 3809-3813; Shier et al., 1995, Gene 169: 147-155; Yelton et al., 1995, Immunol. 155: 1994-2004; Jackson et al., 1995, J. Immunol. 154(7): 3310-9; Hawkins et al., 1992, J. Mol. Biol. 226(3): 889896; KS Johnson and RE Hawkins, "Affinity maturation of antibodies using phage display", Oxford University Press 1996.

Generally, the PD-1 antibody or the immunoconjugate of the present disclosure will bind to an antigen to be bound (i.e., PD-1) with a dissociation constant (KD) of preferably $10^{-7}$ to $10^{-10}$ mol/L (M), more preferably $10^{-8}$ to $10^{-10}$ mol/L, even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured by a Biacore or KinExA or Fortibio assay. Any KD value greater than $10^{-4}$ M is generally considered to indicate non-specific binding. Specific binding of an antigen-binding protein to an antigen or epitope can be measured in any suitable manner known, including, for example, surface plasmon resonance (SPR) assays, Scatchard assay, and/or competitive binding assay (e.g., radioimmunoassay (RIA), enzyme immunoassay (EIA), and sandwich competitive assay) described in the present disclosure.

"Antigen" refers to a molecule used for immunization of an immunocompetent vertebrate to produce an antibody that recognizes the antigen or to screen an expression library (e.g., particularly phage, yeast, or ribosome display library). In the present disclosure, the antigen is defined in a broader sense, and includes a target molecule that is specifically recognized by the antibody, and a portion or a mimic of a molecule used in an immunization process for producing the antibody or in library screening for selecting the antibody. For example, for the antibody of the present disclosure that binds to human PD-1, monomers and multimers (e.g., dimers, trimers, etc.) of human PD-1, and truncated variants and other variants of human PD-1 are all referred to as PD-1 antigens.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Specific binding" or "selective binding" refers to binding of an antibody to an epitope on a predetermined antigen. For example, an antibody binds to a predetermined antigen or epitope thereof with an equilibrium dissociation constant ($K_D$) of about less than $10^{-7}$ M or even less and with an affinity that is at least twice as high as its affinity for binding to a non-specific antigen other than the predetermined antigen or the epitope thereof (or non-specific antigens other than closely related antigens, e.g., BSA, etc.), when measured by surface plasmon resonance (SPR) techniques in an instrument using human PD-1 or an epitope thereof as an analyte and the antibody as a ligand. "Antigen-recognizing antibody" is used interchangeably in the present disclosure with "specifically bound antibody".

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D$ = kd/ka. The value of the dissociation constant can be determined directly by well-known methods and can be calculated by methods such as those described by Caceci et al (1984, Byte 9:340-362) even for complex mixtures. For example, $K_D$ can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore™ system or KinExA. The binding affinities associated with different molecular interactions, e.g., the binding affinities of different antibodies to a given antigen, can be compared by comparing the $K_D$ values of antibodies/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to CD40).

"Conservative substitution" refers to a substitution with another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity" or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared $\times$ 100%. For example, if 6 out of 10 positions are matched or homologous when two sequences are optimally aligned, the two sequences are 60% homologous. Generally, when two sequences are aligned, a comparison is performed to obtain the maximum homology percentage.

"Nucleic acid molecule", "nucleic acid", or "polynucleotide" is used interchangeably and refers to DNA and RNA. The nucleic acid molecule may be single-stranded or double-stranded, and is preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Vector" refers to a construct capable of delivering and, in some embodiments, expressing one or more genes or sequences of interest in a host cell. Examples of vectors include, but are not limited to, viral vectors, naked DNA or RNA expression vectors, plasmid, cosmid or phage vectors, DNA or RNA expression vectors bound to cationic condensing agents, DNA or RNA expression vectors encapsulated in liposomes, and certain eukaryotic cells such as producer cells.

"Host cell" includes individual cells or cell cultures which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental or deliberate mutations. The host cell includes cells transfected and/or transformed *in vivo* with polynucleotides of the present disclosure. "Cell", "cell line", and "cell culture" are used interchangeably, and all such designations include their progeny. It should also be understood that all progenies may not be precisely identical in DNA content due to intentional or unintentional mutations. Mutant progeny with identical function or biological activity as screened in the original transformed cells is included.

The term "pharmaceutical composition" refers to a mixture containing one or more of the antibodies described herein or a physiologically/pharmaceutically acceptable salt or prodrug thereof, and other chemical components, as well as other components such as physiologically/pharmaceutically acceptable carriers and excipients. The pharmaceutical composition is intended to promote administration to an organism and facilitate the absorption of the active ingredient so that it can exert its biological activity.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for aerosol or parenteral administration is phosphate buffered saline (PBS) or normal (0.9%) saline. Compositions containing such carriers are formulated by well-known conventional methods (see, e.g.,

Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Inhibition" and "blocking" are used interchangeably and encompass both partial and complete inhibition/blocking. "Inhibition of growth" (e.g., involving cells) is intended to include any measurable reduction in cell growth.

"Proliferative disease" refers to a disorder associated with a certain degree of abnormal cell proliferation. In one embodiment, the proliferative disorder is cancer.

"Cancer" refers to or describes a physiological disease in mammals that is typically characterized by unregulated cell growth. Examples of cancer include, but are not limited to, carcinoma, lymphoma, blastoma, sarcoma, and leukemia or lymphoid malignancies. More specific examples of such cancers include, but are not limited to, squamous cell cancer (e.g., epithelial squamous cell cancer), lung cancer (including small-cell lung cancer, non-small cell lung cancer, adenocarcinoma of the lung, and squamous carcinoma of the lung), peritoneal cancer, hepatocellular cancer, stomach/gastric cancer (including gastrointestinal cancer and gastrointestinal stromal cancer), pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine cancer, salivary gland carcinoma, kidney cancer, prostate cancer, vulval cancer, thyroid cancer, liver cancer, anal cancer, penile cancer, melanoma, superficial spreading melanoma, lentigo maligna melanoma, acral melanomas, nodular melanomas, multiple myeloma and B-cell lymphoma; chronic lymphocytic leukemia (CLL), acute lymphoblastic leukemia (ALL), hairy cell leukemia, chronic myeloblastic leukemia, and post-transplant lymphoproliferative disorder (PTLD), as well as abnormal vascular proliferation associated with phakomatoses, edema (such as that associated with brain tumors), and Meigs syndrome, brain tumor and brain cancer, as well as head and neck cancer, and related metastases. In certain embodiments, cancers that are suitable for being treated by the PD-1-binding protein of the present disclosure include breast cancer, colorectal cancer, rectal cancer, non-small cell lung cancer, glioblastoma, non-Hodgkin lymphoma (NHL), renal cell cancer, prostate cancer, liver cancer, pancreatic cancer, soft-tissue sarcoma, Kaposi's sarcoma, carcinoid carcinoma, head and neck cancer, ovarian cancer, mesothelioma, and multiple myeloma. In some embodiments, the cancer is selected from the group consisting of: non-small cell lung cancer, glioblastoma, neuroblastoma, melanoma, breast cancer (e.g. triple-negative breast cancer), stomach/gastric cancer, colorectal cancer (CRC), and hepatocellular carcinoma. Also, in some embodiments, the cancer is selected from the group consisting of: non-small cell lung cancer, colorectal cancer, glioblastoma, and breast cancer (e.g. triple-negative breast cancer), including metastatic forms of those cancers.

"Tumor" refers to all neoplastic cell growth and proliferation, whether malignant or benign, and all pre-cancerous and cancerous cells and tissues.

"Cancer", "cancerous", "proliferative disorder", and "tumor" are not mutually exclusive when referred to in the present disclosure.

"Preventing cancer" or "prevention of cancer" refers to delaying, inhibiting, or preventing the onset of cancer in a subject in which the onset of oncogenesis or tumorigenesis has not been evidenced but a predisposition toward cancer has been identified, as determined by, for example, genetic screening or otherwise. The term also encompasses treating a subject having a premalignant disorder to stop the progression of, or cause regression of, the premalignant disorder towards malignancy.

"Giving", "administering" and "treating", when applied to animals, humans, experimental subjects, cells, tissues, organs, or biological fluid, refer to contact of an exogenous drug, a therapeutic agent, a diagnostic agent, or a composition with the animals, humans, subjects, cells, tissues, organs or biological fluid, e.g., therapeutic, pharmacokinetic, diagnostic, research, and experimental methods. The treatment of cells comprises contacting the reagent with the cells and contacting the reagent with fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary or research subjects, they refer to therapeutic treatment, preventive or prophylactic measures, and research and diagnostic applications.

"Treating" or "treatment" refers to administering a therapeutic agent, such as a therapeutic agent comprising any one of the antibodies of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular disease symptom (also referred to as the "therapeutically effective amount") may vary depending on factors such as the disease state, age and weight of the subject, and the ability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical disorder. An effective amount also refers to an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

"Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

In the present disclosure, unless otherwise defined, the use of "first", "second", and "third", for example, are not intended to confer a specific order or orientation but is for, for example, convenience of distinguishing when more than one moiety of the same type is present.

In the present disclosure, "f", when used for mutations, means that the mutations are present simultaneously in the same IL-2 variant or in the same Fc or in the same immunoconjugate; for example, "F42A/L72G" means that both the F42A and L72A mutations are present in the same IL-2 variant.

In the present disclosure, "subject" or "patient" means a mammal, particularly a primate, and particularly a human.

Unless otherwise specified, the amino acid position numbering for the IL-2 or the variant thereof in the present disclosure is relative to wild-type IL-2 (e.g., set forth in SEQ ID NO: 87).

## Examples

The present disclosure is further described below with reference to examples, but these examples are not intended to limit the scope of the present disclosure.

Experimental methods without specific conditions indicated in the examples or test examples of the present disclosure are generally conducted under conventional conditions, or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

## Example 1. Screening and Preparation of Anti-PD-1 Nanobodies

In the present disclosure, His-tagged human PD-1 was used as an immunizing antigen, and biotinylated human PD-1 and cynomolgus monkey PD-1 as screening antigens; the antigens were all purchased from AcroBiosystems.

1. Sequences and preparation of immunizing and screening antigens

Table 2. PD-1 antigens for immunizing alpacas and screening

| Antigen | Cat. No. | Starting and ending amino acids | Accession No. |
|---|---|---|---|
| Human PD-1 protein (His Tag) | PD1-H5221 | Leu25-Gln167 | # NP_005009.2 |
| Biotinylated human PD-1 protein (Avitag -His Tag) | PD1-H82E4 | | # Q15116-1 |
| Biotinylated monkey PD-1 protein (Avitag -His Tag) | PD1-C82E6 | | # B0LAJ3 |

The sequence of human PD-1 protein (NP_005009.2) is shown below, with the sequence of the extracellular region underlined-it starts with the amino acid Leu25 and ends with the amino acid Gln167.
> Human PD-1 amino acid sequence

MQIPQAPWPVVWAVLQLGWRPGWFLDSPDRPWNPPTFSPALLVVTEGDNATFT
CSFSNTSESFVLNWYRMSPSNQTDKLAAFPEDRSQPGQDCRFRVTQLPNGRDFH
MSVVRARRNDSGTYLCGAISLAPKAQIKESLRAELRVTERRAEVPTAHPSPSPRP
AGQFQTLVVGVVGGLLGSLVLLVWVLAVICSRAARGTIGARRTGQPLKEDPSAV
PVFSVDYGELDFQWREKTPEPPVPCVPEQTEYATIVFPSGMGTSSPARRGSADGP
RSAQPLRPEDGHCSWPL (SEQ ID NO: 1)

2. Alpaca immunization, nanobody yeast display library construction, and antibody screening

1) Alpaca immunization

Two healthy alpacas were immunized. At day 0, the first immunization was performed: 0.25 mg of human PD-1 antigen was well mixed with 1 mL of Freund's complete adjuvant (CFA), and the mixture was injected subcutaneously. At days 21, 42, and 63, 0.125 mg of human PD-1 antigen was well mixed with 1 mL of Freund's incomplete adjuvant (IFA), and the mixture was injected subcutaneously. 10 mL, 50 mL, and 50 mL of blood were collected at day 28, day 49, and day 70, respectively. The serum titer was measured, and alpaca peripheral lymphocytes up to the library construction standard were used to construct a nanobody yeast display library.

2) Yeast library construction

After the third and fourth immunizations of the 2 alpacas, 50 mL peripheral blood samples were taken. PBMCs were separated from the peripheral blood samples, and RNA was extracted from the PBMCs and reverse-transcribed to obtain total cDNA. The cDNA was used as a template for the first round of nested PCR. After two rounds of nested PCR amplification, the fragment products of the second round of nested PCR were ligated to a yeast library vector (the yeast library vector pYDN3), and the ligation products were introduced into yeast competent cells by electroporation. Colony PCR was used to verify the insertion rate and library diversity. The results of analysis based on the number of library transformants, the library insertion rate, and the library diversity and sequencing show that one alpaca's library capacity was $9.28 \times 10^7$ and the other alpaca's was $1.54 \times 10^8$.

3) Nanobody (VHH) screening

The yeast display system was screened for antibodies using fluorescence-activated cell sorting (FACS), library screening, and identification. Two rounds of sorting were performed. In the first round, enrichment was performed using biotinylated human PD-1 protein (Avitag -His Tag) magnetic beads. In the second round, sorting was performed using a biotinylated monkey PD-1 protein (His, Avitag) flow cytometry sorter (flow cytometry antibody: mouse anti-HA tag Dylight 488; streptavidin Dylight 650). After the two rounds of sorting, monoclonal identification, sequencing, and sequence analysis were performed, and 18 unique VHH sequences that cross-bind to human and monkey PD-1 antigens were obtained. The sequences A6 and A17 among them are shown below.

> A6 variable region

EVQLVESGGGLVQAGGSLRLSCAVSGRTFS**SAAMG**WFRQAPGKEREFVAA**ISW
SFGSTYYADSVKG**RFTISRDNAKNTVYLQMNSLKPEDTAVYYCAA**DPSIETMG
GGWDY**WGQGTQVTVSS(SEQ ID NO: 2)

> A17 variable region

EVQVVESGGGLVQPGGSLRLSCVASGLTFS**DYSMS**WYRQAPGKERELVA**IISGS
GVIAHYVDSVKG**RFTISRDNAKSTVYLQMVSLKPEDRGVYYCRA**VSDWDDY**
WGQGTQVTVSS(SEQ ID NO: 3)

Table 3. The CDRs of the anti-PD-1 nanobodies (Kabat numbering scheme)

| No. | CDR sequences | |
|-----|------|------|
| A6 | CDR1 | SAAMG (SEQ ID NO: 4) |
| | CDR2 | ISWSFGSTYYADSVKG (SEQ ID NO: 5) |
| | CDR3 | DPSIETMGGGWDY (SEQ ID NO: 6) |
| A17 | CDR1 | DYSMS (SEQ ID NO: 7) |
| | CDR2 | IISGSGVIAHYVDSVKG (SEQ ID NO: 8) |
| | CDR3 | VSDWDDY (SEQ ID NO: 9) |

The sequences described above were each linked to a human IgG4 Fc (comprising a hinge region and S228P, according to the EU numbering scheme) fragment to construct VHH-Fc antibodies. Plasmids were constructed and transiently transfected into 293 cells. The antibodies were expressed and purified using a protein A column. The column was washed with PBS, and elution was performed with a 0.1 M glycine buffer, pH 2.5. The antibodies were transferred to a pH 7.4 PBS buffer by dialysis. The human IgG4 Fc (comprising a hinge region) sequences are as follows:

> hIgG4

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
(SEQ ID NO: 10)

> A6_hIgG4

EVQLVESGGGLVQAGGSLRLSCAVSGRTFSSAAMGWFRQAPGKEREFVAAISW
SFGSTYYADSVKGRFTISRDNAKNTVYLQMNSLKPEDTAVYYCAADPSIETMGG
GWDYWGQGTQVTVSSESKYGPPCPPCPAPEFLGGPSVFLFPPKPKDTLMISRTPE
VTCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQV
SLTCLVKGFYPSDIAVEWESNGQPENNYKTTPPVLDSDGSFFLYSRLTVDKSRWQ
EGNVFSCSVMHEALHNHYTQKSLSLSLGK (SEQ ID NO: 11)

> A17_hIgG4

EVQVVESGGGLVQPGGSLRLSCVASGLTFSDYSMSWYRQAPGKERELVAIISGS
GVIAHYVDSVKGRFTISRDNAKSTVYLQMVSLKPEDRGVYYCRAVSDWDDYW
GQGTQVTVSSESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLGK
(SEQ ID NO: 12)

**Example 2. Identification of Affinities of Anti-PD-1 Nanobodies for Antigen PD-1**

1. ELISA

Human PD-1 protein (his tag) was dissolved in PBS to form a 1 $\mu$g/mL solution, and the solution was added to a 96-well plate at 100 $\mu$L/well. The plate was coated with the antigen overnight at 4 °C. The plate was washed with PBST (PBS + 0.05% tween20) solution three times. PBST/1% BSA solution was added, and the plate was incubated at 37 °C for 1 h for blocking. After three washes with PBST solution, different concentrations of the PD-1 candidate VHH-Fc antibodies (diluted with PBST/1% BSA solution) were added, and the plate was incubated at 37 °C for 1.5 h. The plate was washed with PBST solution three times. 100 $\mu$L of HRP-conjugated anti-human IgG4 Fc secondary antibody (Thermo) solution was added, and the plate was incubated at 37 °C for 1 h. After three washes with PBST solution, TMB solution was added at 100 $\mu$L/well. After 5 min of reaction at room temperature, 50 $\mu$L of stop solution was added. Then 450 nM values were measured using a microplate reader, and $EC_{50}$ was calculated. According to the results shown in Table 4, both A6_hIgG4 and A17_hIgG4 have relatively strong binding affinity for PD-1 antigen.

Table 4. The $EC_{50}$ values of anti-PD-1 nanobodies measured by ELISA

| Antibody No. | $EC_{50}$ (nM) |
|---|---|
| A6_hIgG4 | 0.08605 |
| A17_hIgG4 | 0.06099 |

2. FACS

To measure the ability of the anti-PD-1 nanobodies to bind to PD-1 expressed on the cell membrane, different concentrations of the candidate anti-PD-1 VHH-Fc antibodies were added to $10^5$/well Jurkat cells overexpressing human PD-1 (Cat: J1250, Promega). The plate was incubated at room temperature for 20 min and then washed with PBS twice. 100 $\mu$L of anti-human IgG Fc fluorescent secondary antibody (Biolegend) was added. The plate was incubated at room temperature for 20 min and then washed with PBS twice, and the cells were resuspended in 250 $\mu$L of PBS. Fluorescence signals were detected by FACS, and $EC_{50}$ was calculated.

Pembrolizumab (purchased from Biointron) was used as a control. The results are shown in Table 5. The $EC_{50}$ value of A17_hIgG4 is 0.3568 nM, which is significantly better than those of the other antibodies obtained from the same screening (results not shown).

Table 5. The $EC_{50}$ values of anti-PD-1 nanobodies binding to cell surface PD-1 measured by FACS

| Antibody | $EC_{50}$ (nM) |
|---|---|
| A6_hIgG4 | 1.09 |
| A17_hIgG4 | 0.3568 |
| Pembrolizumab | 0.2169 |

**Example 3. Sequence Modification of Anti-PD-1 Nanobodies**

1. Humanization and back mutation of anti-PD-1 nanobodies

Antibody humanization was performed by CDR grafting. Through the website of IMGT or NCBI, the parent anti-PD-1 antibodies were aligned to fully human germline genes in the IMGT or NCBI/igblast database, and the human germline gene IGHV 3-23 (IGHV3-23*01; IGHV3-23*04) that was highly homologous with the PD-1 nanobodies was selected as a humanization template. CDRs were grafted, and 4 key residues (Y37, E44, R45, and L47) in the FR2 of the nanobodies were kept. The key core residues close to the CDRs and the Vernier zone residues that interact with the CDRs were back-mutated. Humanized antibodies A17h1, A17h2, A17h3, and A6h1 were obtained.

> A17h1 variable region

EVQLQESGGGLVQPGGSLRLSCTASGLTFS<u>DYSMS</u>WYRQAPGKGRELVA<u>IISGSG VIAHYVDSVKG</u>RFTISRDNSKNTVYLQMRSLTPEDRAVYYCRA<u>VSDWDDY</u>WG QGTQVTVSS

  (SEQ ID NO: 13)

> A17h2 variable region

EVQLVESGGGLVQPGGSLRLSCAASGLTFS<u>DYSMS</u>WVRQAPGKGLEWVA<u>IISGS GVIAHYVDSVKG</u>RFTISRDNSKNTVYLQMNSLRAEDTAVYYCRA<u>VSDWDDY</u>W GQGTQVTVSS(SEQ ID NO: 14)

> A17h3 variable region

EVQLVESGGGLVQPGGSLRLSCAASGLTFS<u>DYSMS</u>WVRQAPGKGLEWVA<u>IISGS GVIAHYVDSVKG</u>RFTISRDNSKNTVYLQMNSLRAEDTAVYYCRA<u>VSDWDEY</u>W GQGTQVTVSS(SEQ ID NO: 15)

> A6h1 variable region

EVQLLESGGGLVQAGGSLRLSCATSGRTFS<u>SAAMG</u>WFRQAPGKGLEWVA<u>ISW SFGSTYYADSVKG</u>RFTISRDNSKNTVYLQMNSLRPEDTAVYYCAA<u>DPSIETMGG GWDY</u>WGQGTQVTVSS(SEQ ID NO: 16)

2. TCE (T cell epitope)-removing, antibody deamidation-reducing, and antibody isomerization-reducing modifications

To reduce the potential immunogenic risk of the antibodies, TCE-removing modifications were performed. To improve the druggability of the antibodies, antibody deamidation and antibody isomerization site modifications were performed. The following sequences were obtained:

> A17m01 variable region

EVQLQESGGGLVQPGGSLRLSCTASGLTFS<u>DYSMS</u>WYRQAPGKGRELVA<u>IISGSG VIAHYVDSVKG</u>RFTISRDNAKNTVYLQMRSLTPEDRAVYYCRA<u>VSDWDDY</u>WG QGTQVTVSS(SEQ ID NO: 17)

> A17m02 variable region

EVQLQESGGGLVQPGGSLRLSCTASGLTFS<u>DYSMS</u>WYRQAPGKGRELVA<u>IISGSG VITHYVDSVKG</u>RFTISRDNSKNTVYLQMRSLTPEDRAVYYCRA<u>VSDWDDY</u>WG QGTQVTVSS(SEQ ID NO: 18)

> A17m03 variable region

EVQLQESGGGLVQPGGSLRLSCTASGLTFS<u>DYSMS</u>WYRQAPGKGRELVA<u>IISGSG GIAHYVDSVKG</u>RFTISRDNSKNTVYLQMRSLTPEDRAVYYCRA<u>VSDWDDY</u>WG QGTQVTVSS(SEQ ID NO: 19)

> A17m04 variable region

EVQLQESGGGLVQPGGSLRLSCTASGLTFS<u>DYSMS</u>WYRQAPGKGRELVA<u>IISGSG</u>
<u>VSAHYVDS</u>VKGRFTISRDNSKNTVYLQMRSLTPEDRAVYYCRA<u>VSDWDDY</u>WG
QGTQVTVSS(SEQ ID NO: 20)

> A17m05 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNSKNTVYLQMRSLTPEDRAVYYCRA<u>VSDWEDY</u>WG
QGTQVTVSS(SEQ ID NO: 21)

> A17m06 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNSKNTVYLQMRSLTPEDRAVYYCRA<u>VSDWDEY</u>WG
QGTQVTVSS(SEQ ID NO: 22)

> A17m07 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNAKNTVYLQMRSLTPEDRAVYYCRA<u>VSDWEDY</u>WG
QGTQVTVSS(SEQ ID NO: 23)

> A17m08 variable region

EVQLQESGGGLVQPGGSLRLSCTASGLTFS<u>DYSMS</u>WYRQAPGKGRELVA<u>IISGSG</u>
<u>VIAHYVDS</u>VKGRFTISRDNAKNTVYLQMRSLTPEDRAVYYCRA<u>VSDWDEY</u>WG
QGTQVTVSS(SEQ ID NO: 24)

> A17m09 variable region

EVQLQESGGGLVQPGGSLRLSCTASGLTFS<u>DYSMS</u>WYRQAPGKGRELVA<u>IISGSG</u>
<u>VITHYVDS</u>VKGRFTISRDNAKNTVYLQMRSLTPEDRAVYYCRA<u>VSDWEDY</u>WG
QGTQVTVSS(SEQ ID NO: 25)

> A17m10 variable region

EVQLQESGGGLVQPGGSLRLSCTASGLTFS<u>DYSMS</u>WYRQAPGKGRELVA<u>IISGSG</u>
<u>VITHYVDS</u>VKGRFTISRDNAKNTVYLQMRSLTPEDRAVYYCRA<u>VSDWDEY</u>WG
QGTQVTVSS(SEQ ID NO: 26)

> A17m11 variable region

EVQLQESGGGLVQPGGSLRLSCTASGLTFS<u>DYSMS</u>WYRQAPGKGRELVA<u>IISGSG</u>
<u>GIAHYVDS</u>VKGRFTISRDNAKNTVYLQMRSLTPEDRAVYYCRA<u>VSDWEDY</u>WG
QGTQVTVSS(SEQ ID NO: 27)

> A17m12 variable region

EVQLQESGGGLVQPGGSLRLSCTASGLTFS<u>DYSMS</u>WYRQAPGKGRELVA<u>IISGSG
GIAHYVDSVKG</u>RFTISRDNAKNTVYLQMRSLTPEDRAVYYCRA<u>VSDWDEY</u>WG
QGTQVTVSS(SEQ ID NO: 28)

> A17m13 variable region

EVQLQESGGGLVQPGGSLRLSCTASGLTFS<u>DYSMS</u>WYRQAPGKGRELVA<u>IISGSG
VSAHYVDSVKG</u>RFTISRDNAKNTVYLQMRSLTPEDRAVYYCRA<u>VSDWEDY</u>WG
QGTQVTVSS(SEQ ID NO: 29)

> A17m14 variable region

EVQLQESGGGLVQPGGSLRLSCTASGLTFS<u>DYSMS</u>WYRQAPGKGRELVA<u>IISGSG
VSAHYVDSVKG</u>RFTISRDNAKNTVYLQMRSLTPEDRAVYYCRA<u>VSDWDEY</u>WG
QGTQVTVSS(SEQ ID NO: 30)

> A17m15 variable region

EVQLQESGGGLVQPGGSLRLSCTASGLTFS<u>DYSMS</u>WYRQAPGKGRELVA<u>IISGSG
GITHYVDSVKG</u>RFTISRDNAKNTVYLQMRSLTPEDRAVYYCRA<u>VSDWEDY</u>WG
QGTQVTVSS(SEQ ID NO: 31)

A17m09 was selected, and the antibody sequence was further subjected to a germlining modification to improve the degree of humanization; the following sequences were obtained:

> A17m0901 variable region

EVQLVESGGGLVQPGGSLRLSCAASGFTFS<u>DYSMS</u>WYRQAPGKGRELVA<u>IISGS
GVITHYVDSVKG</u>RFTISRDNAKNTLYLQMRSLRAEDTAVYYCRA<u>VSDWEDY</u>W
GQGTQVTVSS(SEQ ID NO: 32)

> A17m0902 variable region

EVQLVESGGGLVQPGGSLRLSCAASGLTFS<u>DYSMS</u>WYRQAPGKGRELVA<u>IISGS
GVITHYVDSVKG</u>RFTISRDNAKNTLYLQMRSLRAEDTAVYYCRA<u>VSDWEDY</u>W
GQGTQVTVSS(SEQ ID NO: 33)

> A17m0903 variable region

EVQLVESGGGLVQPGGSLRLSCAASGLTFS<u>DYSMS</u>WYRQAPGKGRELVA<u>IISGS
GVITHYVDSVKG</u>RFTISRDNAKNTVYLQMRSLRAEDTAVYYCRA<u>VSDWEDY</u>W
GQGTQVTVSS(SEQ ID NO: 34)

> A17m0905 variable region

EVQLVESGGGLVQPGGSLRLSCAASGLTFS<u>DYSMS</u>WYRQAPGKGRELVA<u>IISGS
GVITHYVDSVKG</u>RFTISRDNAKNTVYLQMRSLRAEDRAVYYCRA<u>VSDWEDY</u>W
GQGTQVTVSS(SEQ ID NO: 35)

That is, the CDRs of the A17 of the present disclosure have the following general sequences:

CDR1: DYSMS (SEQ ID NO: 7)
CDR2: IISGSGX$_1$X$_2$X$_3$HYVDSVKG, wherein X$_1$ is selected from the group consisting of V and G; X$_2$ is selected from the group consisting of I and S; and X$_3$ is selected from the group consisting of T and A (SEQ ID NO: 36)
CDR3: VSDWX$_4$X$_5$Y, wherein X$_4$ is selected from the group consisting of D and E, and X$_5$ is selected from the group consisting of D and E (SEQ ID NO: 37)

Specifically, the CDR2 may be:

IISGSGVIAHYVDSVKG (SEQ ID NO: 8)
IISGSGVITHYVDSVKG (SEQ ID NO: 38)
IISGSGGIAHYVDSVKG (SEQ ID NO: 39)
IISGSGVSAHYVDSVKG (SEQ ID NO: 40)
IISGSGGITHYVDSVKG (SEQ ID NO: 94)
the CDR3 may be:

VSDWDDY (SEQ ID NO: 9)
VSDWEDY (SEQ ID NO: 41)
VSDWDEY (SEQ ID NO: 42)

**Example 4. PD-1/PD-L1 Reporter Gene System Test of Blocking of PD-1 by Anti-PD-1 Nanobodies**

The biological activity function of the PD-1 antibodies at the cellular level can be measured using the PD-1/PD-L1 NFAT gene reporter system (Cat: J1250, Promega). The system consists of two genetically engineered cell lines: a PD-1-overexpressing effector cell Jurkat (containing NFAT reporter gene luciferase) and a PD-L1-overexpressing antigen-presenting cell CHO-K1 cell. When the two cells are co-cultured, the interaction of PD-1/PD-L1 inhibits TCR-mediated NFAT activation and thereby inhibits the expression of NFAT reporter gene luciferase. When the PD-1/PD-L1 interaction is disrupted, TCR activation induces luciferase expression through the NFAT pathway. By adding Bio-Glo reagent and using a luminometer to quantitatively measure fluorescence, the extent of effector cell activation and the biological activity of an antibody can be measured.

CHO-K1 cells overexpressing PD-L1 were diluted to $4 \times 10^5$/mL and added to a 96-well plate at 100 $\mu$L/well, and the plate was incubated overnight. After the culture medium was pipetted off, 40 $\mu$L of $1.25 \times 10^6$/mL PD-1 Jurkat cells was added rapidly, along with 40 $\mu$L of anti-PD-1 nanobody solutions (diluted with 1640 + 2% FBS solution) with different concentrations. The plate was incubated at 37 °C for 6 h and cooled to room temperature. Bright-glo reagent (Cat: E2620, promega) was added at 40 $\mu$L/well. The plate was shaken in the dark at 350 rpm for 5 min and left to stand in the dark at room temperature for 5 min. Then fluorescence values were measured using a microplate reader, and IC$_{50}$ was calculated.

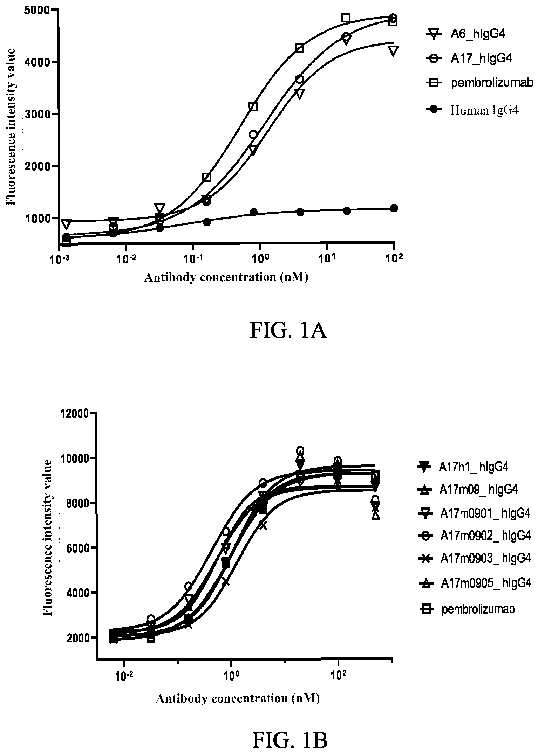

Pembrolizumab and hIgG4 isotype were used as controls. As shown in FIG. 1A and Table 6, the biologically functional activity IC$_{50}$ value of A17_hIgG4 is 1.199 nM, which is significantly better than those of the other antibodies obtained from the same screening in the present disclosure (for example, 7.62 for A3_hIgG4, and 3.208 for A 13_hIgG4; the sequences of A3_hIgG4 and A13_hIgG4 are not shown).

Table 6. The activity IC$_{50}$ values of the anti-PD-1 nanobodies, as measured using the PD-1/PD-L1 NFAT reporter gene system

| Antibody | IC$_{50}$ (nM) |
|---|---|
| A6_hIgG4 | 1.354 |
| A17_hIgG4 | 1.199 |
| Pembrolizumab | 0.5126 |
| hIgG4 | - |

Using the method described above, the anti-PD-1 nanobodies were functionally verified. Referring to FIG. 1B and Table 7, the $IC_{50}$ activity of A17h1_hIgG4 is similar to that of the positive antibody pembrolizumab. Among the modified candidate molecules, the antibodies A17m0901_hIgG4 and A17m0902_hIgG4 have biological activity $IC_{50}$ values of 0.5307 nM and 0.4352 nM, respectively, which are better than that of pembrolizumab.

Table 7. The activity $IC_{50}$ of the modified anti-PD-1 nanobodies, as measured using the PD-1/PD-L1 NFAT reporter gene system

| Antibody | $IC_{50}$ (nM) |
| --- | --- |
| A17h1_hIgG4 | 1.039 |
| A17m09_hIgG4 | 1.083 |
| A17m0901_hIgG4 | 0.5307 |
| A17m0902_hIgG4 | 0.4352 |
| A17m0903_hIgG4 | 1.284 |
| A17m0905_hIgG4 | 0.5588 |
| Pembrolizumab | 0.9756 |

## Example 5. Identification of Binding Affinities of Modified Anti-PD-1 Nanobodies for Antigen PD-1

The affinities of the anti-PD-1 nanobodies for PD-1 antigen were measured using surface plasmon resonance (SPR) technology; Biacore 8k (GE Healthcare) was used. In the experiment, a CM5 sensor chip was used, and HBS-EP + buffer solution (10 mM HEPES, 150 mM NaCl, 3 mM EDTA, 0.05% surfactant P20) was used as the mobile phase. A 30 $\mu$g/mL solution of anti-human IgG (Fc) antibody was prepared in 10 mM sodium acetate buffer (pH 5.0), and the Immobilization program was selected to automatically perform anti-human IgG (Fc) antibody channel amino coupling immobilization. The test antibodies were separately diluted in HBS-EP + buffer solution as ligands and captured using the anti-human IgG (Fc) antibody on the chip channels. The human or cynomolgus monkey PD-1 antigen protein (Sino Biological, 10377-H08H; 90311-C08H) was used as an analyte. The analyte was serially diluted 2-fold with HBS-EP + buffer solution, and the dilution was made to flow through the experimental and reference channels at a flow rate of 30 $\mu$L/min, with association for 1 min and dissociation for 15 min. The regeneration buffer 10 mM Glycine pH 1.5 (GE Healthcare, BR-1003-54) was run at a flow rate of 10 $\mu$L/min for 30 s. The association rate Ka, the dissociation rate Kd, and the dissociation constant (i.e., affinity $K_D$) were calculated. The results are shown in Tables 8 and 9.

Table 8. SPR affinity data for the anti-PD-1 nanobodies binding to human PD-1 antigen

| Antibody | Ka (1/Ms) | Kd (1/s) | $K_D$(M) |
| --- | --- | --- | --- |
| A17h1_hIgG4 | 4.30E+05 | 8.68E-04 | 2.02E-09 |
| A17m09_hIgG4 | 4.58E+05 | 1.63E-03 | 3.56E-09 |
| A17m0901_hIgG4 | 2.97E+05 | 1.92E-03 | 6.44E-09 |
| A17m0902_hIgG4 | 2.91E+05 | 1.77E-03 | 6.09E-09 |
| Pembrolizumab | 1.25E+06 | 3.89E-03 | 3.11E-09 |

Table 9. SPR affinity data for the anti-PD-1 nanobodies binding to cynomolgus monkey PD-1 antigen

| Antibody | Ka (1/Ms) | Kd (1/s) | $K_D$(M) |
| --- | --- | --- | --- |
| A17h1_hIgG4 | 4.44E+05 | 8.07E-04 | 1.82E-09 |
| A17m09_hIgG4 | 4.81E+05 | 8.43E-04 | 1.75E-09 |
| A17m0901_hIgG4 | 3.40E+05 | 1.13E-03 | 3.32E-09 |
| A17m0902_hIgG4 | 3.28E+05 | 1.08E-03 | 3.28E-09 |
| Pembrolizumab | 1.31E+06 | 1.15E-03 | 8.82E-10 |

The results of binding to human PD-1 antigen show that the humanized antibody A17h1_hIgG4 and the subsequently modified, optimized antibodies A17m09_hIgG4, A17m0901_hIgG4, and A17m0902_hIgG4 have similar $K_D$ values to pembrolizumab. A17hl_hIgG4, A17m09_hIgG4, A17m0901_hIgG4, and A17m0902_hIgG4 all dissociated more slowly than pembrolizumab. The antibodies A17h1_hIgG4, A17m09_hIgG4, A17m0901_hIgG4, and A17m0902_hIgG4 cross-bound to monkey PD-1, and the affinities of the antibodies binding to human and monkey PD-1 were similar.

## Example 6. DC:T Mix Lymphocyte Reaction Test of *In Vitro* Efficacy of Modified Anti-PD-1 Nanobodies (MLR)

Co-culture of mature dendritic cells (DCs) with allogeneic T cells can activate T cells and promote cytokine secretion by T cells. This process is inhibited by PD-1/PD-L1 signals, and a PD-1 antibody can be used to remove the inhibition and promote T cell activation. Thus, a DC:T mix lymphocyte reaction experiment can be used to test the *in vitro* efficacy of the PD-1 antibody.

Monocytes were sorted from fresh peripheral blood (PBMCs) of a healthy person using a sorting kit (cat: 19359, stemcell). After seven days of culture using a DC induction kit (cat: 10985, stemcell), the monocytes were induced to differentiate into mature DCs. Then allogeneic T cells were sorted from fresh PBMCs of another healthy person using a sorting kit (cat: 17951, stemcell). 5000 mature DCs were co-cultured with 50,000 allogeneic T cells, and a candidate antibody was added at a corresponding concentration. After six days of co-culture, the cell supernatant was collected and assayed for IFN-γ concentration using a Cisbio-HTRF IFN-γ assay kit (Cat: 62HIFNGPEH, Perkinelmer), and $EC_{50}$ was calculated.

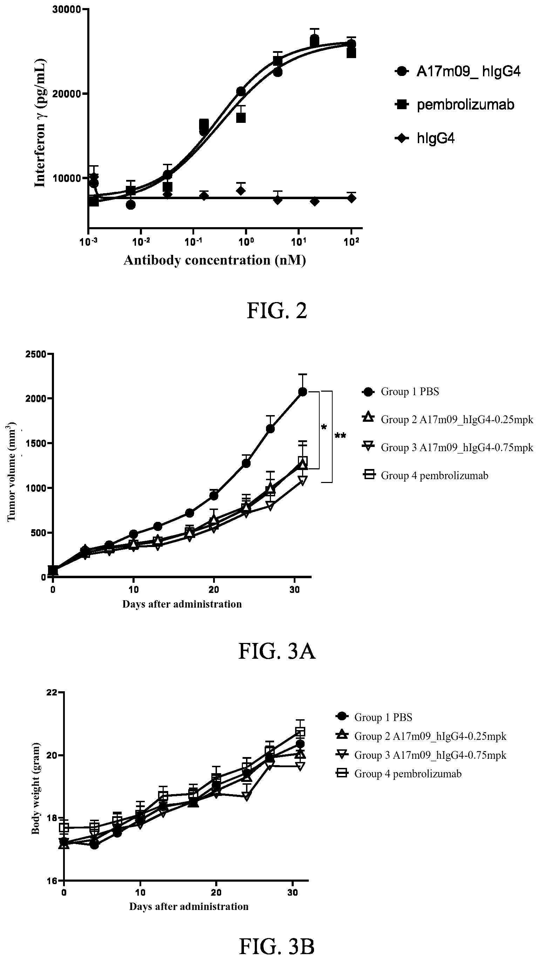

As shown in FIG. 2, A17m09_hIgG4 exhibited *in vitro* efficacy substantially in line with that of pembrolizumab in the MLR experiment. The $EC_{50}$ of A17m09_hIgG4 is 0.2931 nM, and the $EC_{50}$ of pembrolizumab is 0.3271 nM.

## Example 7. Inhibition of Tumor Growth in Colon Cancer Mouse Model by Anti-PD-1 Nanobodies

In this example, the *in vivo* anti-tumor efficacy of the PD-1 antibodies was verified using an MC38 tumor model of humanized PD-1 C57BL/6 mice.

The MC38 mouse colon cancer cell line was cultured with DMEM culture medium (10% FBS), and $5 \times 10^5$ MC38 cells were subcutaneously inoculated into humanized PD-1 C57BL/6 female mice (GemPharmatech). When the mean tumor volume of the mice reached about 80 mm³, the mice were randomized into groups of 8 and administered intraperitoneal injections of antibodies or controls. Tumor volume and body weight were measured twice a week, and data were recorded. The grouping and administration regimen for the experiment are shown in Table 10. Given that the molecular weight of A17m09_hIgG4 is about 75 kDa, which is only half of the normal IgG molecular weight, its dose was set to be half of that of pembrolizumab to achieve an equimolar dose.

As shown in FIGs. 3A and 3B, A17m09_hIgG4 exhibited *in vivo* anti-tumor efficacy in line with that of pembrolizumab at the same equimolar dose. Tumor volumes and tumor growth inhibition rates are shown in Table 11. At an equimolar dose, the tumor growth inhibition rate of A17m09_hIgG4 was slightly better than that of pembrolizumab.

Table 10. The grouping and administration regimen for the mouse experiment

| Group | Drug or control | Administration dose (mg/kg) | Route of administration | Frequency of administration |
|---|---|---|---|---|
| Group 1 | PBS | -- | i.p. | BIW*7 |
| Group 2 | A17m09_hIgG4 | 0.25 | | |
| Group 3 | A17m09_hIgG4 | 0.75 | | |
| Group 4 | Pembrolizumab | 0.5 | | |

Table 11. Mouse tumor volumes and tumor growth inhibition rates

| Group | Drug or control | Tumor volume/mm³ (Mean ± SEM) | | Tumor growth inhibition (%) | P value |
|---|---|---|---|---|---|
| | | Day 0 | Day 31 | Day 31 | |
| Group 1 | PBS | 79±4 | 2074±198 | / | / |
| Group 2 | A17m09_hIgG4 (0.25 mg/kg) | 79±4 | 1261±260 | 41% | 0.0263 |
| Group 3 | A17m09_hIgG4 (0.75 mg/kg) | 78±3 | 1078±186 | 50% | 0.0026 |

(continued)

| Group | Drug or control | Tumor volume/mm³ (Mean ± SEM) | | Tumor growth inhibition (%) | P value |
|---|---|---|---|---|---|
| | | Day 0 | Day 31 | Day 31 | |
| Group 4 | Pembrolizumab (0.5 mg/kg) | 78±3 | 1300±174 | 39% | 0.0109 |
| (Note: Compared to the control group (hIgG1), *P < 0.05, **P < 0.01, and ***P < 0.001 are considered to indicate significant differences.) | | | | | |

## Example 8. Construction of Fusion Proteins of Anti-PD-1 Antibody and IL-2 Mutant (PD-1-IL-2v)

Compared to the activity on NK and CD8⁺ T cells, wild-type IL-2 preferentially activates regulatory T cells (Tregs) due to the high expression of the high-affinity IL-2 receptor IL-2Rαβγ on Tregs. To reduce IL-2's preferential activation of Tregs, a common approach is to introduce mutations, such as F42A and F72G, into IL-2 to eliminate IL-2's binding to IL-2Rα. However, such an IL-2 mutant, IL-2v (F42A, L72G), can still systemically activate NK and CD8⁺ T cells, causing toxicity. To increase the specificity of IL-2, the mutation V91T was introduced to reduce IL-2's affinity for IL-2Rβγ, thereby reducing IL-2's activity on NK and CD8⁺ T cells. Furthermore, by conjugating the IL-2 mutant described above to an anti-PD-1 antibody, the IL-2 mutant was brought onto PD-1 positive NK and T cells to specifically activate these cells.

Fusion proteins of the anti-PD-1 antibody and the IL-2 mutant were constructed below according to the schematic of molecular formats shown in FIG. 4. For the Fcs of the molecules in FIG. 4, the Knob-in-Hole (KIH) technique was used; that is, the S354C and T366W (Eu numbering scheme) (constituting a Knob chain), and Y349C, T366S, L368A, and Y407V (Eu numbering scheme) (constituting a Hole chain) mutations were introduced into the two Fcs, respectively, to promote heterodimer formation. For ease of purification, H435R and Y436F (Eu numbering scheme) were additionally introduced into the Hole chain. Additionally, the L234A, L235A, and P329G mutations were introduced into both the Knob and Hole chains to eliminate ADCC effects. For heavy chains not paired with a light chain, the C220S mutation was introduced into the hinge region to remove free, unpaired cysteines. The IL-2 mutant, IL-2v (V91T), was used, and it comprises: 1) T3A, to remove potential glycosylation modifications during protein expression in mammalian cells; 2) R38E and F42A, to eliminate IL-2's binding to CD25 (i.e., IL-2Rα); 3) V91T, to reduce IL-2's binding to the intermediate-affinity IL-2 receptor IL-2Rβγ; and 4) C125A, to remove free cysteines in IL-2 to prevent intermolecular disulfide bond formation in IL-2.

In molecule A-V91T shown in A of FIG. 4, the anti-PD-1 nanobody A17m0902 is conjugated to the N-termini of the Knob and Hole chains via an IgG1 heavy chain hinge region. In molecule B-V91T shown in B of FIG. 4, two anti-PD-1 nanobodies A17m0902 are linked end-to-end by a $(G_4S)_2$ linker, and its C-terminus is then conjugated to the N-termini of the Knob and Hole chains via an IgG1 heavy chain hinge region. In molecule C-V91T shown in C of FIG. 4, the anti-PD-1 nanobody A17m0902 is conjugated to the N-terminus of the Hole chain via an IgG1 heavy chain hinge region, and the heavy chain of the corresponding Fab fragment of the anti-PD-1 antibody pembrolizumab is conjugated to the N-terminus of the Knob chain via an IgG1 heavy chain hinge region. In molecule D-V91T shown in D of FIG. 4, two anti-PD-1 nanobodies A17m0902 are linked end-to-end by a $(G_4S)_2$ linker, and its C-terminus is then conjugated to the N-terminus of the Hole chain via an IgG1 heavy chain hinge region; the heavy chain of the corresponding Fab fragment of the anti-PD-1 antibody pembrolizumab is conjugated to the N-terminus of the Knob chain via an IgG1 heavy chain hinge region. In molecule E-V91T shown in E of FIG. 4, the heavy chain of the corresponding Fab fragment of the anti-PD-1 antibody pembrolizumab is conjugated to the N-termini of the Knob and Hole chains via an IgG1 heavy chain hinge region, and the anti-PD-1 nanobody A17m0902 is conjugated to the N-terminus of the heavy chain of the corresponding Fab fragment of pembrolizumab via a $(G_4S)_2$ linker. In molecule F-V91T shown in F of FIG. 4, two anti-PD-1 nanobodies A17m0902 are linked end-to-end by a $(G_4S)_2$ linker, and its C-terminus is then conjugated to the N-terminus of the Hole chain via an IgG1 heavy chain hinge region. In the molecules of A-E in FIG. 4, IL-2v (V91T) is linked to the C-terminus of the Knob chain of the Fc by a $(G_4S)_3$ linker. In the molecule of F, IL-2v (V91T) is linked to the N-terminus of the Knob chain by a $G_4S$ linker.

The sequences of the molecules are as follows, with the Fc underlined and the linker in italics.

> The Knob chain of A-V91 T

EVQLQESGGGLVQPGGSLRLSCTASGLTFSDYSMSWYRQAPGKGRELVAIISGSG
VIAHYVDSVKGRFTISRDNSKNTVYLQMRSLTPEDRAVYYCRAVSDWDDYWG
QGTQVTVSSEPKSSDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALGAPIEKTISKAKGQPREPQVYTLPPCRDELTKNQVSL
WCLVKGFYPSDIAVEWESNGQPENNYKTTPPVLDSDGSFFLYSKLTVDKSRWQQ
GNVFSCSVMHEALHNHYTQKSLSLSPG*GGGGSGGGGSGGGGS*APASSSTKKTQ
LQLEHLLLDLQMILNGINNYKNPKLTEMLTAKFYMPKKATELKHLQCLEEELKP
LEEVLNLAQSKNFHLRPRDLISNINTIVLELKGSETTFMCEYADETATIVEFLNRW
ITFAQSIISTLT                         (SEQ ID NO: 43)

> The Hole chain of A-V91 T

EVQLQESGGGLVQPGGSLRLSCTASGLTFSDYSMSWYRQAPGKGRELVAIISGSG
VIAHYVDSVKGRFTISRDNSKNTVYLQMRSLTPEDRAVYYCRAVSDWDDYWG
QGTQVTVSSEPKSSDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALGAPIEKTISKAKGQPREPQVCTLPPSRDELTKNQVSLS
CAVKGFYPSDIAVEWESNGQPENNYKTTPPVLDSDGSFFLVSKLTVDKSRWQQG
NVFSCSVMHEALHNRFTQKSLSLSP                (SEQ ID NO: 44)

> The Knob chain of B-V91T

EVQLQESGGGLVQPGGSLRLSCTASGLTFSDYSMSWYRQAPGKGRELVAIISGSG
VIAHYVDSVKGRFTISRDNSKNTVYLQMRSLTPEDRAVYYCRAVSDWDDYWG
QGTQVTVSS*GGGGSGGGGS*EVQLQESGGGLVQPGGSLRLSCTASGLTFSDYSMS
WYRQAPGKGRELVAIISGSGVIAHYVDSVKGRFTISRDNSKNTVYLQMRSLTPE
DRAVYYCRAVSDWDDYWGQGTQVTVSSEPKSSDKTHTCPPCPAPEAAGGPSVF
LFPPKPKDTLMISRTPEVTCVVVDVSHEDPEVKFNWYVDGVEVHNAKTKPREE
QYNSTYRVVSVLTVLHQDWLNGKEYKCKVSNKALGAPIEKTISKAKGQPREPQ
VYTLPPCRDELTKNQVSLWCLVKGFYPSDIAVEWESNGQPENNYKTTPPVLDSD
GSFFLYSKLTVDKSRWQQGNVFSCSVMHEALHNHYTQKSLSLSPG*GGGGSGGG
GSGGGGS*APASSSTKKTQLQLEHLLLDLQMILNGINNYKNPKLTEMLTAKFYMP
KKATELKHLQCLEEELKPLEEVLNLAQSKNFHLRPRDLISNINTIVLELKGSETTF
MCEYADETATIVEFLNRWITFAQSIISTLT            (SEQ ID NO: 45)

> The Hole chain of B-V91T

EVQLQESGGGLVQPGGSLRLSCTASGLTFSDYSMSWYRQAPGKGRELVAIISGSG VIAHYVDSVKGRFTISRDNSKNTVYLQMRSLTPEDRAVYYCRAVSDWDDYWG QGTQVTVSS*GGGGSGGGGS*EVQLQESGGGLVQPGGSLRLSCTASGLTFSDYSMS WYRQAPGKGRELVAIISGSGVIAHYVDSVKGRFTISRDNSKNTVYLQMRSLTPE DRAVYYCRAVSDWDDYWGQGTQVTVSS<u>EPKSSDKTHTCPPCPAPEAAGGPSVF LFPPKPKDTLMISRTPEVTCVVVDVSHEDPEVKFNWYVDGVEVHNAKTKPREE QYNSTYRVVSVLTVLHQDWLNGKEYKCKVSNKALGAPIEKTISKAKGQPREPQ VCTLPPSRDELTKNQVSLSCAVKGFYPSDIAVEWESNGQPENNYKTTPPVLDSD GSFFLVSKLTVDKSRWQQGNVFSCSVMHEALHNRFTQKSLSLSP</u>(SEQ ID NO: 46)

> The Knob chain of C-V91T

QVQLVQSGVEVKKPGASVKVSCKASGYTFTNYYMYWVRQAPGQGLEWMGGI NPSNGGTNFNEKFKNRVTLTTDSSTTTAYMELKSLQFDDTAVYYCARRDYRFD MGFDYWGQGTTVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTK VDKKV<u>EPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALGAPIEKTISKAKGQPREPQVYTLPPCRDELTKNQVSLWCLV KGFYPSDIAVEWESNGQPENNYKTTPPVLDSDGSFFLYSKLTVDKSRWQQGNVF SCSVMHEALHNHYTQKSLSLSPG</u>*GGGGSGGGGSGGGGS*APASSSTKKTQLQLE HLLLDLQMILNGINNYKNPKLTEMLTAKFYMPKKATELKHLQCLEEELKPLEEV LNLAQSKNFHLRPRDLISNINTIVLELKGSETTFMCEYADETATIVEFLNRWITFA QSIISTLT (SEQ ID NO: 47)

The Hole chain of C-V91T is SEQ ID NO: 44.
> The light chain of C-V91T

EIVLTQSPATLSLSPGERATLSCRASKGVSTSGYSYLHWYQQKPGQAPRLLIYLA SYLESGVPARFSGSGSGTDFTLTISSLEPEDFAVYYCQHSRDLPLTFGGGTKVEIK RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 48)

The Knob chain of D-V91T is SEQ ID NO: 47, the Hole chain is SEQ ID NO: 46, and the light chain is SEQ ID NO: 48.

> The Knob chain of E-V91T

EVQLQESGGGLVQPGGSLRLSCTASGLTFSDYSMSWYRQAPGKGRELVAIISGSG
VIAHYVDSVKGRFTISRDNSKNTVYLQMRSLTPEDRAVYYCRAVSDWDDYWG
QGTQVTVSS*GGGGSGGGGS*QVQLVQSGVEVKKPGASVKVSCKASGYTFTNYY
MYWVRQAPGQGLEWMGGINPSNGGTNFNEKFKNRVTLTTDSSTTTAYMELKS
LQFDDTAVYYCARRDYRFDMGFDYWGQGTTVTVSSASTKGPSVFPLAPSSKST
SGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPEAAGGPSVFLFPP

KPKDTLMISRTPEVTCVVVDVSHEDPEVKFNWYVDGVEVHNAKTKPREEQYN
STYRVVSVLTVLHQDWLNGKEYKCKVSNKALGAPIEKTISKAKGQPREPQVYT
LPPCRDELTKNQVSLWCLVKGFYPSDIAVEWESNGQPENNYKTTPPVLDSDGSF
FLYSKLTVDKSRWQQGNVFSCSVMHEALHNHYTQKSLSLSPG*GGGGSGGGGSG
GGGS*APASSSTKKTQLQLEHLLLDLQMILNGINNYKNPKLTEMLTAKFYMPKKA
TELKHLQCLEEELKPLEEVLNLAQSKNFHLRPRDLISNINTIVLELKGSETTFMCE
YADETATIVEFLNRWITFAQSIISTLT                    (SEQ ID NO: 49)

> The Hole chain of E-V91T

EVQLQESGGGLVQPGGSLRLSCTASGLTFSDYSMSWYRQAPGKGRELVAIISGSG
VIAHYVDSVKGRFTISRDNSKNTVYLQMRSLTPEDRAVYYCRAVSDWDDYWG
QGTQVTVSS*GGGGSGGGGS*QVQLVQSGVEVKKPGASVKVSCKASGYTFTNYY
MYWVRQAPGQGLEWMGGINPSNGGTNFNEKFKNRVTLTTDSSTTTAYMELKS
LQFDDTAVYYCARRDYRFDMGFDYWGQGTTVTVSSASTKGPSVFPLAPSSKST
SGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALGAPIEKTISKAKGQPREPQVCT
LPPSRDELTKNQVSLSCAVKGFYPSDIAVEWESNGQPENNYKTTPPVLDSDGSFF
LVSKLTVDKSRWQQGNVFSCSVMHEALHNRFTQKSLSLSP
(SEQ ID NO: 50)

The light chain of E-V91T is SEQ ID NO: 48.
> The Knob chain of F-V91T

APASSSTKKTQLQLEHLLLDLQMILNGINNYKNPKLTEMLTAKFYMPKKATELK
HLQCLEEELKPLEEVLNLAQSKNFHLRPRDLISNINTIVLELKGSETTFMCEYAD
ETATIVEFLNRWITFAQSIISTLT*GGGGS*EPKSSDKTHTCPPCPAPEAAGGPSVFLF
PPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVY
TLPPCRDELTKNQVSLWCLVKGFYPSDIAVEWESNGQPENNYKTTPPVLDSDGS
FFLYSKLTVDKSRWQQGNVFSCSVMHEALHNHYTQKSLSLSPG(SEQ ID NO: 51)

The Hole chain of F-V91T is SEQ ID NO: 46.

The sequences of the pembrolizumab mentioned above are:

> The Fab heavy chain of pembrolizumab

QVQLVQSGVEVKKPGASVKVSCKASGYTFTNYYMYWVRQAPGQGLEWMGGI
NPSNGGTNFNEKFKNRVTLTTDSSTTTAYMELKSLQFDDTAVYYCARRDYRFD
MGFDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKV　　　(SEQ ID NO: 52)

> The Fab light chain of pembrolizumab

EIVLTQSPATLSLSPGERATLSCRASKGVSTSGYSYLHWYQQKPGQAPRLLIYLA
SYLESGVPARFSGSGSGTDFTLTISSLEPEDFAVYYCQHSRDLPLTFGGGTKVEIK
RTVAAPSVFIFPPSDEQLKSGTASVVCLLNNFYPREAKVQWKVDNALQSGNSQE

SVTEQDSKDSTYSLSSTLTLSKADYEKHKVYACEVTHQGLSSPVTKSFNRGEC
(SEQ ID NO: 53)

> The HCDR1 of pembrolizumab
NYYMY (SEQ ID NO: 54)
> The HCDR2 of pembrolizumab
GINPSNGGTNFNEKFKN (SEQ ID NO: 55)
> The HCDR3 of pembrolizumab
RDYRFDMGFDY (SEQ ID NO: 56)
> The LCDR1 of pembrolizumab
RASKGVSTSGYSYLH (SEQ ID NO: 57)
> The LCDR2 of pembrolizumab
LASYLES (SEQ ID NO: 58)
> The LCDR3 of pembrolizumab
QHSRDLPLT (SEQ ID NO: 59)

The IL-2v (V91T) sequence in the PD-1-IL-2v above is:

APASSSTKKTQLQLEHLLLDLQMILNGINNYKNPKLTEMLTAKFYMPKKATELK
HLQCLEEELKPLEEVLNLAQSKNFHLRPRDLISNINTIVLELKGSETTFMCEYAD
ETATIVEFLNRWITFAQSIISTLT　　　　(SEQ ID NO: 60)

That is, IL-2v (V91T) comprises T3A/R38E/F42A/V91 TIC 125A.

The fusion proteins described above were expressed in HEK293 cells and purified through Protein A. The molecular weights of the purified products were consistent with the expectations as confirmed by SDS-PAGE, indicating that the target proteins had been prepared and obtained.

## Example 9. Assay for M07e Cell Proliferation Promoting Activity of PD-1-IL-2v

M07e is a human cell line that expresses only IL-2Rβ and IL-2Rγ but does not express IL-2Rα. To measure the activity and PD-1 expression dependency of the PD-1-IL-2v (V91T) fusion proteins of different formats described above, human PD-1 (SEQ ID NO: 1) was overexpressed in the M07e cell strain to obtain an M07e-hPD-1 cell strain, and the effects of the fusion proteins described above on cell proliferation were assessed in the M07e and M07e-hPD-1 cell strains.

Complete culture medium: RPMI 1640 + 2 mM L-glutamine + 1 mM sodium pyruvate + 10% fetal bovine serum + 15 ng/mL GM-CSF; basic culture medium: RPMI 1640 + 2 mM L-glutamine + 1 mM sodium pyruvate + 10% fetal bovine serum.

M07e cell proliferation experiment: M07e or M07e-hPD1 cells were cultured in complete culture medium at 37 °C with 5% $CO_2$. The cells were passaged, collected by centrifugation after 1-2 days, washed three times with PBS, and resuspended in basic culture medium to prepare a cell suspension containing $6.0 \times 10^5$ cells per mL. The suspension was added to a 96-well plate at 50 μL/well. 50 μL of the test drug, prepared at 2× the corresponding concentration in basic culture medium, was added. After 72 hours of cell culture, the lysis buffer CELLTITER-Glo reagent (Promega) was added at 100 μL/well and mixed well, and absorbance was measured at the wavelength of 570 nm with 630 nm as the reference wavelength using a microplate reader.

Data on the proliferation activity of the PD-1-IL-2v (V91T) fusion proteins of different formats on M07e and M07e-hPD-1 cells are shown in Table 12 and FIGs. 5A and 5B. A PEGylated IL-2 mutant (PEG-IL-2v) was used as a positive control. The IL-2 mutant comprises the T3A, F42A, L72G, N26Q, N29S, N71Q, and C125A mutations. The mutant does not bind to IL-2Rα but can bind to the intermediate-affinity IL-2 receptor IL-2Rβγ.

The results show that PEG-IL-2v exhibited comparable proliferation activity on M07e and M07e-hPD-1 cells; the activity of the PD-1-IL-2 (V91T) of different formats on M07e-hPD-1 was significantly stronger than their activity on M07e cell proliferation, indicating that the anti-PD-1 antibody can increase the selectivity of the IL-2 mutant for PD-1 positive cells. Additionally, the PD-1-IL-2 (V91T) of different formats exhibited comparable proliferation activity on M07e cells; however, in terms of activity on M07e-hPD-1 proliferation, C-V91T was slightly stronger than A-V91T, and A-V91T was stronger than B-V91T and E-V91T.

Table 12. The proliferation activity of PD-1-IL-2v (V91T) on M07e and M07e-hPD-1 cells

| Name | M07e | | M07e-hPD1 | |
| --- | --- | --- | --- | --- |
| | Maximal fluorescence value (Max Lum) | $EC_{50}$ (nM) | Maximal fluorescence value (Max Lum) | $EC_{50}$ (nM) |
| PEG-IL-2v | 4.45E+06 | 20.88 | 7.94E+06 | 13.34 |
| A-V91T | 4.24E+06 | 84.58 | 7.02E+06 | 0.032 |
| B-V91T | 3.85E+06 | 50.08 | 7.064E+06 | 0.693 |
| C-V91T | 3.90E+06 | 54.26 | 6.72E+06 | 0.010 |
| E-V91T | 4.11E+06 | 84.58 | 7.60E+06 | 0.761 |

### Example 10. Construction of More PD-1-IL-2v

PD-1-IL-2v (V91T), despite being less active than PEG-IL-2v in M07e that does not express PD-1, was still able to stimulate cell proliferation, indicating that IL-2v (V91T) can still relatively strongly bind to the intermediate-affinity IL-2 receptor IL-2Rβγ, and that PD-1-IL-2 (V91T) may still systemically activate NK and CD8[+] T cells and cause toxicity. To further reduce the toxicity of PD-1-IL-2v and make the activity of IL-2 more dependent on PD-1 expression, PD-1-IL-2v with different IL-2 mutants was constructed based on the A-V91T molecular format. All the IL-2 mutants comprise the F42A and L72G mutations to eliminate IL-2's binding to IL-2Rα. Additionally, considering stability, N26Q, N29S, and N71Q were added into the IL-2 mutants to remove potential deamidation modifications in IL-2, thereby increasing the stability of IL-2. Moreover, the mutants comprise their respective different unique mutations, as shown in Table 13, which reduce the affinity of IL-2 for the intermediate-affinity receptor IL-2Rβγ to varying extents.

**EP 4 477 673 A1**

Table 13. IL-2 mutations in PD-1-IL-2v

| Name | Common mutations in IL-2 | Unique mutations in IL-2 | Corresponding sequence number |
|---|---|---|---|
| A-PC | T3A/F42A/L72G/N26Q/ N29S/N71Q/C125A | / | SEQ ID NO:61 |
| A-V91T-02 | | V91T | SEQ ID NO:62 |
| A-D20A | | D20A | SEQ ID NO:63 |
| A-D20N | | D20N | SEQ ID NO:64 |
| A-N88D | | N88D | SEQ ID NO:65 |
| A-N88R | | N88R | SEQ ID NO:66 |
| A-Q126D | | Q126D | SEQ ID NO:67 |
| A-H16A/D84S | | H16A/D84S | SEQ ID NO:68 |

> The IL-2 sequence in A-PC

APASSSTKKTQLQLEHLLLDLQMILQGISNYKNPKLTRMLTAKFYMPKKATELK HLQCLEEELKPLEEVLQGAQSKNFHLRPRDLISNINVIVLELKGSETTFMCEYAD ETATIVEFLNRWITFAQSIISTLT (SEQ ID NO: 61)

> The IL-2 sequence in A-V91 T-02

APASSSTKKTQLQLEHLLLDLQMILQGISNYKNPKLTRMLTAKFYMPKKATELK HLQCLEEELKPLEEVLQGAQSKNFHLRPRDLISNINTIVLELKGSETTFMCEYAD ETATIVEFLNRWITFAQSIISTLT (SEQ ID NO: 62)

> The IL-2 sequence in A-D20A

APASSSTKKTQLQLEHLLLALQMILQGISNYKNPKLTRMLTAKFYMPKKATELK HLQCLEEELKPLEEVLQGAQSKNFHLRPRDLISNINVIVLELKGSETTFMCEYAD ETATIVEFLNRWITFAQSIISTLT (SEQ ID NO: 63)

> The IL-2 sequence in A-D20N

APASSSTKKTQLQLEHLLLNLQMILQGISNYKNPKLTRMLTAKFYMPKKATELK HLQCLEEELKPLEEVLQGAQSKNFHLRPRDLISNINVIVLELKGSETTFMCEYAD ETATIVEFLNRWITFAQSIISTLT (SEQ ID NO: 64)

> The IL-2 sequence in A-N88D

APASSSTKKTQLQLEHLLLDLQMILQGISNYKNPKLTRMLTAKFYMPKKATELK HLQCLEEELKPLEEVLQGAQSKNFHLRPRDLISDINVIVLELKGSETTFMCEYAD ETATIVEFLNRWITFAQSIISTLT (SEQ ID NO: 65)

> The IL-2 sequence in A-N88R

48

APASSSTKKTQLQLEHLLLDLQMILQGISNYKNPKLTRMLTAKFYMPKKATELK
HLQCLEEELKPLEEVLQGAQSKNFHLRPRDLISRINVIVLELKGSETTFMCEYAD
ETATIVEFLNRWITFAQSIISTLT (SEQ ID NO: 66)

> The IL-2 sequence in A-Q126D

APASSSTKKTQLQLEHLLLDLQMILQGISNYKNPKLTRMLTAKFYMPKKATELK
HLQCLEEELKPLEEVLQGAQSKNFHLRPRDLISNINVIVLELKGSETTFMCEYAD
ETATIVEFLNRWITFADSIISTLT (SEQ ID NO: 67)

> The IL-2 sequence in A-H16A/D84S

APASSSTKKTQLQLEALLLDLQMILQGISNYKNPKLTRMLTAKFYMPKKATELK
HLQCLEEELKPLEEVLQGAQSKNFHLRPRSLISNINVIVLELKGSETTFMCEYAD
ETATIVEFLNRWITFAQSIISTLT (SEQ ID NO: 68)

The L234A and L235A mutations were introduced into the Fc to eliminate ADCC effects. The S354C, T366W (constituting the Knob chain), and Y349C, and T366S, L368A, and Y407V (Eu numbering scheme) (constituting the Hole chain) mutations were introduced to promote heterodimer formation. Additionally, the C220S mutation was introduced into the hinge region. For ease of purification, the antibody may comprise the H435R and Y436F (Eu numbering scheme) mutations in the Hole chain. Furthermore, the amino acids at positions 356 and 358 of the Hole chains of the Fc regions of these antibodies may be D356 and L358, or E356 and M358 (Eu numbering scheme), respectively, which appeared in different alleles and did not affect antibody function.

> The Knob chain of the Fc (D356, L358)

EPKSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPCRDELTKNQVSLWCLVKGFYPSD
IAVEWESNGQPENNYKTTPPVLDSDGSFFLYSKLTVDKSRWQQGNVFSCSVMH
EALHNHYTQKSLSLSPG          (SEQ ID NO: 69)

> The Hole chain of the Fc (D356, L358)

EPKSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVCTLPPSRDELTKNQVSLSCAVKGFYPSDI
AVEWESNGQPENNYKTTPPVLDSDGSFFLVSKLTVDKSRWQQGNVFSCSVMHE
ALHNHYTQKSLSLSP          (SEQ ID NO: 70)

> The Knob chain of the Fc (E356, M358)

EPKSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPCREEMTKNQVSLWCLVKGFYPS
DIAVEWESNGQPENNYKTTPPVLDSDGSFFLYSKLTVDKSRWQQGNVFSCSVM
HEALHNHYTQKSLSLSPG          (SEQ ID NO: 71)

> The Hole chain of the Fc (E356, M358)

EPKSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVCTLPPSREEMTKNQVSLSCAVKGFYPSD
IAVEWESNGQPENNYKTTPPVLDSDGSFFLVSKLTVDKSRWQQGNVFSCSVMH
EALHNHYTQKSLSLSPG (SEQ ID NO: 72)

Illustratively, the full-length sequences of A-D20A and A-N88D are shown, with the Fc underlined and the linker in italics.

> The Knob chain of A-D20A (D356, L358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW

GQGTQVTVSSEPKSSDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQG
NVFSCSVMHEALHNHYTQKSLSLSPG*GGGGSGGGGSGGGGS*APASSSTKKTQL
QLEHLLLALQMILQGISNYKNPKLTRMLTAKFYMPKKATELKHLQCLEEELKPL
EEVLQGAQSKNFHLRPRDLISNINVIVLELKGSETTFMCEYADETATIVEFLNRWI
TFAQSIISTLT (SEQ ID NO: 73)

> The Hole chain of A-D20A (D356, L358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSSEPKSSDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSRDELTKNQVSLS
CAVKGFYPSDIAVEWESNGQPENNYKTTPPVLDSDGSFFLVSKLTVDKSRWQQG
NVFSCSVMHEALHNRFTQKSLSLSP (SEQ ID NO: 74)

> The Knob chain of A-D20A (E356, M358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVYTLPPCREEMTKNQVSL</u>
<u>WCLVKGFYPSDIAVEWESNGQPENNYKTTPPVLDSDGSFFLYSKLTVDKSRWQQ</u>
<u>GNVFSCSVMHEALHNHYTQKSLSLSPG</u>*GGGGSGGGGSGGGGS*APASSSTKKTQ
LQLEHLLLALQMILQGISNYKNPKLTRMLTAKFYMPKKATELKHLQCLEEELKP
LEEVLQGAQSKNFHLRPRDLISNINVIVLELKGSETTFMCEYADETATIVEFLNR
WITFAQSIISTLT    (SEQ ID NO: 75)

> The Hole chain of A-D20A (E356, M358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVCTLPPSREEMTKNQVSLS</u>
<u>CAVKGFYPSDIAVEWESNGQPENNYKTTPPVLDSDGSFFLVSKLTVDKSRWQQG</u>
<u>NVFSCSVMHEALHNHYTQKSLSLSPG</u>    (SEQ ID NO: 76)

> The Knob chain of A-N88D (D356, L358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVYTLPPCRDELTKNQVSLW</u>

<u>CLVKGFYPSDIAVEWESNGQPENNYKTTPPVLDSDGSFFLYSKLTVDKSRWQQG</u>
<u>NVFSCSVMHEALHNHYTQKSLSLSPG</u>*GGGGSGGGGSGGGGS*APASSSTKKTQL
QLEHLLLDLQMILQGISNYKNPKLTRMLTAKFYMPKKATELKHLQCLEEELKPL
EEVLQGAQSKNFHLRPRDLISDINVIVLELKGSETTFMCEYADETATIVEFLNRWI
TFAQSIISTLT    (SEQ ID NO: 77)

> The Hole chain ofA-N88D (D356, L358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVCTLPPSRDELTKNQVSLS</u>
<u>CAVKGFYPSDIAVEWESNGQPENNYKTTPPVLDSDGSFFLVSKLTVDKSRWQQG</u>
<u>NVFSCSVMHEALHNHYTQKSLSLSP</u>          (SEQ ID NO: 78)

> The Knob chain of A-N88D (E356, M358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVYTLPPCREEMTKNQVSL</u>
<u>WCLVKGFYPSDIAVEWESNGQPENNYKTTPPVLDSDGSFFLYSKLTVDKSRWQQ</u>
<u>GNVFSCSVMHEALHNHYTQKSLSLSPG</u>*GGGGSGGGGSGGGGS*APASSSTKKTQ
LQLEHLLLDLQMILQGISNYKNPKLTRMLTAKFYMPKKATELKHLQCLEEELKP
LEEVLQGAQSKNFHLRPRDLISDINVIVLELKGSETTFMCEYADETATIVEFLNR
WITFAQSIISTLT          (SEQ ID NO: 79)

> The Hole chain ofA-N88D (E356, M358)

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWEDYW
GQGTQVTVSS<u>EPKSSDKTHTCPPCPAPEAAGGPSVFLFPPKPKDTLMISRTPEVT</u>
<u>CVVVDVSHEDPEVKFNWYVDGVEVHNAKTKPREEQYNSTYRVVSVLTVLHQD</u>
<u>WLNGKEYKCKVSNKALPAPIEKTISKAKGQPREPQVCTLPPSREEMTKNQVSLS</u>
<u>CAVKGFYPSDIAVEWESNGQPENNYKTTPPVLDSDGSFFLVSKLTVDKSRWQQG</u>
<u>NVFSCSVMHEALHNHYTQKSLSLSPG</u>          (SEQ ID NO: 80)

Additionally, to better demonstrate the PD-1 dependency of PD-1-IL-2v, the anti-PD-1 antibody moieties of some PD-1-IL-2v were replaced with antibodies against unrelated antigens, and the mutations in the IL-2 moieties were kept. This resulted in IgG-PC, IgG-V91T (comprising V91T in IL-2), IgG-D20A (comprising D20A in IL-2), and IgG-N88D (comprising N88D in IL-2). The difference between IgG-PC and IgG-V91T, IgG-D20A, or IgG-N88D lay only in the IL-2 portion. The sequences are as follows:

> The Knob chain of IgG-PC

QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALGAPIEKTISKAKGQPREPQVYTLPPCRDELTKNQVSLWCLVKGFYPS
DIAVEWESNGQPENNYKTTPPVLDSDGSFFLYSKLTVDKSRWQQGNVFSCSVM
HEALHNHYTQKSLSLSPG*GGGGSGGGGSGGGGS*APASSSTKKTQLQLEHLLLDL
QMILQGISNYKNPKLTRMLTAKFYMPKKATELKHLQCLEEELKPLEEVLQGAQS
KNFHLRPRDLISNINVIVLELKGSETTFMCEYADETATIVEFLNRWITFAQSIISTLT
(SEQ ID NO: 81)

> The Knob chain of IgG-V91T

QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALGAPIEKTISKAKGQPREPQVYTLPPCRDELTKNQVSLWCLVKGFYPS
DIAVEWESNGQPENNYKTTPPVLDSDGSFFLYSKLTVDKSRWQQGNVFSCSVM
HEALHNHYTQKSLSLSPG*GGGGSGGGGSGGGGS*APASSSTKKTQLQLEHLLLDL
QMILNGINNYKNPKLTEMLTAKFYMPKKATELKHLQCLEEELKPLEEVLNLAQS
KNFHLRPRDLISNINTIVLELKGSETTFMCEYADETATIVEFLNRWITFAQSIISTLT
(SEQ ID NO: 82)

> The Knob chain of IgG-N88D

QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALGAPIEKTISKAKGQPREPQVYTLPPCRDELTKNQVSLWCLVKGFYPS
DIAVEWESNGQPENNYKTTPPVLDSDGSFFLYSKLTVDKSRWQQGNVFSCSVM
HEALHNHYTQKSLSLSPG*GGGGSGGGGSGGGGS*APASSSTKKTQLQLEHLLLDL
QMILQGISNYKNPKLTRMLTAKFYMPKKATELKHLQCLEEELKPLEEVLQGAQS
KNFHLRPRDLISDINVIVLELKGSETTFMCEYADETATIVEFLNRWITFAQSIISTLT
(SEQ ID NO: 83)

> The Knob chain of IgG-D20A

QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY

WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALGAPIEKTISKAKGQPREPQVYTLPPCRDELTKNQVSLWCLVKGFYPS
DIAVEWESNGQPENNYKTTPPVLDSDGSFFLYSKLTVDKSRWQQGNVFSCSVM
HEALHNHYTQKSLSLSPG*GGGGSGGGGSGGGGS*APASSSTKKTQLQLEHLLLAL
QMILQGISNYKNPKLTRMLTAKFYMPKKATELKHLQCLEEELKPLEEVLQGAQS
KNFHLRPRDLISNINVIVLELKGSETTFMCEYADETATIVEFLNRWITFAQSIISTLT
(SEQ ID NO: 84)

> The Hole chain of IgG-PC, IgG-V91T, IgG-D20A, or IgG-N88D:

QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALGAPIEKTISKAKGQPREPQVCTLPPSRDELTKNQVSLSCAVKGFYPSD
IAVEWESNGQPENNYKTTPPVLDSDGSFFLVSKLTVDKSRWQQGNVFSCSVMH
EALHNRFTQKSLSLSP     (SEQ ID NO: 85)

> The light chain of IgG-PC, IgG-V91T, IgG-D20A, or IgG-N88D

DIQMTQSPSSLSASVGDRVTMSCKSSQSLLNSGDQKNYLTWYQQKPGKAPKLLI
YWASTGESGVPSRFSGSGSGTDFTFTISSLQPEDIATYYCQNDYSYPWTFGQGTK
VEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC    (SEQ ID NO: 86)

The fusion proteins described above were expressed in HEK293 cells and purified through Protein A. The molecular weights of the purified products were consistent with the expectations as confirmed by SDS-PAGE, indicating that the target proteins had been obtained.

Additionally, the wild-type IL-2 sequence of the present disclosure is:

> wt IL-2

APTSSSTKKTQLQLEHLLLDLQMILNGINNYKNPKLTRMLTFKFYMPKKATELK
HLQCLEEELKPLEEVLNLAQSKNFHLRPRDLISNINVIVLELKGSETTFMCEYAD
ETATIVEFLNRWITFCQSIISTLT (SEQ ID NO: 87)

**Example 11. Assay for M07e Cell Proliferation Promoting Activity of More PD-1-IL-2v**

To measure the activity and PD-1 expression dependency of the PD-1-IL-2v fusion proteins with different IL-2 mutants described above, the effects of the fusion proteins of Example 10 on cell proliferation were assessed in the M07e and M07e-hPD-1 cell strains.

See Example 9 for the experimental method. The relative cell proliferation activity of PD-1-IL-2v was calculated as follows:

$$Activity_{PD-1-IL-2v}^{Conc} = 100\% * \frac{Lum_{PD-1-IL-2v}^{Conc} - Lum_{Medium}}{Lum_{A-PC}^{1000\,nM} - Lum_{Medium}},$$

wherein $Activity_{PD-1-IL-2v}^{Conc}$ represents the activity of PD-1-IL-2v at the concentration of conc, $Lum_{PD-1-IL-2v}^{Conc}$ represents the luminescence intensity of the cell lysate when stimulated by PD-1-IL-2v at the concentration of conc, $Lum_{A-PC}^{1000\,nM}$ represents the luminescence intensity of the cell lysate after the cells were stimulated by the positive control molecule A-PC at the concentration of 1000 nM in the M07e or M07e-hPD-1 cell strain, and $Lum_{Medium}$ represents the background luminescence intensity when no PD-1-IL-2v or PEG-IL-2v stimulation was used in the M07e or M07e-hPD-1 cell strain and only the culture medium was added.

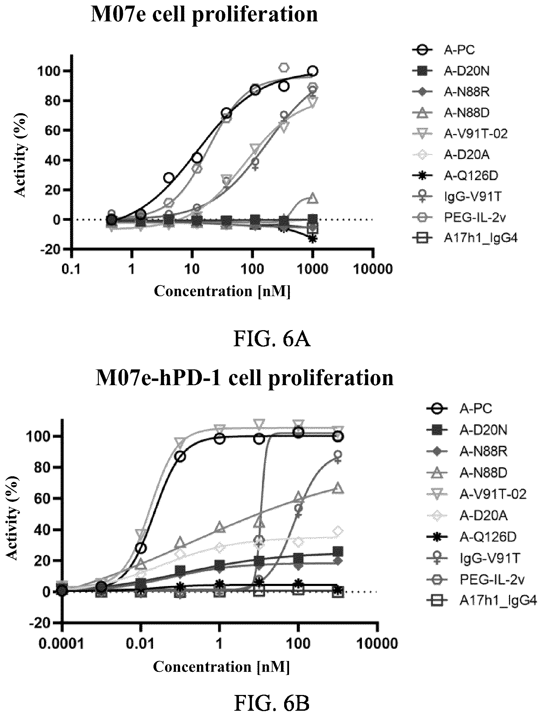

The experimental results are shown in Table 14 and FIGs. 6A and 6B. The results show that: 1) In M07e cells, A-PC and PEG-IL-2v exhibited comparable activity, indicating that in the absence of PD-1 overexpression, the activity of the IL-2 binding to the antibody is consistent with that of the PEG-conjugated IL-2; A-V91T and IgG-V91T exhibited comparable activity, and their activity was weaker than that of A-PC, indicating that V91 T indeed reduced the activity of IL-2 but it still can stimulate M07e proliferation; other mutants, including D20A, D20N, N88R, N88D, and Q126D, substantially did not activate M07e cells, with only A-N88D showing weak activity at the concentration of 1000 nM.2) In M07e-hPD-1 cells, due to the overexpression of PD-1 on the cells, the activity of the PD-1-IL-2v fusion proteins at relatively low concentrations far exceeded that of IgG-V91T and PEG-IL-2v, indicating that the anti-PD-1 antibody greatly increased the dependency of the cell proliferation promoting activity of PD-1-IL-2v on PD-1 expression; the other PD-1-IL-2v fusion proteins exhibited slightly lower maximum activity on M07e-hPD-1 cell proliferation than A-PC and A-V91T-02. 3) According to a comparison of the activity of the PD-1-IL-2v fusion proteins in the M07e and M07e-hPD-1 cell proliferation experiments, PD-1-IL-2v with the D20A, D20N, N88R, N88D, or Q126D mutation substantially did not activate M07e cells but strongly stimulated M07e-hPD-1 cell proliferation, demonstrating that PD-1-IL-2v reduced the activation of PD-1 negative cells while retaining the selective activation of PD-1 positive cells; although A-PC and A-V91T-02 have selective activation of PD-1 positive cells, they can also activate PD-1 negative cells.

To better demonstrate the PD-1 dependency of PD-1-IL-2v (N88D) and PD-1-IL-2v (D20A), the effects of A-N88D and IgG-N88D, as well as A-D20A and IgG-D20, on cell proliferation were compared in the M07e-hPD-1 cell strain.

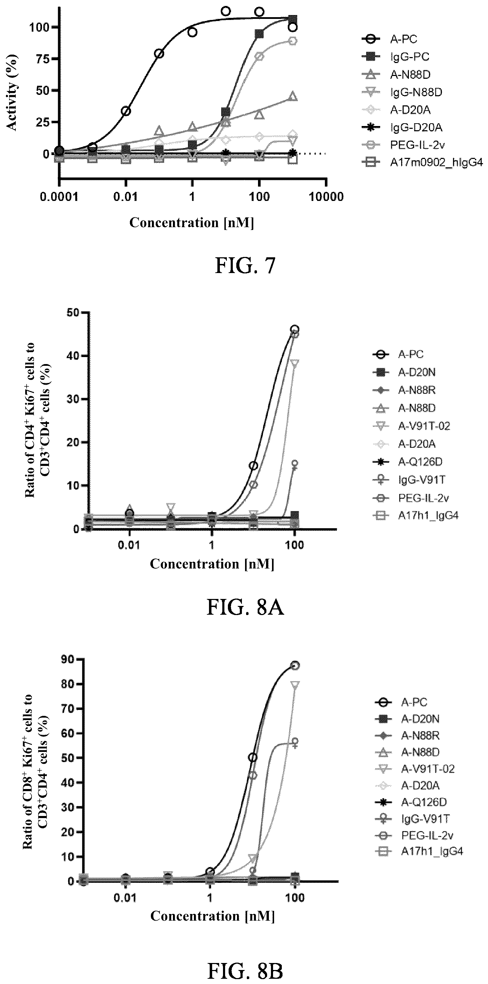

The experimental results are shown in Table 15 and FIG. 7. The results show that at the relatively high concentration (1000 nM), IgG-N88D and IgG-D20A still cannot activate M07e-hPD-1 cell proliferation; however, A-N88D and A-D20A can activate M07e-hPD-1 cell proliferation at relatively low concentrations.

Table 14. The proliferation promoting activity of PD-1-IL-2v with different IL-2 mutations on M07e and M07e-hPD-1 cells

| Name | M07e cell proliferation activity (%) | | | | | | | |
|---|---|---|---|---|---|---|---|---|
| | 1000 nM | 333 nM | 111 nM | 37 nM | 12 nM | 4.1 nM | 1.4 nM | 0.46 nM |
| PEG-IL-2v | 89.0 | 102.2 | 88.3 | 68.2 | 34.2 | 11.2 | 2.6 | 0.7 |
| IgG-V91T | 85.3 | 68.5 | 36.9 | 23.9 | 6.1 | 1.7 | -0.1 | 2.0 |
| A-PC | 100.0 | 89.8 | 87.2 | 71.7 | 41.6 | 28.1 | 3.6 | 0.1 |
| A-V91T-02 | 78.7 | 62.0 | 47.3 | 26.6 | 1.7 | -3.3 | -4.4 | -4.6 |
| A-D20A | 0.6 | -1.9 | -2.4 | -3.7 | -3.0 | -1.6 | -2.6 | -3.0 |
| A-D20N | 0.3 | -0.9 | 0.1 | -0.6 | -0.7 | 0.2 | -1.3 | -0.8 |
| A-N88R | -5.1 | -5.2 | -4.5 | -3.0 | -2.2 | -1.9 | -1.3 | -0.9 |
| A-N88D | 14.6 | 0.2 | -1.8 | -2.8 | -1.9 | -1.5 | -1.1 | -1.7 |
| A-Q126D | -12.8 | -5.8 | -2.9 | -2.7 | -0.5 | -1.7 | -0.6 | -0.8 |
| A17h1_IgG4 | -5.8 | -3.5 | -3.0 | -1.2 | -1.3 | -0.2 | 0.0 | -1.4 |
| Name | M07e-hPD-1 cell proliferation activity (%) | | | | | | | |
| | 1000 nM | 100 nM | 10 nM | 1 nM | 0.1 nM | 0.01 nM | 0.001 nM | 0.0001 nM |
| PEG-IL-2v | 100.1 | 104.0 | 33.3 | 3.3 | 0.4 | 0.5 | 1.1 | 1.9 |
| IgG-V91T | 86.3 | 51.7 | 6.1 | 1.1 | -0.4 | 0.2 | 0.5 | 1.6 |
| A-PC | 100 | 102 | 98 | 99 | 87 | 28 | 3 | 1 |
| A-V91T-02 | 102.9 | 107.0 | 107.4 | 103.9 | 95.5 | 36.6 | 5.1 | 3.4 |
| A-D20A | 39.2 | 32.0 | 30.2 | 28.5 | 24.2 | 11.4 | 2.9 | 2.3 |
| A-D20N | 26.0 | 21.6 | 19.9 | 17.2 | 13.6 | 5.4 | 2.0 | 1.8 |
| A-N88R | 20.0 | 16.8 | 16.9 | 14.9 | 11.5 | 4.8 | 1.8 | 1.3 |
| A-N88D | 66.9 | 61.1 | 45.0 | 41.8 | 32.1 | 18.1 | 3.3 | 2.8 |
| A-Q126D | 1.1 | 5.4 | 6.3 | 5.1 | 3.3 | 1.6 | 1.4 | -0.1 |
| A17h1_IgG4 | -0.1 | 1.6 | 0.7 | 0.3 | 0.0 | 0.3 | 0.2 | 1.8 |

Table 15. The proliferation promoting activity of PD-1-IL-2v or IgG-IL-2v on M07e-hPD-1 cells

| Name | M07e-hPD-1 cell proliferation ratio (%) | | | | | | | |
|---|---|---|---|---|---|---|---|---|
| | 1000 nM | 100 nM | 10nM | 1nM | 0.1 nM | 0.01 nM | 0.001 nM | 0.0001 nM |
| A-PC | 100.0 | 112.0 | 112.6 | 95.9 | 79.0 | 33.7 | 4.9 | 2.4 |
| IgG-PC | 106.0 | 94.7 | 32.9 | 6.9 | 2.9 | 3.1 | 1.8 | 1.2 |
| A-N88D | 45.5 | 31.1 | 25.2 | 21.3 | 18.3 | 4.8 | 0.0 | 0.8 |
| IgG-N88D | 9.7 | -1.3 | -5.6 | -1.3 | -0.5 | -1.5 | -0.6 | -1.2 |
| A-D20A | 15.4 | 13.5 | 10.8 | 10.8 | 8.4 | 2.6 | -0.3 | 0.6 |
| IgG-D20A | 0.6 | -0.3 | 0.3 | -0.1 | -0.2 | 0.1 | 0.0 | 1.1 |
| PEG-IL-2v | 89.1 | 76.7 | 24.4 | -0.6 | -2.2 | -2.6 | -2.1 | -2.2 |
| A17m0902_hIgG4 | -4.4 | -2.0 | -2.4 | -2.9 | -3.3 | -4.2 | -3.5 | -3.1 |

**Example 12. Assay for T Cell Proliferation Promoting Activity of PD-1-IL-2v**

Unstimulated T cells do not express or lowly express PD-1. T cells stimulated with CD3 antibodies highly express PD-1. To determine whether the PD-1-IL-2v with different IL-2 mutants described above can activate T cells among human peripheral blood mononuclear cells (PBMCs) that lowly express PD-1 and highly express PD-1, T cells were isolated from PBMCs and stimulated with a CD3 antibody. The effects of the fusion proteins described above on the proliferation of unstimulated and stimulated T cells were assessed.

Proliferation experiment of T cells stimulated with a CD3 antibody: After frozen human PBMCs were thawed, T cells were obtained by isolation using a Pan T Cell Isolation Kit (Miltenyi, 130-096-535). The CD3 antibody was diluted with PBS to 0.1 $\mu$g/mL and added to a 10 cm culture dish. The dish was coated at 37 °C for 2 h and washed with PBS for later use. The T cells were plated at $2 \times 10^6$ cells/mL onto the 10 cm culture dish precoated with the CD3 antibody and stimulated at 37 °C with 5% $CO_2$ for 24 h. The stimulated T cells were collected, washed, and counted, and the cell density was adjusted to $5 \times 10^5$ cells/mL. The cells were plated at 200 $\mu$L/well onto a 96-well plate. The test samples were serially diluted with culture medium to prepare 5× working solutions, and 50 $\mu$L of each concentration of the test samples was added to the stimulated T cells and mixed well. The plate was incubated for 4 days. After the drug treatment, the cells were washed twice with FACS buffer (PBS + 2% FBS), and dead cells were labeled with Fixable Viability Dye (eBioscience, 65-0865-14). After flow cytometry antibody staining with CD3 (BD, 565491), CD4 (BD, 558116), CD8 (BD, 565310), Ki-67 (eBioscience, 12-5698-82)), cell proliferation was examined by FACS.

Unstimulated T cell proliferation experiment: The isolated T cells were counted and then plated at $5 \times 10^5$ cells/mL onto a 96-well plate at 200 $\mu$L/well. The test samples were serially diluted with culture medium to prepare 5× working solutions, and 50 $\mu$L of each concentration of the test samples was added to the stimulated T cells and mixed well. The plate was incubated for 4 days. After the drug treatment, the cells were washed twice with FACS buffer, and dead cells were labeled with FVD780. After flow cytometry antibody staining, cell proliferation was examined by FACS.

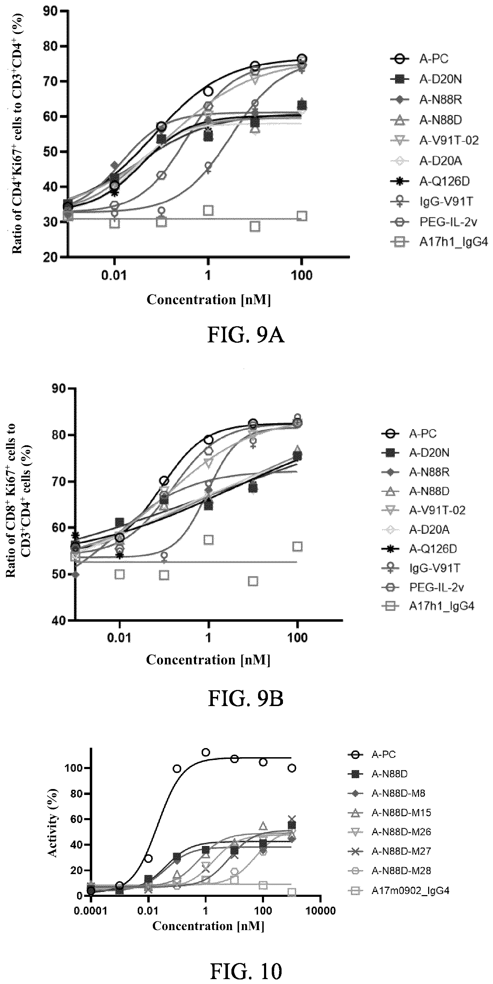

The experimental results are shown in Tables 16 and 17 and FIGs. 8A, 8B, 9A, and 9B. The results show that: 1) In the unstimulated T cells, A-PC exhibited T proliferation activating activity at concentrations above 1 nM (CD8$^+$ T cell proliferation EC$_{50}$ = 21.46 nM, CD4$^+$ T cell proliferation EC$_{50}$ = 8.61 nM); A-V91T-02 showed weaker activity than A-PC, indicating that V91T indeed reduced IL-2 activity but it still can promote the proliferation of the unstimulated T cells; other mutants, including D20A, D20N, N88R, N88D, and Q126D, did not exhibit T cell activation at concentrations up to 100 nM. 2) In the T cells stimulated with the CD3 antibody, the PD-1-IL-2v fusion proteins exhibited significant T cell proliferation promoting activity at a mere relatively low concentration (0.1 nM). PD-1-IL-2v with the other mutations exhibited slightly lower maximum activity on the proliferation of the pan T cells stimulated with the CD3 antibody than A-PC and A-V91T-02. 3) According to a comparison of the activity of the fusion proteins in the proliferation experiments of the T cells unstimulated and stimulated with the CD3 antibody, PD-1-IL-2v with the D20A, D20N, N88R, N88D, or Q126D mutation did not activate the proliferation of the unstimulated T cells but relatively strongly stimulated the proliferation of the T cells stimulated with the CD3 antibody; although A-PC and A-V91T-02 can more strongly stimulate the proliferation of the T cells stimulated with the CD3 antibody, they can also relatively strongly stimulate the proliferation of the T cells not stimulated with the CD3 antibody.

Table 16. The proliferation activity of PD-1-IL-2v with different IL-2 mutations on unstimulated T cells

| Name | CD4$^+$ Ki67$^+$ cells (%) | | | | | |
|---|---|---|---|---|---|---|
| | 100 nM | 10 nM | 1 nM | 0.1 nM | 0.01 nM | 0.001 nM |
| PEG-IL-2v | 45 | 10.2 | 1.77 | 1.38 | 1.51 | 0.29 |
| IgG-V91T | 14.6 | 1.58 | 1.61 | 1.88 | 2.74 | 1.99 |
| A-PC | 46.1 | 14.6 | 2.83 | 1.31 | 3.6 | 1.42 |
| A-V91T-02 | 38.1 | 3.31 | 2.84 | 4.99 | 2.28 | 2.57 |
| A-D20A | 1.37 | 2.7 | 2.66 | 0.94 | 1.47 | 1.91 |
| A-D20N | 3.24 | 1.53 | 3.01 | 2.03 | 2.2 | 1.85 |
| A-N88R | 1.01 | 2.83 | 2.52 | 2.22 | 3.2 | 1.35 |
| A-N88D | 1.84 | 1.71 | 3.13 | 3.48 | 4.72 | 1.42 |
| A-Q126D | 2.97 | 1.78 | 1.23 | 2.74 | 2.64 | 1.76 |
| A17h1_IgG4 | 1.23 | 0.67 | 2.33 | 0.85 | 1.97 | 1.84 |

(continued)

| Name | CD8+ Ki67+ cells (%) | | | | | |
|---|---|---|---|---|---|---|
| | 100 nM | 10 nM | 1 nM | 0.1 nM | 0.01 nM | 0.001 nM |
| PEG-IL-2v | 87.3 | 43 | 1.54 | 0.29 | 0.59 | 0.22 |
| IgG-V91T | 55.9 | 3.46 | 0.63 | 0.99 | 0.83 | 0.59 |
| A-PC | 87.6 | 50.3 | 3.91 | 1.4 | 1.2 | 0 |
| A-V91T-02 | 79.3 | 9.11 | 1.89 | 2.25 | 0.65 | 1.29 |
| A-D20A | 1.34 | 1.23 | 0.75 | 0.33 | 0.24 | 0.71 |
| A-D20N | 1.9 | 0.58 | 0.6 | 0.87 | 0.48 | 0.42 |
| A-N88R | 0.6 | 1.71 | 1.34 | 0.93 | 1.27 | 0.38 |
| A-N88D | 2.21 | 1.12 | 1.76 | 1.5 | 1.44 | 0.83 |
| A-Q126D | 1.47 | 0.88 | 0.34 | 0.77 | 1 | 0.34 |
| A17h1_IgG4 | 0.32 | 0.12 | 0.55 | 0.57 | 0.54 | 0.49 |

Table 17. The proliferation activity of PD-1-IL-2v with different IL-2 mutations on the T cells stimulated with the CD3 antibody

| Name | CD4+ Ki67+ cells (%) | | | | | |
|---|---|---|---|---|---|---|
| | 100 nM | 10 nM | 1 nM | 0.1 nM | 0.01 nM | 0.001 nM |
| PEG-IL-2v | 74.7 | 73 | 63 | 43.3 | 34.8 | 32.9 |
| IgG-V91T | 73.8 | 63 | 45.3 | 32.5 | 31.7 | 32.4 |
| A-PC | 76.4 | 74.4 | 67.2 | 57.2 | 40.4 | 34.3 |
| A-V91T-02 | 75.6 | 70.5 | 60.2 | 56.9 | 41.3 | 33.6 |
| A-D20A | 61.9 | 56.1 | 54.9 | 52.7 | 40.5 | 31.5 |
| A-D20N | 63.2 | 58.2 | 54.3 | 53.6 | 42.5 | 35.2 |
| A-N88R | 63.4 | 61 | 59 | 56.2 | 46.2 | 32.4 |
| A-N88D | 63.9 | 56.8 | 54.9 | 54 | 40.7 | 33.9 |
| A-Q126D | 63.1 | 58.9 | 55.8 | 53.5 | 38.4 | 34.8 |
| A17h1_IgG4 | 31.7 | 28.8 | 33.3 | 30.1 | 29.7 | 31.7 |
| Name | CD8+ Ki67+ cells (%) | | | | | |
| | 100 nM | 10 nM | 1 nM | 0.1 nM | 0.01 nM | 0.001 nM |
| PEG-IL-2v | 82.5 | 82 | 76.6 | 67.1 | 55.5 | 55.5 |
| IgG-V91T | 83.3 | 78.2 | 69 | 53.5 | 54.4 | 54.3 |
| A-PC | 82.6 | 82.5 | 79 | 70.2 | 57.9 | 55.9 |
| A-V91T-02 | 82.4 | 79.9 | 73.9 | 67.9 | 58.1 | 55 |
| A-D20A | 75.9 | 68.2 | 67.7 | 65 | 57.1 | 54.3 |
| A-D20N | 75.6 | 68.5 | 64.7 | 66 | 61.2 | 56.3 |
| A-N88R | 75.6 | 69.4 | 68.2 | 66.6 | 60.9 | 49.9 |
| A-N88D | 76.8 | 69.3 | 66.3 | 64.8 | 57.4 | 55.5 |
| A-Q126D | 75.2 | 69.4 | 65.8 | 65.9 | 54.2 | 58.4 |
| A17h1_IgG4 | 56 | 48.5 | 57.4 | 49.8 | 50 | 53.8 |

**Example 13. Identification of Binding Affinities of PD-1-IL-2v for Antigens PD-1 and IL-2Rα or IL-2Rβ/γ**

The binding affinities of the PD-1-IL-2v fusion proteins with different IL-2 mutants described above for PD-1 and human IL-2Rα or IL-2Rβ/γ were measured using surface plasmon resonance (SPR) technology. The experiment was conducted using a Biacore 8K instrument (GE Healthcare). A CM5 sensor chip was used, and HBS-EP + buffer solution (10 mM HEPES, 150 mM NaCl, 3 mM EDTA, 0.05% surfactant P20) was used as the mobile phase. A 25 μg/mL solution of anti-human IgG (Fc) antibody was prepared in 10 mM sodium acetate buffer (pH 5.0), and the Immobilization program was selected to automatically perform anti-human IgG (Fc) antibody channel amino coupling immobilization. The test PD-1-IL-2v fusion proteins were separately diluted in HBS-EP + buffer solution as ligands and captured using the anti-human IgG (Fc) antibody on the chip channels. Human PD-1 antigen protein (Sino Biological, 10377-H08H), human IL-2Rα receptor protein (Sino Biological, 10165-H08H), or human IL-2Rβ/γ receptor heterodimer protein (Acrosystem, ILG-H5283) were used as analytes and diluted in HBS-EP + buffer solution. The analytes were serially diluted 2-fold from 200 nM to obtain a total of 7 concentration points. The dilutions were allowed to flow through the experimental and reference channels at a flow rate of 30 μL/min, with an association time of 140 s and a dissociation time of 600 s. The regeneration buffer 10 mM Glycine pH 1.5 (GE Healthcare, 29238268-AA) was run at a flow rate of 10 μL/min for 30 s. The association rate Ka, the dissociation rate Kd, and the dissociation constant (i.e., affinity $K_D$ value) were calculated. The results are shown in Tables 18 and 19.

Table 18. The SPR affinities of the fusion proteins of the anti-PD-1 antibody and the IL-2 mutants binding to human PD-1 antigen

| Antibody No. | Ka (1/Ms) | Kd (1/s) | $K_D$(M) |
|---|---|---|---|
| A-PC | 9.37E+04 | 1.82E-03 | 1.94E-08 |
| A-N88D | 9.98E+04 | 1.75E-03 | 1.76E-08 |
| A-D20A | 9.06E+04 | 1.68E-03 | 1.85E-08 |

Table 19. The SPR binding affinities of the fusion proteins of the anti-PD-1 antibody and the IL-2 mutants binding to human IL-2Rα

| Antibody No. | Ka (1/Ms) | Kd (1/s) | $K_D$(M) |
|---|---|---|---|
| A-PC | NB | NB | NB |
| A-N88D | NB | NB | NB |
| A-D20A | NB | NB | NB |
| (NB: not binding) | | | |

Table 20. The SPR binding affinities of the fusion proteins of the anti-PD-1 antibody and the IL-2 mutants binding to human IL-2Rβ/γ

| Antibody No. | Ka (1/Ms) | Kd (1/s) | $K_D$(M) |
|---|---|---|---|
| A-PC | 8.81E+04 | 2.23E-05 | 2.53E-10 |
| A-N88D | 6.37E+04 | 2.15E-04 | 3.38E-09 |
| A-D20A | 4.27E+04 | 6.81E-04 | 1.60E-08 |

The results of binding to human PD-1 antigen show that A-PC, A-N88D, and A-D20A exhibited similar capacities to bind to human PD-1, and their capacities were similar to the capacity of A 17m0902_hIgG4 to bind to human PD-1 (Table 8). The results of binding to human IL-2Rα show that A-PC, A-N88D, and A-D20A all did not bind to human IL-2Rα. The results of binding to human IL-2Rβ/γ show that A-PC exhibited the strongest affinity for human IL-2Rβ/γ, and the affinities of A-N88D and A-D20A for human IL-2Rβ/γ were about 13 times lower and about 63 times lower, respectively.

**Example 14. Construction of Fusion Proteins of Anti-PD-1 Nanobodies with Different PD-1 Binding Affinities and IL-2 Mutant (PD-1-IL-2v) and Affinity Identification**

1. Construction of fusion proteins of anti-PD-1 nanobody mutants and IL-2 mutant (PD-1-IL-2v)

By introducing mutations into anti-PD-1 nanobodies, nanobodies with different affinities for PD-1 can be obtained. By introducing mutations into the anti-PD-1 nanobody AN88D while keeping the IL-2 mutant sequence of A-N88D unchanged, different A-N88D mutants were constructed. The anti-PD-1 nanobody mutations introduced into A-N88D are shown in Table 21. The sequences of the anti-PD-1 nanobodies with introduced mutations are as follows (CDRs are underlined):

> A17m0902M08

EVQLVESGGGLVQPGGSLRLSCAASGLTFS<u>DYSMS</u>WYRQAPGKGRELVA<u>IISGS GVITHY</u>VDSVKGRFTISRDNAKNTLYLQMRSLRAEDTAVYYCRA<u>VSDWQDY</u>W GQGTQVTVSS (SEQ ID NO: 88)

> A17m0902M15

EVQLVESGGGLVQPGGSLRLSCAASGLTFS<u>DYSMS</u>WYRQAPGKGRELVA<u>IISGS GGITHY</u>VDSVKGRFTISRDNAKNTLYLQMRSLRAEDTAVYYCRA<u>VSDWEDY</u>W GQGTQVTVSS (SEQ ID NO: 89)

> A17m0902M26

EVQLVESGGGLVQPGGSLRLSCAASGLTFS<u>SYSMS</u>WYRQAPGKGRELVA<u>IISGSG GITHY</u>VDSVKGRFTISRDNAKNTLYLQMRSLRAEDTAVYYCRA<u>VSDWEDY</u>WG QGTQVTVSS (SEQ ID NO: 90)

> A17m0902M27

EVQLVESGGGLVQPGGSLRLSCAASGLTFS<u>SYSMS</u>WYRQAPGKGRELVA<u>IISGSG GITHY</u>VDSVKGRFTISRDNAKNTLYLQMRSLRAEDTAVYYCRA<u>VSDWQDY</u>WG QGTQVTVSS (SEQ ID NO: 91)

> A17m0902M28

EVQLVESGGGLVQPGGSLRLSCAASGLTFS<u>SYSMS</u>WYRQAPGKGRELVA<u>IISGSG GITHYA</u>DSVKGRFTISRDNAKNTLYLQMRSLRAEDTAVYYCRA<u>VSDWQDY</u>WG QGTQVTVSS (SEQ ID NO: 92)

Table 21. The anti-PD-1 nanobody mutations introduced into A-N88D

| Name | Corresponding PD-1 antibody mutant name | Anti-PD-1 nanobody mutations | Corresponding anti-PD-1 nanobody sequence number |
|---|---|---|---|
| A-N88D | A17m0902 | / | SEQ ID NO: 33 |
| A-N88D-M08 | A17m0902M08 | E103Q | SEQ ID NO: 88 |

(continued)

| Name | Corresponding PD-1 antibody mutant name | Anti-PD-1 nanobody mutations | Corresponding anti-PD-1 nanobody sequence number |
|------|------|------|------|
| A-N88D-M15 | A17m0902M15 | V56G | SEQ ID NO: 89 |
| A-N88D-M26 | A17m0902M26 | D31S, V56G | SEQ ID NO: 90 |
| A-N88D-M27 | A17m0902M27 | D31S, V56G, E103Q | SEQ ID NO: 91 |
| A-N88D-M28 | A17m0902M28 | D31S, V56G, E103Q, V61A | SEQ ID NO: 92 |

The CDR sequences of the anti-PD-1 nanobody mutants are shown in the table below:

Table 22. The CDRs of the anti-PD-1 nanobodies (Kabat numbering scheme)

| No. | CDR sequences | | |
|------|------|------|------|
| A17m0902M08 | CDR1 | DYSMS | SEQ ID NO: 7 |
| | CDR2 | IISGSGVITHYVDSVKG | SEQ ID NO: 38 |
| | CDR3 | VSDWQDY | SEQ ID NO: 96 |
| A17m0902M15 | CDR1 | DYSMS | SEQ ID NO: 7 |
| | CDR2 | IISGSGGITHYVDSVKG | SEQ ID NO: 94 |
| | CDR3 | VSDWEDY | SEQ ID NO: 41 |
| A17m0902M26 | CDR1 | SYSMS | SEQ ID NO: 93 |
| | CDR2 | IISGSGGITHYVDSVKG | SEQ ID NO: 94 |
| | CDR3 | VSDWEDY | SEQ ID NO: 41 |
| A17m0902M27 | CDR1 | SYSMS | SEQ ID NO: 93 |
| | CDR2 | IISGSGGITHYVDSVKG | SEQ ID NO: 94 |
| | CDR3 | VSDWQDY | SEQ ID NO: 96 |
| A17m0902M28 | CDR1 | SYSMS | SEQ ID NO: 93 |
| | CDR2 | IISGSGGITHYADSVKG | SEQ ID NO: 95 |
| | CDR3 | VSDWQDY | SEQ ID NO: 96 |

That is, the A17m09-related antibodies have the following general sequences:

CDR1, $X_6$YSMS, wherein $X_6$ is selected from the group consisting of D and S (SEQ ID NO: 97);
CDR2, IISGSGX$_1$ITHYX$_7$DSVKG, wherein $X_1$ is selected from the group consisting of V and G, and $X_7$ is selected from the group consisting of V and A (SEQ ID NO: 98); and CDR3, VSDWX$_8$DY, wherein $X_8$ is selected from the group consisting of Q and E (SEQ ID NO: 99).

Specifically, the CDR1 may be selected from the group consisting of:

DYSMS (SEQ ID NO: 7)
SYSMS (SEQ ID NO: 93).

The CDR2 may be selected from the group consisting of:

IISGSGVITHYVDSVKG (SEQ ID NO: 38)
IISGSGGITHYVDSVKG (SEQ ID NO: 94)

IISGSGGGITHYADSVKG (SEQ ID NO: 95).

The CDR3 may be selected from the group consisting of:

VSDWEDY (SEQ ID NO: 41)
VSDWQDY (SEQ ID NO: 96).

Further, the A17 of the present disclosure has the following general sequences:

CDR1: $X_6$YSMS (SEQ ID NO: 97)
CDR2: IISGSGX$_1$X$_2$X$_3$HYX$_7$DSVKG, wherein X$_1$ is selected from the group consisting of V and G, X$_2$ is selected from the group consisting of I and S, X$_3$ is selected from the group consisting of T and A, and X$_7$ is selected from the group consisting of V and A (SEQ ID NO: 100)
CDR3: VSDWX$_9$X$_5$Y, wherein X$_9$ is selected from the group consisting of Q, D, and E, and X$_5$ is selected from the group consisting of D and E (SEQ ID NO: 101)

Specifically, the CDR1 may be selected from the group consisting of:

DYSMS (SEQ ID NO: 7)
SYSMS (SEQ ID NO: 93).

The CDR2 may be selected from the group consisting of:

IISGSGVIAHYVDSVKG (SEQ ID NO: 8)
IISGSGVITHYVDSVKG (SEQ ID NO: 38)
IISGSGGIAHYVDSVKG (SEQ ID NO: 39)
IISGSGVSAHYVDSVKG (SEQ ID NO: 40)
IISGSGGITHYVDSVKG (SEQ ID NO: 94)
IISGSGGITHYADSVKG (SEQ ID NO: 95).
the CDR3 may be:

VSDWDDY (SEQ ID NO: 9)
VSDWEDY (SEQ ID NO: 41)
VSDWDEY (SEQ ID NO: 42)
VSDWQDY (SEQ ID NO: 96).

The fusion proteins of the anti-PD-1 nanobody mutants and the IL-2 mutant (PD-1-IL-2v) are A-N88D-M08, A-N88D-M15, A-N88D-M26, A-N88D-M27, and A-N88D-M28. Illustratively, the full-length sequence of A-N88D-M08 is shown, with the Fc underlined and the linker in italics.

> The Knob chain of A-N88D-M08

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWQDYW
GQGTQVTVSSEPKSSDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPCREEMTKNQVSL
WCLVKGFYPSDIAVEWESNGQPENNYKTTPPVLDSDGSFFLYSKLTVDKSRWQQ
GNVFSCSVMHEALHNHYTQKSLSLSPG*GGGGSGGGGSGGGGS*APASSSTKKTQ
LQLEHLLLDLQMILQGISNYKNPKLTRMLTAKFYMPKKATELKHLQCLEEELKP
LEEVLQGAQSKNFHLRPRDLISDINVIVLELKGSETTFMCEYADETATIVEFLNR
WITFAQSIISTLT   (SEQ ID NO: 102)

> The Hole chain of A-N88D-M08

EVQLVESGGGLVQPGGSLRLSCAASGLTFSDYSMSWYRQAPGKGRELVAIISGS
GVITHYVDSVKGRFTISRDNAKNTLYLQMRSLRAEDTAVYYCRAVSDWQDYW

GQGTQVTVSS<u>EPKSSDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSREEMTKNQVSLS
CAVKGFYPSDIAVEWESNGQPENNYKTTPPVLDSDGSFFLVSKLTVDKSRWQQG
NVFSCSVMHEALHNHYTQKSLSLSPG</u>   (SEQ ID NO: 103)

2. Identification of binding affinities of fusion proteins of anti-PD-1 antibody comprising anti-PD-1 nanobody mutants and IL-2 mutant (PD-1-IL-2v) for human PD-1 antigen

The binding affinities of A-N88D with different anti-PD-1 nanobody mutants for PD-1 antigen were measured using SPR. The experimental method was the same as that in Example 13. The results are shown in Table 23.

Table 23. Data on the SPR affinities of PD-1-IL-2v comprising different anti-PD-1 nanobody mutants for PD-1 antigen

| Antibody No. | Ka (1/Ms) | Kd (1/s) | $K_D$(M) |
|---|---|---|---|
| A-N88D-M08 | 2.15E+05 | 2.64E-03 | 1.23E-08 |
| A-N88D-M15 | 2.41E+05 | 7.06E-03 | 2.93E-08 |
| A-N88D-M26 | 1.79E+05 | 9.48E-03 | 5.31E-08 |
| A-N88D-M27 | 1.37E+05 | 1.02E-02 | 7.43E-08 |
| A-N88D-M28 | 1.48E+05 | 1.98E-02 | 1.34E-07 |

The results in Table 23 show that all the mutants can achieve effective binding to human PD-1.

**Example 15. Assay for M07e Cell Proliferation Promoting Activity of Fusion Proteins of Anti-PD-1 Antibodies with Different PD-1 Binding Affinities and IL-2 Mutant (PD-1-IL-2v)**

To measure the activity of PD-1-IL-2v fusion proteins with different PD-1 binding affinities and the dependency on PD-1 binding affinity, the effects of fusion proteins of the PD-1 antibody mutants mentioned in Example 14 and IL-2v on cell proliferation were examined in the M07e-hPD-1 cell strain. See Example 11 for the experimental method and the method of calculating the relative cell proliferation activity of PD-1- IL-2v.

The experimental results are shown in Table 24 and FIG. 10. The results show that AN88D-M08, A-N88D-M15, A-N88D-M26, A-N88D-M27, and A-N88D-M28 all exhibited M07e-hPD-1 cell proliferation promoting activity. The M07e-hPD-1 cell proliferation promoting activity of the PD-1-IL-2v fusion proteins and their PD-1 antigen binding affinities (Table 23) exhibited substantially consistent trends, indicating that the levels of cell proliferation promoting activity of the PD-1-IL-2v fusion proteins depend on the levels of binding affinity of PD-1-IL-2v for PD-1.

Table 24. The proliferation promoting activity of the PD-1-IL-2v fusion proteins with different PD-1 affinities on M07e-hPD-1 cells

| Name | M07e-hPD-1 cell proliferation ratio (%) | | | | | | | |
|---|---|---|---|---|---|---|---|---|
| | 1000 nM | 100 nM | 10 nM | 1 nM | 0.1 nM | 0.01 nM | 0.001 nM | 0.0001 nM |
| A-PC | 100.0 | 104.6 | 107.2 | 112.3 | 99.5 | 29.3 | 8.1 | 4.0 |
| A-N88D | 55.4 | 41.1 | 35.5 | 36.0 | 30.8 | 13.4 | 4.4 | 5.2 |
| A-N88D-M8 | 44.2 | 35.5 | 35.5 | 36.0 | 27.3 | 11.9 | 4.5 | 3.8 |
| A-N88D-M15 | 47.7 | 54.9 | 41.6 | 32.9 | 17.0 | 7.0 | 4.1 | 8.9 |

(continued)

| Name | M07e-hPD-1 cell proliferation ratio (%) | | | | | | | |
|---|---|---|---|---|---|---|---|---|
| | 1000 nM | 100 nM | 10 nM | 1 nM | 0.1 nM | 0.01 nM | 0.001 nM | 0.0001 nM |
| A-N88D-M26 | 54.4 | 43.1 | 37.0 | 23.2 | 10.7 | 9.3 | 4.8 | 3.7 |
| A-N88D-M27 | 60.0 | 39.5 | 31.9 | 21.1 | 9.3 | 4.9 | 4.9 | 7.5 |
| A-N88D-M28 | 51.3 | 34.4 | 19.1 | 12.4 | 7.6 | 9.4 | 6.3 | 7.2 |
| A17m0902_hIg G4 | 2.8 | 8.3 | 12.4 | 12.4 | 10.5 | 7.8 | 5.1 | 6.2 |

## Example 16. A-N88D Can Inhibit Mouse CT26 Tumor Growth

In this example, the *in vivo* anti-tumor efficacy of the A-N88D constructed in Example 10 was verified using a CT26 tumor model of humanized PD-1 Balb/c mice. The CT26 mouse colon cancer cell line was cultured with DMEM culture medium (10% FBS), and $5 \times 10^6$ CT26 cells were subcutaneously inoculated into humanized PD-1 Balb/c female mice (purchased from GemPharmatech). When the mean tumor volume of the mice reached about 90 mm$^3$, the mice were randomized into groups of 6 and administered intraperitoneal injections of antibodies or controls. Tumor volume and body weight were measured twice a week, and data were recorded. The grouping and administration regimen for the experiment are shown in Table 25. Considering that the molecular weight of A-N88D is about 92 kDa and the molecular weight of A17m0902_hIgG4 is about 76 kDa, the dose of A-N88D was adjusted to 120% of that of A17m0902_hIgG4 to achieve equimolar doses.

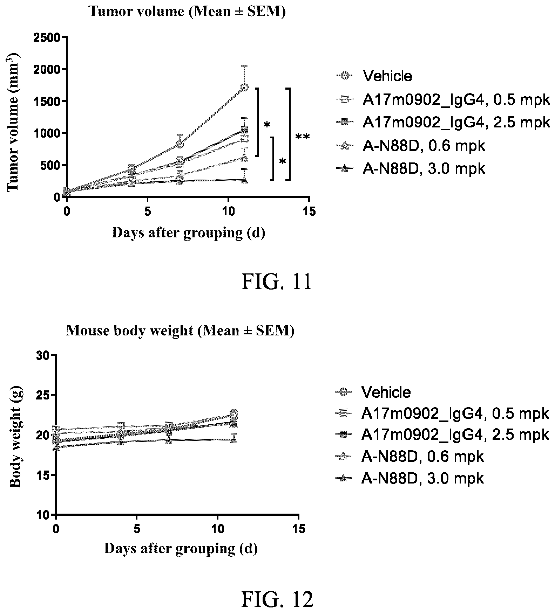

As shown in FIG. 11, compared to the control group, A-N88D exhibited relatively strong anti-tumor efficacy, and at equimolar doses, the *in vivo* anti-tumor efficacy of A-N88D was significantly stronger than that of A17m0902_hIgG4 (in the figure, *P < 0.05, and **P < 0.01). Meanwhile, in terms of mouse toxicity, A-N88D did not cause changes in mouse body weight (FIG. 12), indicating relatively good safety.

Table 25. The grouping and administration regimen for the mouse experiment

| Group | Drug or control | Dose (mg/kg) | Route of administration | Frequency of administration |
|---|---|---|---|---|
| Group 1 | PBS | -- | | |
| Group 2 | A17m0902_hIgG4 | 0.5 | | |
| Group 3 | A17m0902_hIgG4 | 2.5 | i.p. | BIW*3 |
| Group 4 | A-N88D | 0.6 | | |
| Group 5 | A-N88D | 3.0 | | |

## Example 17. A-N88D Can Inhibit Mouse B16F10 Tumor Growth

In this example, the *in vivo* anti-tumor efficacy of A-N88D was verified using a B16F10 tumor model of humanized PD-1 C57BL/6 mice. The B16F10 mouse cutaneous melanoma cancer cell line was cultured with DMEM culture medium (10% FBS), and $1 \times 10^5$ B16F10 cells were subcutaneously inoculated into humanized PD-1 C57BL/6 female mice (purchased from Biocytogen). When the mean tumor volume of the mice reached about 75 mm$^3$, the mice were randomized into groups of 8 and administered intraperitoneal injections of PBS and A-N88D (24 mg/kg). Tumor volume and body weight were measured every two days, and data were recorded. The result shows that the tumor growth inhibition rate of A-N88D at day 10 was 39.9%, which was significantly higher than that of the PBS control group (p, 0.0007). A-N88D exhibited relatively strong anti-tumor efficacy. Meanwhile, A-N88D did not cause changes in mouse body weight, indicating relatively good safety.

## Claims

1. An immunoconjugate, comprising a PD-1 antibody or an antigen-binding fragment thereof, and an IL-2 variant, wherein the IL-2 variant comprises a mutation represented by the following mutation (i), and any one or any combination of the mutations (ii)-(iii):

(i) a mutation that makes the IL-2 variant have a reduced affinity for IL-2Rβ/γ as compared to wild-type IL-2;

(ii) a mutation that makes the IL-2 variant have a reduced affinity for IL-2Rα as compared to wild-type IL-2 or do not bind to IL-2Rα; and
(iii) a mutation that makes the IL-2 variant have increased stability, expression, and/or purity;

preferably,

(i) a mutation that makes the IL-2 variant have a reduced affinity for IL-2Rβ/γ as compared to wild-type IL-2, selected from the group consisting of any one or any combination of positions 12, 15, 16, 18, 19, 20, 23, 43, 69, 84, 87, 88, 91, 92, 95, 123, 126, 127, 129, and 130;
(ii) a mutation that makes the IL-2 variant have a reduced affinity for IL-2Rα as compared to wild-type IL-2 or do not bind to IL-2Rα, selected from the group consisting of any one or any combination of positions 38, 42, 45, 62, 65, 68, and 72; and
(iii) a mutation that makes the IL-2 variant have increased stability, expression, and/or purity as compared to wild-type IL-2, selected from the group consisting of any one or any combination of positions 11, 26, 27, 29, 30, 70, 71, 78, 82, and 132;

the positions described above are numbered according to a wild-type IL-2 amino acid sequence set forth in SEQ ID NO: 87.

2. The immunoconjugate according to claim 1, wherein:

the mutation (i) comprises any one or any combination of the following mutations: L12R, L12K, L12E, L12Q, E15Q, E15R, E15A, E15S, H16N, H16A, H16E, H16D, H16G, H16S, H16T, H16V, H16P, D20A, D20H, D20Y, D20N, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88T, N88R, N88I, V91A, V91T, V91E, I92A, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, and S127Q; and/or,
the mutation (ii) comprises any one or any combination of the following mutations: R38A, R38D, R38E, E62Q, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, P65R, P65E, P65K, P65H, P65Y, P65Q, P65D, P65N, E68A, E68Q, E68K, E68R, L72G, L72A, L72S, L72T, L72Q, L72E, L72N, L72D, L72R, and L72K; and/or,
the mutation (iii) comprises at least one of the following groups of mutations: N26Q, N29S, N30S, N71Q, Q11C/L132C, L70C/P82C, and G27C/F78C;
preferably, the mutation (i) comprises at least one of the following groups of mutations:

N88D, N88R, D20A, D20N, V91T, Q126D, and H16A/D84S; and/or,
the mutation (ii) comprises at least one of the following groups of mutations: F42A, Y45A, L72G, R38E/F42A, F42A/L72G, F42A/Y45A, Y45A/L72G, and F42A/Y45A/L72G; and/or,
the mutation (iii) comprises at least one of the following groups of mutations: N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

3. The immunoconjugate according to claim 1 or 2, wherein the IL-2 variant further comprises a mutation at position 3 and/or a mutation at position 125; the mutation at position 3 is preferably T3A; the mutation at position 125 is preferably C125A or C125S.

4. The immunoconjugate according to any one of claims 1-3, wherein the IL-2 variant comprises any one of the following groups of mutations:

F42A/L72G/N88D,
F42A/L72G/N88R,
F42A/L72G/D20A,
F42A/L72G/D20N,
F42A/L72G/V91T,
F42A/R3 8E/V91T,
F42A/L72G/Q126D, and
F42A/L72G/H16A/D84S;
preferably, the IL-2 variant comprises any one of the following groups of mutations:

F42A/L72G/N88D/N26Q/N29S/N71Q,
F42A/L72G/N88R/N26Q/N29S/N71Q,
F42A/L72G/D20A/N26Q/N29S/N71Q,
F42A/L72G/D20N/N26Q/N29S/N71Q,
F42A/L72G/V91T/N26Q/N29S/N71Q,
F42A/L72G/Q126D/N26Q/N29S/N71Q, and
F42A/L72G/H16A/D84S/N26Q/N29S/N71Q;
more preferably, the IL-2 variant comprises any one of the following groups of mutations:

T3 A/F42 A/R3 8E/V91T/C125A,
T3A/F42A/L72G/N88D/N26Q/N29S/N71 Q/C 125A,
T3A/F42A/L72G/N88R/N26Q/N29S/N71Q/C125A,
T3A/F42A/L72G/D20A/N26Q/N29S/N71Q/C125A,
T3 A/F42A/L72G/D20N/N26Q/N29S/N71Q/C125A,
T3A/F42A/L72G/V91T/N26Q/N29S/N71Q/C125A,
T3A/F42A/L72G/Q126D/N26Q/N29S/N71Q/C125A, and
T3A/F42A/L72G/H16A/D84S/N26Q/N29S/N71Q/C125A.

5. The immunoconjugate according to any one of claims 1-4, wherein the IL-2 variant comprises the amino acid sequence set forth in any one of SEQ ID NOs: 60-68 or a sequence having at least 90% identity thereto.

6. The immunoconjugate according to any one of claims 1-5, wherein the PD-1 antibody or the antigen-binding fragment thereof comprises at least one immunoglobulin single variable domain, and the immunoglobulin single variable domain comprises

1) a CDR1, a CDR2, and a CDR3 set forth in SEQ ID NOs: 97, 100, and 101, or
2) a CDR1, a CDR2, and a CDR3 set forth in SEQ ID NOs: 4, 5, and 6;
preferably, the immunoglobulin single variable domain comprises

1-1) a CDR1, a CDR2, and a CDR3 set forth in SEQ ID NOs: 7, 36, and 37,
1-2) a CDR1, a CDR2, and a CDR3 set forth in SEQ ID NOs: 97, 98, and 99, or
1-3) a CDR1 set forth in SEQ ID NO: 7 or 93, a CDR2 set forth in any one of SEQ ID NOs: 8, 38-40, and 94-95, and a CDR3 set forth in any one of SEQ ID NOs: 9, 41, 42, and 96;
preferably, the immunoglobulin single variable domain comprises the CDR1, CDR2, and CDR3 set forth in any one of 1) to 19):

1) a CDR1, a CDR2, and a CDR3 set forth in SEQ ID NOs: 7, 38, and 41;
2) a CDR1, a CDR2, and a CDR3 set forth in SEQ ID NOs: 7, 38, and 96;
3) a CDR1, a CDR2, and a CDR3 set forth in SEQ ID NOs: 7, 94, and 41;
4) a CDR1, a CDR2, and a CDR3 set forth in SEQ ID NOs: 93, 94, and 41;
5) a CDR1, a CDR2, and a CDR3 set forth in SEQ ID NOs: 93, 94, and 96;
6) a CDR1, a CDR2, and a CDR3 set forth in SEQ ID NOs: 93, 95, and 96;
7) a CDR1, a CDR2, and a CDR3 set forth in SEQ ID NOs: 7, 8, and 9;
8) a CDR1, a CDR2, and a CDR3 set forth in SEQ ID NOs: 7, 8, and 42;
9) a CDR1, a CDR2, and a CDR3 set forth in SEQ ID NOs: 7, 38, and 6;
10) a CDR1, a CDR2, and a CDR3 set forth in SEQ ID NOs: 7, 39, and 9;
11) a CDR1, a CDR2, and a CDR3 set forth in SEQ ID NOs: 7, 40, and 6;
12) a CDR1, a CDR2, and a CDR3 set forth in SEQ ID NOs: 7, 8, and 41;
13) a CDR1, a CDR2, and a CDR3 set forth in SEQ ID NOs: 7, 8, and 42;
14) a CDR1, a CDR2, and a CDR3 set forth in SEQ ID NOs: 7, 38, and 41;
15) a CDR1, a CDR2, and a CDR3 set forth in SEQ ID NOs: 7, 38, and 42;
16) a CDR1, a CDR2, and a CDR3 set forth in SEQ ID NOs: 7, 39, and 41;
17) a CDR1, a CDR2, and a CDR3 set forth in SEQ ID NOs: 7, 39, and 42;
18) a CDR1, a CDR2, and a CDR3 set forth in SEQ ID NOs: 7, 40, and 41; and
19) a CDR1, a CDR2, and a CDR3 set forth in SEQ ID NOs: 7, 40, and 42;

preferably, the immunoglobulin single variable domain comprises or consists of the following sequence: the amino acid sequence set forth in any one of SEQ ID NOs: 3, 13-15, 17-35, and 88-92; the amino acid sequence set forth in any one of SEQ ID NOs: 2 and 16; or an amino acid sequence having at least 90% sequence identity to any one of the aforementioned sequences.

7. The immunoconjugate according to any one of claims 1-6, wherein the PD-1 antibody or the antigen-binding fragment thereof comprises a PD-1-binding domain 1 and/or a PD-1-binding domain 2; the PD-1-binding domain 1 comprises at least one said immunoglobulin single variable domain, and the PD-1-binding domain 2 comprises a heavy chain variable region (VH) and a light chain variable region (VL);

preferably, the PD-1-binding domain 2 is selected from the group consisting of a Fab, a Fab', an Fv, and an ScFv; more preferably, the PD-1-binding domain 2 is a Fab; preferably, the VH comprises a HCDR1, a HCDR2, and a HCDR3 of the amino acid sequences set forth in SEQ ID NOs: 54-56, and the VL comprises a LCDR1, a LCDR2, and a LCDR3 of the amino acid sequences set forth in SEQ ID NOs: 57-59; more preferably, the VH comprises a heavy chain variable region from a Fab heavy chain of the amino acid sequence set forth in SEQ ID NO: 52, and the VL comprises a light chain variable region from a Fab light chain of the amino acid sequence set forth in SEQ ID NO: 53.

8. An immunoconjugate, comprising a PD-1 antibody or an antigen-binding fragment thereof, and an IL-2 or a variant thereof; wherein:

the PD-1 antibody or the antigen-binding fragment thereof comprises at least one immunoglobulin single variable domain, and the immunoglobulin single variable domain comprises

1) a CDR1, a CDR2, and a CDR3 set forth in SEQ ID NOs: 97, 100, and 101, or
2) a CDR1, a CDR2, and a CDR3 set forth in SEQ ID NOs: 4, 5, and 6;

preferably, the immunoglobulin single variable domain comprises

1-1) a CDR1, a CDR2, and a CDR3 set forth in SEQ ID NOs: 7, 36, and 37,
1-2) a CDR1, a CDR2, and a CDR3 set forth in SEQ ID NOs: 97, 98, and 99, or
1-3) a CDR1 set forth in SEQ ID NO: 7 or 93, a CDR2 set forth in any one of SEQ ID NOs: 8, 38-40, and 94-95, and a CDR3 set forth in any one of SEQ ID NOs: 9, 41, 42, and 96.

9. The immunoconjugate according to claim 8, wherein the immunoglobulin single variable domain comprises the CDR1, CDR2, and CDR3 set forth in any one of 1) to 19):

1) a CDR1, a CDR2, and a CDR3 set forth in SEQ ID NOs: 7, 38, and 41;
2) a CDR1, a CDR2, and a CDR3 set forth in SEQ ID NOs: 7, 38, and 96;
3) a CDR1, a CDR2, and a CDR3 set forth in SEQ ID NOs: 7, 94, and 41;
4) a CDR1, a CDR2, and a CDR3 set forth in SEQ ID NOs: 93, 94, and 41;
5) a CDR1, a CDR2, and a CDR3 set forth in SEQ ID NOs: 93, 94, and 96;
6) a CDR1, a CDR2, and a CDR3 set forth in SEQ ID NOs: 93, 95, and 96;
7) a CDR1, a CDR2, and a CDR3 set forth in SEQ ID NOs: 7, 8, and 9;
8) a CDR1, a CDR2, and a CDR3 set forth in SEQ ID NOs: 7, 8, and 42;
9) a CDR1, a CDR2, and a CDR3 set forth in SEQ ID NOs: 7, 38, and 6;
10) a CDR1, a CDR2, and a CDR3 set forth in SEQ ID NOs: 7, 39, and 9;
11) a CDR1, a CDR2, and a CDR3 set forth in SEQ ID NOs: 7, 40, and 6;
12) a CDR1, a CDR2, and a CDR3 set forth in SEQ ID NOs: 7, 8, and 41;
13) a CDR1, a CDR2, and a CDR3 set forth in SEQ ID NOs: 7, 8, and 42;
14) a CDR1, a CDR2, and a CDR3 set forth in SEQ ID NOs: 7, 38, and 41;
15) a CDR1, a CDR2, and a CDR3 set forth in SEQ ID NOs: 7, 38, and 42;
16) a CDR1, a CDR2, and a CDR3 set forth in SEQ ID NOs: 7, 39, and 41;
17) a CDR1, a CDR2, and a CDR3 set forth in SEQ ID NOs: 7, 39, and 42;
18) a CDR1, a CDR2, and a CDR3 set forth in SEQ ID NOs: 7, 40, and 41; and
19) a CDR1, a CDR2, and a CDR3 set forth in SEQ ID NOs: 7, 40, and 42.

10. The immunoconjugate according to claim 8 or 9, wherein the immunoglobulin single variable domain is modified by humanization, affinity maturation, T cell epitope removal, reduction of antibody deamidation, and/or reduction of antibody isomerization; preferably, a heavy chain framework region of a human germline template used in the humanization modification is IGHV3-23 *01 or IGHV3-23*04.

11. The immunoconjugate according to any one of claims 8-10, wherein the immunoglobulin single variable domain comprises or consists of the following sequence:

the amino acid sequence set forth in any one of SEQ ID NOs: 3, 13-15, 17-35, and 88-92;
the amino acid sequence set forth in any one of SEQ ID NOs: 2 and 16; or
an amino acid sequence having at least 90% sequence identity to any one of the aforementioned sequences.

12. The immunoconjugate according to any one of claims 8-11, wherein the PD-1 antibody or the antigen-binding fragment thereof comprises a PD-1-binding domain 1 and/or a PD-1-binding domain 2; the PD-1-binding domain 1 comprises at least one said immunoglobulin single variable domain, and the PD-1-binding domain 2 comprises a heavy chain variable region (VH) and a light chain variable region (VL);

preferably, the PD-1-binding domain 2 is selected from the group consisting of a Fab, a Fab', an Fv, and an ScFv; more preferably, the PD-1-binding domain 2 is a Fab;
preferably, the VH comprises a HCDR1, a HCDR2, and a HCDR3 of the amino acid sequences set forth in SEQ ID NOs: 54-56, and the VL comprises a LCDR1, a LCDR2, and a LCDR3 of the amino acid sequences set forth in SEQ ID NOs: 57-59; more preferably, the VH comprises a heavy chain variable region from a Fab heavy chain of the amino acid sequence set forth in SEQ ID NO: 52, and the VL comprises a light chain variable region from a Fab light chain of the amino acid sequence set forth in SEQ ID NO: 53.

13. The immunoconjugate according to any one of claims 8-12, wherein the IL-2 variant comprises any one or any combination of the following mutations (i)-(iii):

(i) a mutation that makes the IL-2 variant have a reduced affinity for IL-2Rβ/γ as compared to wild-type IL-2;
(ii) a mutation that makes the IL-2 variant have a reduced affinity for IL-2Rα as compared to wild-type IL-2 or do not bind to IL-2Rα; and
(iii) a mutation that makes the IL-2 variant have increased stability, expression, and/or purity;

preferably,

(i) the mutation that makes the IL-2 variant have a reduced affinity for IL-2Rβ/γ as compared to wild-type IL-2 is selected from the group consisting of any one or any combination of positions 12, 15, 16, 18, 19, 20, 23, 43, 69, 84, 87, 88, 91, 92, 95, 123, 126, 127, 129, and 130;
(ii) the mutation that makes the IL-2 variant have a reduced affinity for IL-2Rα as compared to wild-type IL-2 or do not bind to IL-2Rα is selected from the group consisting of any one or any combination of positions 38, 42, 45, 62, 65, 68, and 72;
(iii) the mutation that makes the IL-2 variant have increased stability, expression, and/or purity as compared to wild-type IL-2 is selected from the group consisting of any one or any combination of positions 11, 26, 27, 29, 30, 70, 71, 78, 82, and 132;

the positions described above are numbered according to a wild-type IL-2 amino acid sequence set forth in SEQ ID NO: 87.

14. The immunoconjugate according to claim 13, wherein the mutation (i) comprises any one or any combination of the following mutations: L12R, L12K, L12E, L12Q, E15Q, E15R, E15A, E15S, H16N, H16A, H16E, H16D, H16G, H16S, H16T, H16V, H16P, D20A, D20H, D20Y, D20N, M23A, M23R, M23K, M23G, M23S, M23T, M23V, M23P, S87K, S87A, D84S, D84L, D84N, D84V, D84H, D84Y, D84R, D84K, D84G, D84A, D84T, D84P, N88D, N88A, N88S, N88T, N88R, N88I, V91A, V91T, V91E, I92A, E95S, E95A, E95R, E95Q, E95G, E95T, E95V, E95P, E95H, E95N, T123A, T123E, T123K, T123Q, Q126D, Q126L, Q126A, Q126S, Q126T, Q126E, S127A, S127E, S127K, and S127Q;
preferably, the mutation (i) comprises at least one of the following groups of mutations:
N88D, N88R, D20A, D20N, V91T, Q126D, and H16A/D84S.

15. The immunoconjugate according to claim 13 or 14, wherein the mutation (ii) comprises any one or any combination of the following mutations: R38A, R38D, R38E, E62Q, F42A, F42G, F42S, F42T, F42Q, F42E, F42N, F42D, F42R, F42K, Y45A, Y45G, Y45S, Y45T, Y45Q, Y45E, Y45N, Y45D, Y45R, Y45K, P65R, P65E, P65K, P65H, P65Y, P65Q, P65D, P65N, E68A, E68Q, E68K, E68R, L72G, L72A, L72S, L72T, L72Q, L72E, L72N, L72D, L72R, and L72K;

preferably, the mutation (ii) comprises at least one of the following groups of mutations: F42A, Y45A, L72G, R38E/F42A, F42A/L72G, F42A/Y45A, Y45A/L72G, and F42A/Y45A/L72G.

16. The immunoconjugate according to any one of claims 13 to 15, wherein the mutation (iii) comprises the following mutations: N26Q, N29S, N30S, N71Q, Q11C/L132C, L70C/P82C, and G27C/F78C;
   preferably, the mutation (iii) comprises at least one of the following groups of mutations: N26Q, N29S, N30S, N26Q/N29S/N71Q, N26Q/N29S, N26Q/N30S, N26Q/N30S/Q11C/L132C, N26Q/N29S/L70C/P82C, and N26Q/N30S/G27C/F78C.

17. The immunoconjugate according to any one of claims 13 to 16, wherein the IL-2 variant comprises any one of the following groups of mutations:

   F42A/L72G/N88D,
   F42A/L72G/N88R,
   F42A/L72G/D20A,
   F42A/L72G/D20N,
   F42A/L72G/V91T,
   F42A/R3 8E/V91T,
   F42A/L72G/Q126D, and
   F42A/L72G/H16A/D84S;
   preferably, the IL-2 variant comprises any one of the following groups of mutations:

   F42A/L72G/N88D/N26Q/N29S/N71Q,
   F42A/L72G/N88R/N26Q/N29S/N71Q,
   F42A/L72G/D20A/N26Q/N29S/N71Q,
   F42A/L72G/D20N/N26Q/N29S/N71Q,
   F42A/L72G/V91T/N26Q/N29S/N71Q,
   F42A/L72G/Q126D/N26Q/N29S/N71Q, and
   F42A/L72G/H16A/D84S/N26Q/N29S/N71Q.

18. The immunoconjugate according to any one of claims 13 to 17, wherein the IL-2 variant further comprises a mutation at position 3 and/or a mutation at position 125; the mutation at position 3 is preferably T3A; the mutation at position 125 is preferably C125A or C125S.

19. The immunoconjugate according to any one of claims 13 to 18, wherein the IL-2 variant comprises the amino acid sequence set forth in any one of SEQ ID NOs: 60-68 or a sequence having at least 90% identity thereto.

20. The immunoconjugate according to any one of the preceding claims, wherein the immunoconjugate also comprises an immunoglobulin Fc region;
   preferably, the Fc region is an Fc region of human IgG1 or IgG4.

21. The immunoconjugate according to claim 20, wherein the Fc region of human IgG1 comprises one or more amino acid mutations that reduce binding to Fc receptors (FcRs) and/or reduce effector functions;

   preferably, the Fc region of human IgG1 comprises one or more amino acid mutations that reduce binding to Fcγ receptors (FcγRs) and/or reduce ADCC effects;
   more preferably, the Fc region of human IgG1 comprises any one or any combination of the L234A, L235A, and P329G mutations.

22. The immunoconjugate according to claim 20 or 21, wherein the Fc region comprises a first subunit and a second subunit, and the Fc region comprises a knob-into-hole modification that promotes the association of the first subunit and the second subunit; preferably, the first subunit of the Fc region comprises a knob modification, and the second subunit of the Fc region comprises a hole modification.

23. The immunoconjugate according to claim 22, wherein,

   the first subunit of the Fc region comprises a T366 mutation, and the second subunit comprises a mutation selected from the group consisting of T366, L368, and Y407 or any combination thereof;

the first subunit of the Fc region comprises an S354 or E356 mutation, and the second subunit comprises a Y349 mutation; or

the first subunit of the Fc region comprises an S354 mutation and a T366 mutation, and the second subunit comprises a Y349 mutation, a T366 mutation, an L368 mutation, and a Y407 mutation;

preferably,

the first subunit of the Fc region comprises the T366W mutation, and the second subunit comprises a mutation selected from the group consisting of T366S, L368A, and Y407V or any combination thereof;

the first subunit of the Fc region comprises the S354C or E356C mutation, and the second subunit comprises the Y349C mutation; or

the first subunit of the Fc region comprises the S354C/T366W mutations, and the second subunit comprises the Y349C/T366S/L368A/Y407V mutations.

24. The immunoconjugate according to claim 22 or 23, wherein the second subunit of the Fc region also comprises an H435 mutation and/or a Y436 mutation, preferably the H435R/Y436F, H435R, or H435K mutation(s).

25. The immunoconjugate according to any one of claims 22-24, wherein the first subunit and/or the second subunit of the Fc region also comprise(s) a C220 mutation, preferably the C220S mutation.

26. The immunoconjugate according to any one of claims 20 to 25, wherein the Fc region of human IgG1 comprises a mutation that enhances FcRn-mediated recycling and/or increases half-life;

preferably, the Fc region of human IgG1 comprises a mutation selected from the group consisting of M252, S254, T256, and M428 or any combination thereof;

preferably, the Fc region of human IgG1 comprises the M252Y/M428V, M252Y/M428L, or M252Y/S254T/T256E mutations.

27. The immunoconjugate according to any one of the preceding claims, wherein the PD-1 antibody or the antigen-binding fragment thereof is located at the N-terminus of the Fc region, and the IL-2 or the variant thereof is located at the N-terminus or C-terminus of the Fc region;

preferably, the PD-1 antibody or the antigen-binding fragment thereof is located at the N-terminus of the Fc, and the IL-2 or the variant thereof is located at the C-terminus of the Fc region.

28. The immunoconjugate according to any one of claims 22 to 27, wherein the IL-2 or the variant thereof is located in a polypeptide chain where the first subunit of the Fc region is present; preferably, the IL-2 or the variant thereof is linked to the first subunit of the Fc region directly or by a linker.

29. The immunoconjugate according to any one of the preceding claims, wherein the valence ratio of the PD-1-binding domains to the IL-2 or the variant thereof in the PD-1 antibody or the antigen-binding fragment thereof is between 4:1 and 1:2, preferably 4:1, 3:1,2:1, or 1:1.

30. The immunoconjugate according to any one of the preceding claims, wherein the immunoconjugate also comprises a linker;

preferably, the linker is a $(G_xS)_y$ linker, wherein x is selected from the group consisting of integers of 1-5, and y is selected from the group consisting of integers of 1-6;

more preferably, x is 4, and y is 1, 2, or 3.

31. The immunoconjugate according to any one of the preceding claims, comprising, from N-terminus to C-terminus, a polypeptide chain combination set forth in any one of the following I)-VI):

I) a first polypeptide chain: [PD-1-binding domain 1]-[the first subunit of the Fc region]-[linker 1]-[IL-2 or the variant thereof], and

a second polypeptide chain: [PD-1-binding domain 1]-[the second subunit of the Fc region];

II) a first polypeptide chain: [PD-1-binding domain 1]-[linker 2]-[PD-1-binding domain 1]-[the first subunit of the Fc region]-[linker 1]-[IL-2 or the variant thereof], and

a second polypeptide chain: [PD-1-binding domain 1]-[linker 2]-[PD-1-binding domain 1]-[the second subunit of the Fc region];

III) a first polypeptide chain: [the Fab heavy chain of PD-1-binding domain 2]-[the first subunit of the Fc region]-[linker 1]-[IL-2 or the variant thereof],

a second polypeptide chain: [PD-1-binding domain 1]-[the second subunit of the Fc region], and
a third polypeptide chain: [the Fab light chain of PD-1-binding domain 2];

IV) a first polypeptide chain: [the Fab heavy chain of PD-1-binding domain 2]-[the first subunit of the Fc region]-[linker 1]-[IL-2 or the variant thereof],

a second polypeptide chain: [PD-1-binding domain 1]-[linker 2]-[PD-1-binding domain 1]-[the second subunit of the Fc region], and
a third polypeptide chain: [the Fab light chain of PD-1-binding domain 2];

V) a first polypeptide chain: [PD-1-binding domain 1]-[linker 2]-[the Fab heavy chain of PD-1-binding domain 2]-[the first subunit of the Fc region]-[linker 1]-[IL-2 or the variant thereof],

a second polypeptide chain: [PD-1-binding domain 1]-[linker 2]-[the Fab light chain of PD-1-binding domain 2]-[the second subunit of the Fc region], and
a third polypeptide chain: [the Fab light chain of PD-1-binding domain 2]; and

VI) a first polypeptide chain: [IL-2 or the variant thereof]-[linker 3]-[the first subunit of the Fc region], and

a second polypeptide chain: [PD-1-binding domain 1]-[linker 2]-[PD-1-binding domain 1]-[the second subunit of the Fc region];
wherein - represents a peptide bond; the linkers are polypeptides capable of fulfilling the function of linking, and any two of linker 1, linker 2, and linker 3 may be identical or different; preferably, the linkers are $(G_xS)_y$ linkers, wherein x is selected from the group consisting of integers of 1-5, and y is selected from the group consisting of integers of 1-6; more preferably, linker 1 is $(G_4S)_3$, linker 2 is $(G_4S)_2$, and linker 3 is $G_4S$; any two [PD-1-binding domain 1]s may be identical or different and are selected from the group consisting of any of the PD-1-binding domains according to claims 1 to 3;
preferably, [PD-1-binding domain 2] comprises a heavy chain variable region (VH) and a light chain variable region (VL), wherein the VH comprises a HCDR1, a HCDR2, and a HCDR3 of the amino acid sequences set forth in SEQ ID NOs: 54-56, and the VL comprises a LCDR1, a LCDR2, and a LCDR3 of the amino acid sequences set forth in SEQ ID NOs: 57-59; preferably, the VH comprises a heavy chain variable region from a Fab heavy chain of the amino acid sequence set forth in SEQ ID NO: 52, and the VL comprises a light chain variable region from a Fab light chain of the amino acid sequence set forth in SEQ ID NO: 53.

32. The immunoconjugate according to any one of the preceding claims, comprising a polypeptide chain combination set forth in the following amino acid sequences:

SEQ ID NOs: 73 and 74;
SEQ ID NOs: 75 and 76;
SEQ ID NOs: 77 and 78;
SEQ ID NOs: 79 and 80;
SEQ ID NOs: 43 and 44;
SEQ ID NOs: 45 and 46;
SEQ ID NOs: 47, 44, and 48;
SEQ ID NOs: 47, 46, and 48;
SEQ ID NOs: 49, 50, and 48; or
SEQ ID NOs: 51 and 46;
SEQ ID NOs: 102 and 103.

33. A nucleic acid molecule, encoding the immunoconjugate according to any one of claims 1 to 32.

34. A host cell, comprising the nucleic acid molecule according to claim 33.

35. A method for preparing the immunoconjugate according to any one of claims 1 to 32, comprising culturing the host cell according to claim 34 and expressing the immunoconjugate, and optionally comprising isolating or purifying the immunoconjugate.

36. A pharmaceutical composition, comprising the immunoconjugate according to any one of claims 1-32, and one or more pharmaceutically acceptable carriers, diluents, or excipients.

37. Use of the immunoconjugate according to any one of claims 1 to 32, the nucleic acid molecule according to claim 33, or the pharmaceutical composition according to claim 36 in at least one of the following (1)-(5):

> (1) treating a cancer, or preparing a medicament for treating a cancer;
> (2) activating cytotoxic T cells (CTLs), or preparing a medicament for activating cytotoxic T cells (CTLs);
> (3) promoting the proliferation of $CD4^+$ and/or $CD8^+$ T cells, or preparing a medicament for promoting the proliferation of $CD4^+$ and/or $CD8^+$ T cells;
> (4) making cytotoxic T cells (CTLs) with high PD-1 antibody expression have higher cell activation efficiency than cytotoxic T cells (CTLs) with low or no PD-1 antibody expression;
> (5) making $CD4^+$ and/or $CD8^+$ T cells with high PD-1 antibody expression have higher cell proliferation efficiency than $CD4^+$ and/or $CD8^+$ T cells with low or no PD-1 antibody expression;

> preferably, the cancer is selected from the group consisting of lung cancer, prostate cancer, breast cancer, head and neck cancer, esophageal cancer, stomach/gastric cancer, colon cancer, colorectal cancer, bladder cancer, cervical cancer, uterine cancer, ovarian cancer, liver cancer, melanoma, renal cancer, squamous cell cancer, and hematological cancer.

38. A method for treating or ameliorating a disease or disorder, wherein the method comprises administering to a subject in need thereof the immunoconjugate according to any one of claims 1 to 32, or the nucleic acid molecule according to claim 33, or the pharmaceutical composition according to claim 36;

> preferably, the disease is a cancer;
> more preferably, the cancer is selected from the group consisting of lung cancer, prostate cancer, breast cancer, head and neck cancer, esophageal cancer, stomach/gastric cancer, colon cancer, colorectal cancer, bladder cancer, cervical cancer, uterine cancer, ovarian cancer, liver cancer, melanoma, renal cancer, squamous cell cancer, and hematological cancer.

39. A method for activating cytotoxic T cells (CTLs), wherein the method comprises administering to a subject in need thereof the immunoconjugate according to any one of claims 1 to 32, or the nucleic acid molecule according to claim 33, or the pharmaceutical composition according to claim 36;

> preferably, the subject has a cancer;
> more preferably, the cancer is selected from the group consisting of lung cancer, prostate cancer, breast cancer, head and neck cancer, esophageal cancer, stomach/gastric cancer, colon cancer, colorectal cancer, bladder cancer, cervical cancer, uterine cancer, ovarian cancer, liver cancer, melanoma, renal cancer, squamous cell cancer, and hematological cancer;
> preferably, the cytotoxic T cells (CTLs) specifically express PD-1.

40. A method for promoting the proliferation of $CD4^+$ and/or $CD8^+$ T cells, wherein the method comprises administering to a subject in need thereof the immunoconjugate according to any one of claims 1 to 32, or the nucleic acid molecule according to claim 33, or the pharmaceutical composition according to claim 36;

> preferably, the subject has a cancer;
> more preferably, the cancer is selected from the group consisting of lung cancer, prostate cancer, breast cancer, head and neck cancer, esophageal cancer, stomach/gastric cancer, colon cancer, colorectal cancer, bladder cancer, cervical cancer, uterine cancer, ovarian cancer, liver cancer, melanoma, renal cancer, squamous cell cancer, and hematological cancer;
> preferably, the $CD4^+$ and/or $CD8^+$ T cells specifically express PD-1.

FIG. 1A

FIG. 1B

FIG. 2

FIG. 3A

FIG. 3B

Antibody C domain    A17m0902    Pembrolizumab-VH/VL    IL2

FIG. 4

FIG. 5A

FIG. 5B

FIG. 6A

FIG. 6B

FIG. 7

FIG. 8A

FIG. 8B

FIG. 9A

FIG. 9B

FIG. 10

**Tumor volume (Mean ± SEM)**

FIG. 11

**Mouse body weight (Mean ± SEM)**

FIG. 12

## INTERNATIONAL SEARCH REPORT

| International application No. |
|---|
| **PCT/CN2023/075448** |

**A. CLASSIFICATION OF SUBJECT MATTER**

C07K19/00(2006.01)i;C07K16/28(2006.01)i;C07K14/55(2006.01)i;C12N15/62(2006.01)i;C12N15/63(2006.01)i;A61P35/00(2006.01)i;A61K38/20(2006.01)i;A61K39/00(2006.01)i

According to International Patent Classification (IPC) or to both national classification and IPC

**B. FIELDS SEARCHED**

Minimum documentation searched (classification system followed by classification symbols)

IPC:C07K C12N A61P A61K

Documentation searched other than minimum documentation to the extent that such documents are included in the fields searched

Electronic data base consulted during the international search (name of data base and, where practicable, search terms used)

CNTXT, DWPI, VEN, ENTXTC, CNKI, 万方数据资源系统, WANFANG DATA, PubMed, ISI web of knowledge and search terms: 恒瑞, 白介素-2, IL-2, TCGF , CD25, CD122, CD132, pd-1, Fc, T3A, L12, E15, H16, D20, M23, S87, D84, N88, V91, I92, E95, Q126, S127, T123, R38, F42, Y46, P65, E68, L72, N26, N29, N30, N71, Q11, L132, L70, P82, G27, F78, T3, C125, T366, L368, Y407, S354, E356, Y349, H435, Y436 GenBank, 中国专利生物序列检索, China Patent Biological Sequence Search System, EBI-EMBL, STN and sequences: SEQ ID NOs: 2-9, 13-35, 38-42, 60-68, 188-97, 100-101 etc.

**C. DOCUMENTS CONSIDERED TO BE RELEVANT**

| Category* | Citation of document, with indication, where appropriate, of the relevant passages | Relevant to claim No. |
|---|---|---|
| X | CN 110392692 A (F. HOFFMANN-LA ROCHE AG) 29 October 2019 (2019-10-29) claims 1-37, description paragraphs [0021]-[0038], [0101]-[0217] | 1-5, 7, 20-31, 33-37 |
| X | CN 110382525 A (F. HOFFMANN-LA ROCHE AG) 25 October 2019 (2019-10-25) description paragraphs [0021]-[0056] | 1-5, 7, 20-31, 33-37 |
| X | WO 2022006380 A2 (INHIBRX INC.) 06 January 2022 (2022-01-06) claims 1-188 | 1-5, 7, 20-31, 33-37 |
| A | CN 111647068 A (JIANGSU HENGRUI MEDICINE CO., LTD. et al.) 11 September 2020 (2020-09-11) description paragraphs [0009]-[0126] | 1-37 |
| A | US 2021260163 A1 (ASKGENE PHARMA INC.) 26 August 2021 (2021-08-26) description, paragraphs [0009]-[0023] | 1-37 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **15 March 2023** | **16 May 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

## INTERNATIONAL SEARCH REPORT

| International application No. |
|---|
| **PCT/CN2023/075448** |

**C.    DOCUMENTS CONSIDERED TO BE RELEVANT**

| Category* | Citation of document, with indication, where appropriate, of the relevant passages | Relevant to claim No. |
|---|---|---|
| A | WO 2021255138 A1 (F. HOFFMANN-LA ROCHE AG et al.) 23 December 2021 (2021-12-23)<br>claims 1, 55-61, description, paragraphs [0454]-[0540] | 1-37 |

Form PCT/ISA/210 (second sheet) (July 2022)

**INTERNATIONAL SEARCH REPORT**

| International application No. |
| --- |
| **PCT/CN2023/075448** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/075448** |

**Box No. II       Observations where certain claims were found unsearchable (Continuation of item 2 of first sheet)**

This international search report has not been established in respect of certain claims under Article 17(2)(a) for the following reasons:

1. ☑  Claims Nos.: **37-40**
   because they relate to subject matter not required to be searched by this Authority, namely:

   PCT Rule 39.1(iv) – methods for treatment of the human or animal body by surgery or therapy.

   The search and the written opinion regarding claim 37 are provided on the basis of a reasonable expectation that the subject matter thereof is the use of the immunoconjugate of any one of claims 1 to 32, the nucleic acid molecule of claim 33, or the pharmaceutical composition of claim 36 in at least one of following items (1)-(5): (1) preparing a drug for treating cancer; (2) preparing a drug for activating cytotoxic T lymphocytes (CTL); (3) preparing a drug for promoting proliferation of CD4+ and/or CD8+ T lymphocytes; (4) preparing a drug for enabling a CTL with high expression of a PD-1 antibody to have a higher cell activation efficiency than a CTL with a low expression or no expression of the PD-1 antibody; and (5) preparing a drug for enabling $CD4^+$ and/or $CD8^+$ T lymphocytes with high expression of a PD-1 antibody to have a higher cell proliferation efficiency than $CD4^+$ and/or $CD8^+$ T lymphocytes with a low expression or no expression of the PD-1 antibody.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
|---|
| **PCT/CN2023/075448** |

| Patent document cited in search report | | | Publication date (day/month/year) | Patent family member(s) | | | Publication date (day/month/year) |
|---|---|---|---|---|---|---|---|
| CN | 110392692 | A | 29 October 2019 | TW | 201900220 | A | 01 January 2019 |
| | | | | DK | 3606946 | T3 | 24 October 2022 |
| | | | | PL | 3606946 | T3 | 28 November 2022 |
| | | | | LT | 3606946 | T | 25 October 2022 |
| | | | | MA | 49033 | A | 12 February 2020 |
| | | | | MA | 49033 | B1 | 31 October 2022 |
| | | | | CA | 3053357 | A1 | 11 October 2018 |
| | | | | CL | 2019002346 | A1 | 10 January 2020 |
| | | | | JP | 2020515275 | A | 28 May 2020 |
| | | | | EP | 3606946 | A1 | 12 February 2020 |
| | | | | EP | 3606946 | B1 | 24 August 2022 |
| | | | | JP | 2022062001 | A | 19 April 2022 |
| | | | | ES | 2928718 | T3 | 22 November 2022 |
| | | | | HUE | 059885 | T2 | 28 January 2023 |
| | | | | AU | 2018247765 | A1 | 22 August 2019 |
| | | | | PH | 12019502273 | A1 | 07 December 2020 |
| | | | | KR | 20210124518 | A | 14 October 2021 |
| | | | | KR | 102461885 | B1 | 03 November 2022 |
| | | | | AR | 111400 | A1 | 10 July 2019 |
| | | | | US | 2018326010 | A1 | 15 November 2018 |
| | | | | US | 11413331 | B2 | 16 August 2022 |
| | | | | KR | 20190121816 | A | 28 October 2019 |
| | | | | ZA | 201905517 | B | 26 May 2021 |
| | | | | UA | 125700 | C2 | 18 May 2022 |
| | | | | RS | 63663 | B1 | 30 November 2022 |
| | | | | PT | 3606946 | T | 17 October 2022 |
| | | | | RU | 2019134338 | A | 05 May 2021 |
| | | | | RU | 2019134338 | A3 | 07 December 2021 |
| | | | | CO | 2019009354 | A2 | 09 September 2019 |
| | | | | BR | 112019017329 | A2 | 14 April 2020 |
| | | | | SG | 11201909205 | YA | 28 November 2019 |
| | | | | IL | 269395 | A | 28 November 2019 |
| | | | | HRP | 20221254 | T1 | 23 December 2022 |
| | | | | MX | 2019011770 | A | 09 January 2020 |
| | | | | PE | 20191494 | A1 | 21 October 2019 |
| | | | | CR | 20190426 | A | 01 November 2019 |
| | | | | WO | 2018184964 | A1 | 11 October 2018 |
| CN | 110382525 | A | 25 October 2019 | TW | 201842937 | A | 16 December 2018 |
| | | | | WO | 2018184965 | A1 | 11 October 2018 |
| | | | | US | 2018326011 | A1 | 15 November 2018 |
| | | | | AR | 111203 | A1 | 12 June 2019 |
| | | | | EP | 3606947 | A1 | 12 February 2020 |
| | | | | EP | 3606947 | B1 | 21 December 2022 |
| | | | | JP | 2020512814 | A | 30 April 2020 |
| | | | | JP | 7148539 | B2 | 05 October 2022 |
| WO | 2022006380 | A2 | 06 January 2022 | WO | 2022006380 | A3 | 24 February 2022 |
| | | | | AR | 122863 | A1 | 12 October 2022 |
| | | | | TW | 202216745 | A | 01 May 2022 |
| | | | | AU | 2021299552 | A1 | 02 February 2023 |
| CN | 111647068 | A | 11 September 2020 | None | | | |

Form PCT/ISA/210 (patent family annex) (July 2022)

**INTERNATIONAL SEARCH REPORT**
Information on patent family members

International application No.

**PCT/CN2023/075448**

| Patent document cited in search report | | | Publication date (day/month/year) | Patent family member(s) | | | Publication date (day/month/year) |
|---|---|---|---|---|---|---|---|
| US | 2021260163 | A1 | 26 August 2021 | EP | 3762406 | A2 | 13 January 2021 |
| | | | | WO | 2019173832 | A2 | 12 September 2019 |
| | | | | WO | 2019173832 | A3 | 17 October 2019 |
| | | | | WO | 2019173832 | A4 | 02 January 2020 |
| | | | | CA | 3115461 | A1 | 12 September 2019 |
| | | | | JP | 2021515599 | A | 24 June 2021 |
| WO | 2021255138 | A1 | 23 December 2021 | AU | 2021291405 | A1 | 29 September 2022 |
| | | | | TW | 202219065 | A | 16 May 2022 |
| | | | | AR | 122657 | A1 | 28 September 2022 |
| | | | | BR | 112022025250 | A2 | 27 December 2022 |
| | | | | CA | 3176552 | A1 | 23 December 2021 |
| | | | | IL | 296225 | A | 01 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210127604
- WO 2020125743 A
- WO 9627011 A
- WO 98050431 A
- EP 1870459 A
- WO 2007110205 A
- WO 2007147901 A
- WO 2009089004 A
- WO 2010129304 A
- WO 201190754 A
- WO 2011143545 A
- WO 2012058768 A
- WO 2013157954 A
- WO 2013096291 A
- WO 2012107417 A
- WO 2009040562 A
- WO 2010035012 A
- WO 2005003169 A
- WO 2005003170 A
- WO 2005003171 A
- WO 9222583 A
- WO 05113605 A
- WO 2006082515 A
- WO 2012130831 A

### Non-patent literature cited in the description

- **RILEY et al.** *Immunol. Rev.*, 2009, vol. 29, 114-25
- **CHEN et al.** *Nat. Rev. Immunol.*, 2013, vol. 13, 227-42
- **DONG et al.** *Nat. Med.*, 1999, vol. 5, 1365-9
- **BORGHAEI et al.** *N Engl J Med.*, 2015, vol. 373, 1627-39
- **DAUD et al.** *J. Clin. Invest.*, 2016, vol. 126, 3447-52
- **AYERS et al.** *J. Clin. Invest.*, 2017, vol. 127, 2930-2940
- **DIAB et al.** *Cancer Discov.*, 2020, vol. 10, 1158-1173
- Antibodies: A Laboratory Manual. Cold Spring Harbor Press
- *J. biol. chem.*, 1968, vol. 243, 3558
- **OLEJNICZAK ; KASPRZAK.** *MedSci Monit*, 2008, vol. 14, RA179-189
- **HOLLIGER ; HUDSON.** *Nature Biotech.*, 2005, vol. 23 (9), 1126-1136
- **ADAIR ; LAWSON.** *Drug Design Reviews-Online*, 2005, vol. 2 (3), 209-217
- **VERMA et al.** *Journal of Immunological Methods*, 1998, vol. 216, 165-181
- **KABAT et al.** Sequences of Proteins of Immunological Interest. Public Health Service, National Institutes of Health, 1991
- **MCCOY et al.** *J Mol Biol*, 1997, vol. 268, 570-584
- **LEE et al.** *Protein Sci.*, 2001, vol. 10, 362-377
- **CHAU et al.** *J Comp Mol Des*, 1994, vol. 8, 51325
- **LAWRENCE et al.** *J Mol Biol*, 1993, vol. 234, 946-950
- **WALLS et al.** *J Mol Biol*, 1992, vol. 228, 277-297
- **SCHUELER-FURMAN et al.** *Proteins*, 2005, vol. 60, 187-194
- **JOHNSON ; WU.** *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- **MARTIN et al.** *Proc Natl Acad Sci (USA)*, 1989, vol. 86, 9268-9272
- **SAMUDRALA et al.** Ab Initio Protein Structure Prediction Using a Combined Hierarchical Approach. *PROTEINS, Structure, Function and Genetics*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature*, 1993, vol. 363, 446-448
- **KABAT, E. A. et al.** Sequences of Proteins of Immunological Interest. 1991
- **MARKS et al.** *Biotechnology*, 1992, vol. 10, 779-783
- **BARBAS et al.** *Proc. Nat. Acad. Sci, USA*, 1994, vol. 91, 3809-3813

- **SHIER et al.** *Gene*, 1995, vol. 169, 147-155
- **YELTON et al.** *Immunol.*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226 (3), 889896
- **KS JOHNSON** ; **RE HAWKINS**. Affinity maturation of antibodies using phage display. Oxford University Press, 1996
- **CACECI et al.** *Byte*, 1984, vol. 9, 340-362
- **WONG** ; **LOHMAN**. *Proc. Natl. Acad. Sci. USA*, 1993, vol. 90, 5428-5432
- Remington's Pharmaceutical Sciences. Mack Publishing Co., 1990
- **R REMINGTON**. The Science and Practice of Pharmacy. Mack Publishing, 2000
- **SAMBROOK et al.** Molecular Cloning: A Laboratory Manual. Cold Spring Harbor Laboratory Press
- Current Protocols in Molecular Biology. Greene Publishing Association, Wiley Interscience